# EUROPEAN PATENT APPLICATION

(11) **EP 1 775 304 A1**
(43) Date of publication of application: **18.04.2007**
(21) Application number: 05762068.4
(22) Date of filing: 12.07.2005
(51) Int. Cl.: C07J 9/00

(54) **PROCESS FOR PRODUCTION OF STEROIDS**

(30) Priority: 13.07.2004 JP 2004205515; 23.07.2004 JP 2004215304
(71) Applicant: Mitsubishi Chemical Corporation, Minato-ku Tokyo 108-0014 (JP)
(72) Inventor: TAKEHARA, Jun, MITSUBISHI CHEM. GROUP INC., Yokohama-shi, Kanagawa (JP); FUJIWARA, Naoya, MITSUBISHI CHEM. GROUP INC., Yokohama-shi, Kanagawa (JP); KAWAI, Junya, MITSUBISHI CHEM. GROUP INC., Yokohama-shi, Kanagawa (JP); ENDOU, Kyouko, MITSUBISHI CHEM. GROUP INC., Yokohama-shi, Kanagawa (JP)
(74) Representative: polypatent
(86) International application number: PCT/JP2005/013216
(87) International publication number: WO 2006/006718

(57) **Abstract**

An object of the present invention is to provide a novel method for producing a steroid compound. The present invention provides a method for producing 3,7-dioxo-5β-cholanic acid or ester derivatives thereof, which uses, as raw materials, sterols having double bonds at positions 5 and 24, such as cholesta-5,7,24-trien-3β-ol, ergosta-5,7,24(28)-trien-3β-ol, desmosterol, fucosterol, or ergosta-5, 24(28)-dien-3β-ol, and which comprises the following 4 steps: (I) a step of performing oxidation of a hydroxyl group at position 3 and isomerization of a double bond at position 5 to position 4; (II) a step of converting position 24 to a carboxyl group or an ester derivative thereof by the oxidative cleavage of a side chain; (III) a step of introducing an oxygen functional group into position 7; and (IV) a step of constructing a 5β-configuration by reduction of a double bond at position 4.

## Description

### TECHNICAL FIELD

The present invention relates to a method for producing steroid compounds. Specifically, the present invention relates to a method for producing 3,7-dioxo-5β-cholanic acid or ester derivatives thereof, which comprises reduction of steroid compounds having a double bond at position 4, so as to construct a 5β-configuration. More specifically, the present invention relates to a method for producing 3,7-dioxo-5β-cholanic acid or ester derivatives thereof, which uses, as raw materials, sterols having double bonds at positions 5 and 24, such as cholesta-5,7,24-trien-3β-ol, ergosta-5,7,24(28)-trien-3β-ol, desmosterol, fucosterol, or ergosta-5, 24(28)-dien-3β-ol, and which comprises the following 4 steps:
(I) a step of performing oxidation of a hydroxyl group at position 3 and isomerization of a double bond at position 5 to position 4;
(II) a step of converting position 24 to a carboxyl group or ester derivatives thereof by the oxidative cleavage of a side chain;
(III) a step of introducing an oxygen functional group into position 7; and
(IV) a step of constructing a 5β-configuration by reduction of a double bond at position 4.

Further more specifically, the present invention relates to a method for producing 3,7-dioxo-5β-cholanic acid or ester derivatives thereof by a chemical synthesis method using cholesta-5,7,24-trien-3β-ol as a raw material, via cholesta-4,6,24-trien-3-one. Such 3,7-dioxo-5β-cholanic acid or ester derivatives thereof is useful as synthetic intermediates of medicaments such as ursodeoxycholic acid or chenodeoxycholic acid.

Further, the present invention relates to a method for producing cholesta-4,6,24-trien-3-one using cholesta-5,7,24-trien-3-ol as a raw material. This product is useful as a synthetic intermediate of various steroid medicaments.

### BACKGROUND ART

As a method for producing 3,7-dioxo-5β-cholanic acid or ester derivatives thereof, there have been reported several methods involving oxidation of hydroxyl groups of a raw material having the same skeleton and the same number of carbon atoms, such as chenodeoxycholic acid or ursodeoxycholic acid derived from bile acid (refer to Japanese Patent Application Laid-Open Nos. 52-78864 and 52-78863; Spanish Patent No. 489661; French Patent No. 2453182; Nihon Kagaku Zassi, 1955, vol. 76, p. 297; J. Chem. Soc., Perkin., 1, 1990, vol. 1, p. 1, for example). As a method for producing such chenodeoxycholic acid or ursodeoxycholic acid, the following methods have been reported: (1) a production method using bile acid contained in animals as a raw material (refer to Japanese Patent Application Laid-Open Nos. 64-61496 and 58-029799); Nihon Kagaku Zassi, 1955, vol. 76, p. 297; and J. Chem. Soc., Perkin. 1, 1990, vol. 1, p. 1, for example); (2) a method of producing such chenodeoxycholic acid or ursodeoxycholic acid by inducing them from steroids derived from plants, such as stigmasterol (refer to Chinese Patent No. 1217336; and Yunnan Daxue Xuebao, Ziran Kexueban, 1998, vol. 20, p. 399, for example); and (3) a method of producing such chenodeoxycholic acid or ursodeoxycholic acid by inducing them from raw materials having a few number of carbons on a side chain, such as progesterone (refer to Chinese Patent No. 1308085, for example).

However, the method described in (1) above involves expensive raw materials derived from natural source. It has been difficult to acquire sufficient quantities of such raw materials, and thus it has been desired an inexpensive chemical synthesis method be established. In addition, the methods described in (2) and (3) above also involve expensive raw materials derived from natural source, as with the method (1). These methods require a step of adjusting the number of carbon atoms on a side chain to that of a desired compound, or a multistep oxidation for introducing a functional group at position 7. Thus, these methods require a large number of steps, and they are thereby not economically efficient.

On the other hand, a method of obtaining a 3-oxo-4-ene steroid compound by subjecting a steroid compound having a double bond at position 5 and a hydroxyl group at position 3 to Oppenauer Oxidation, is described, for example, in Org. Synth. Col. Vol. III, 1955, p. 207.

Moreover, International Publication WO02/088166, the publication Steroids, 1983, vol. 42, No. 6, p. 707, and other publications, describe that reduction of a double bond at position 4 is effective as a means for constructing a 5β-configuration.

Furthermore, Helv. Chim. Acta, 1971, vol. 54, No. 8, p. 2775, and other publications, describe a method of obtaining a 3-oxo-4-ene-6,7-epoxy steroid compound by epoxidation of a 3-oxo-4,6-diene steroid compound, as a means for introducing an oxygen functional group into the position 7 of a steroid compound. Still further, Appl. Environ. Microbiol., 1986, vol. 51, p. 946, and other publications, describe a method of obtaining a 3-oxo-4-ene-7-ol steroid compound from a 3-oxo-4-ene steroid compound, using microorganisms. Still further, J. Chem. Res., Synop., 1986, No. 2, p. 48, and other publications, describe a method of obtaining a 3-oxo-7-ol steroid compound from a 3-oxo steroid compound, using microorganisms. Still further, Appl. Environ. Microbiol., 1982, vol. 44, No. 6, p.1249, and other publications, describe a method of obtaining a 5β-3,7-dihydroxy steroid compound from a 5β-3-hydroxy steroid compound, using microorganisms.

Moreover, as methods of cleaving a double bond by oxidation based on common findings of organic chemistry, there have been known a method of cleaving a double bond including by epoxidation or glycolation, a method of cleaving a double bond via ketone, a direct cleavage method using ozone, and other methods.

Furthermore, cholesta-4,6,24-trien-3-one that becomes an intermediate when cholesta-5,7,24-trien-3β-ol is used as a raw material is useful as a synthetic intermediate of various steroid medicaments. This compound has previously been obtained from a raw material derived from natural source, and further it has been produced via a long reaction process. Accordingly, the applicability of this compound has been limited in terms of cost and quantity. For example, Biochem. Biophys. Res. Commun., 1965., vol. 21, No. 2, p. 149, describes a method for producing cholesta-4,6,24-trien-3-one, which comprises: subjecting cholesta-5,24-dien-3-ol (desmo) to Oppenauer Oxidation, so as to obtain 3-oxo-4,24-diene; subjecting the obtained 3-oxo-4,24-diene to enol etherification, so as to obtain 3-ethoxy-3,5,24-triene; and oxidizing the obtained 3-ethoxy-3,5,24-triene with manganese dioxide, so as to produce cholesta-4,6,24-trien-3-one.

On the other hand, Japanese Patent Application Laid-Open No. 2004-141125 describes a method for producing cholesta-5,7,24-trien-3β-ol, which comprises: modifying in a metabolic engineering manner Eumycetes that produce ergosterol via zymosterol; culturing the thus produced mutant strain; and collecting cholesta-5,7,24-trien-3β-ol from the culture product.

### DISCLOSURE OF THE INVENTION

It is an object of the present invention to provide a method for producing 3,7-dioxo-5β-cholanic acid or ester derivatives thereof, which uses, as raw materials, sterols having double bonds at positions 5 and 24, more specifically, such as cholesta-5,7,24-trien-3β-ol, ergosta-5,7,24(28)-trien-3β-ol, desmosterol, fucosterol, or ergosta-5, 24(28)-dien-3β-ol, and which comprises the following 4 steps:
(I) a step of performing oxidation of a hydroxyl group at position 3 and isomerization of a double bond at position 5 to position 4;
(II) a step of converting position 24 to a carboxyl group or ester derivatives thereof by the oxidative cleavage of a side chain;
(III) a step of introducing an oxygen functional group into position 7; and
(IV) a step of constructing a 5β-configuration by reduction of a double bond at position 4.

More specifically, it is an object of the present invention to provide a method for synthesizing 3,7-dioxo-5β-cholanic acid or ester derivatives thereof, which is a compound having oxygen functional groups at positions 3 and 7 and having carboxylic acid or an ester at position 24, wherein cholesta-5,7,24-trien-3β-ol is used as a raw material and the compound of interest is obtained via cholesta-4,6,24-trien-3-one:

In order to efficiently produce 3,7-dioxo-5β-cholanic acid or ester derivatives thereof from raw materials other than those having the same skeleton and the same number of carbon atoms, it is preferable to use a steroid raw material, which is able to construct the same side chain carbon number by a few steps. Accordingly, steroids having a double bond at position 24 can be induced to a carboxyl group at position 24 or ester derivatives thereof by the oxidative cleavage.

In addition, 3-sterols having a double bond at position 5 are subjected reduction via oxidation of position 3 and isomerization of a double bond at position 5 to position 4, so as to construct a 5β-configuration.

Moreover, in the case of 3-oxo-4,6-diene steroids, introduction of an oxygen functional group into position 7 may be achieved by epoxidation of a double bond at position 6. In the case of 3-oxo-4-ene steroids, 3-oxo steroids, and 3-hydroxy steroids, such position 7 can be hydroxylated using microorganisms.

As a result of intensive studies directed towards achieving the aforementioned objects, the present inventors have found that when cholesta-5,7,24-trien-3β-ol is used as a raw material for example, oxidation to a ketone form thereof at position 3 and isomerization of a double bond thereof at position 5 to position 4 are first carried out, and a double bond thereof at position 7 is then isomerized to a double bond thereof at position 6, so as to produce a 3-oxo-4,6,24-triene compound.

In addition, the inventors have also found that the double bonds at positions 6 and 24 of cholesta-4,6,24-trien-3-one are epoxidized, that saturation of a double bond at position 4 by hydrogenation, the reductive cleavage of a carbon-oxygen bond at position 6, and construction of a 5β-configuration, are then carried out, that 24,25-epoxy group is hydrolyzed to 24,25-diol, that oxidation of a hydroxyl group at position 7 to ketone and the oxidative cleavage thereof to 24-carboxylic acid are then carried out, and that the 24-carboxylic acid may be further esterified in some cases, thereby synthesizing 3,7-dioxo-5β-cholanic acid and ester derivatives thereof that are useful as synthetic intermediates of various steroids, such as ursodeoxycholic acid or chenodeoxycholic acid.

Moreover, the inventors have also found that after epoxidation of the double bonds at position 6 and 24 in the aforementioned reaction, the order of the reaction is changed such that only the 24,25-epoxy group is first hydrolyzed, so as to obtain diol, and such that hydrogenation of the 6,7-epoxy group and saturation of the double bond at position 4 are then carried out, thereby synthesizing the same above 3,7-dioxo-5β-cholanic acid and ester derivatives thereof.

Furthermore, they have also found that only the position 24 of cholesta-4,6,24-trien-3-one is epoxidized and hydrolyzed, so as to obtain diol, that the double bond at position 6 is epoxidized, that hydrogenation of the 6,7-epoxy group and saturation of the double bond at position 4 are then carried out, that oxidation of a hydroxyl group at position 7 to ketone and the oxidative cleavage to 24-carboxylic acid are then carried out, and that 24-carboxylic acid may be further esterified in some cases, thereby synthesizing the same above 3,7-dioxo-5β-cholanic acid and ester derivatives thereof.

Still further, they have also found that after hydrogenation of the 6,7-epoxy group and saturation of the double bond at position 4 in the aforementioned reaction, the hydroxyl group at position 7 is oxidized, that the 24,25-epoxy group is hydrolyzed, that the oxidative cleavage to 24-carboxylic acid is carried out, and that the 24-carboxylic acid may be further esterified in some cases, thereby synthesizing the same above 3,7-dioxo-5β-cholanic acid and ester derivatives thereof.

Thus, based on the aforementioned findings, the present inventors have found that 3,7-dioxo-5β-cholanic acid or ester derivatives thereof can be produced using, as raw materials, sterols having double bonds at positions 5 and 24, such as cholesta-5,7,24-trien-3β-ol, ergosta-5,7,24(28)-trien-3β-ol, desmosterol, fucosterol, or ergosta-5, 24(28)-dien-3β-ol, by performing the following 4 steps:
(I) a step of performing oxidation of a hydroxyl group at position 3 and isomerization of a double bond at position 5 to position 4;
(II) a step of converting position 24 to a carboxyl group or ester derivatives thereof by the oxidative cleavage of a side chain;
(III) a step of introducing an oxygen functional group into position 7; and
(IV) a step of constructing a 5β-configuration by reduction of a double bond at position 4, thereby completing the present invention.

The schematic views of the aforementioned steps (I) to (IV) are shown below. 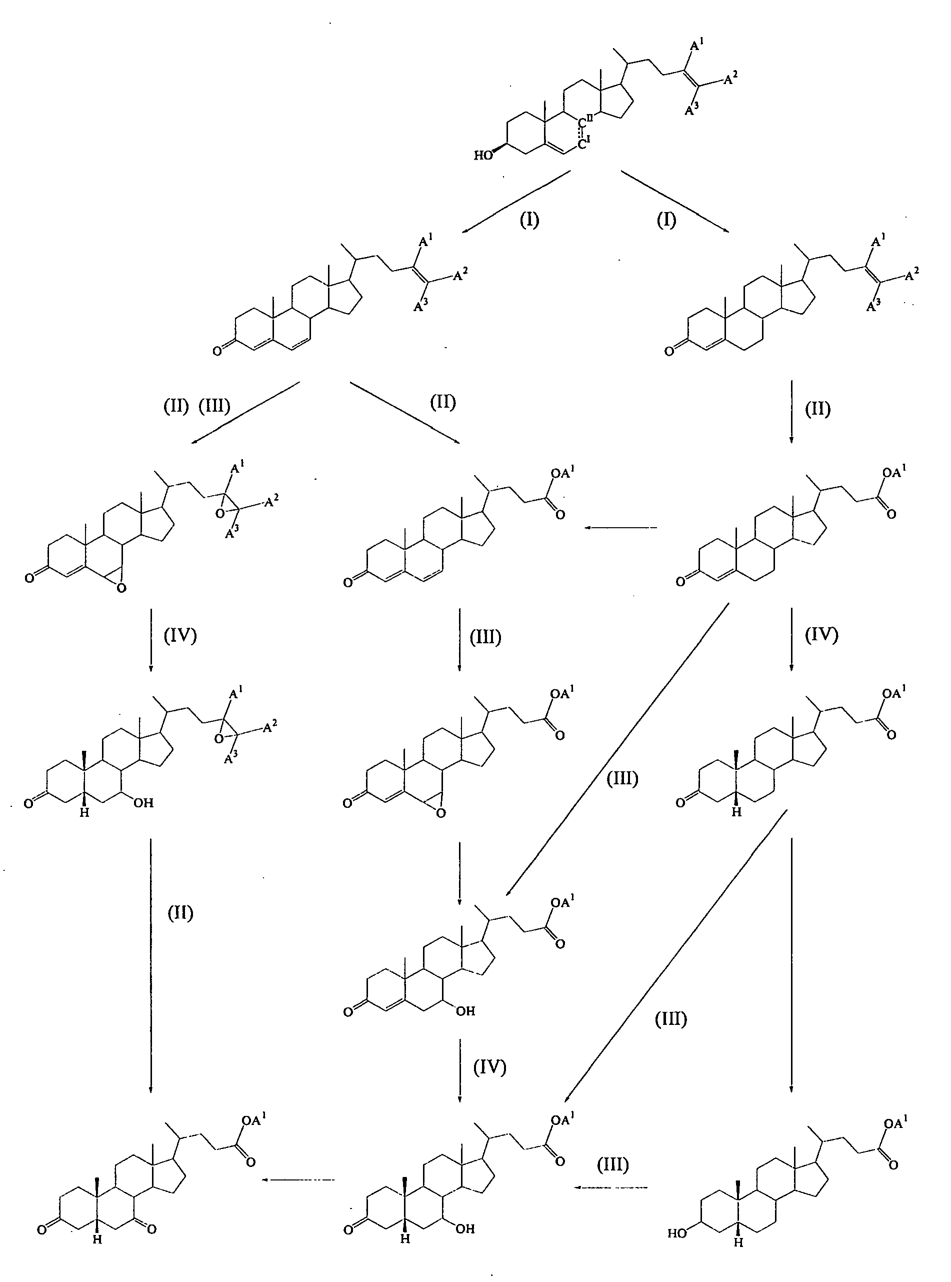 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.

The schematic view of the aforementioned step (II) is shown below. 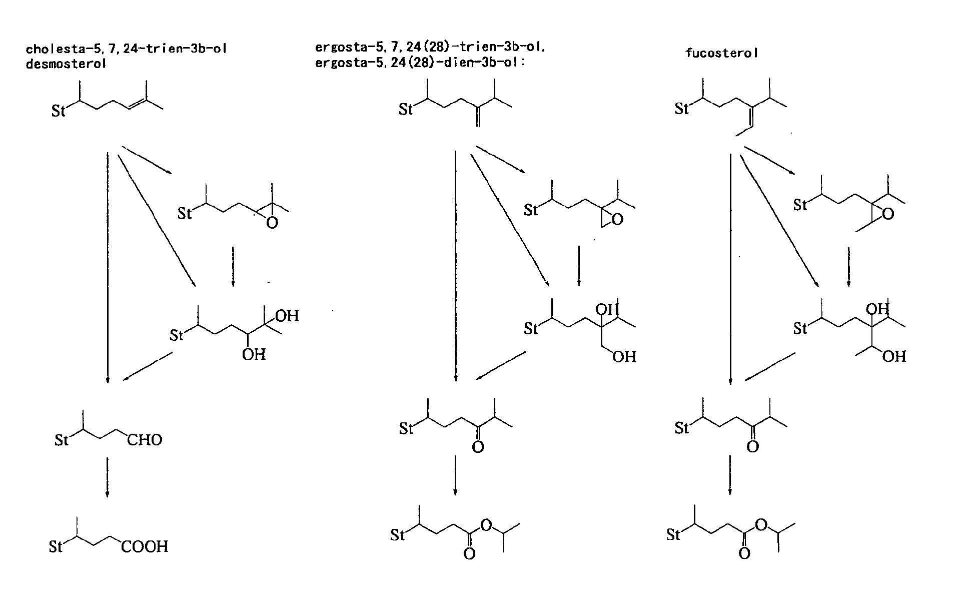 wherein St represents a steroid skeleton consisting of ring A, ring B, ring C, and ring D, and such a steroid skeleton (1) binds to the side chain shown in the formula at position C 17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C 13, and C 14 may be substituted with a methyl group(s).

That is to say, the present invention provides the inventions described in the following (1) to (49):
(1) A method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d): 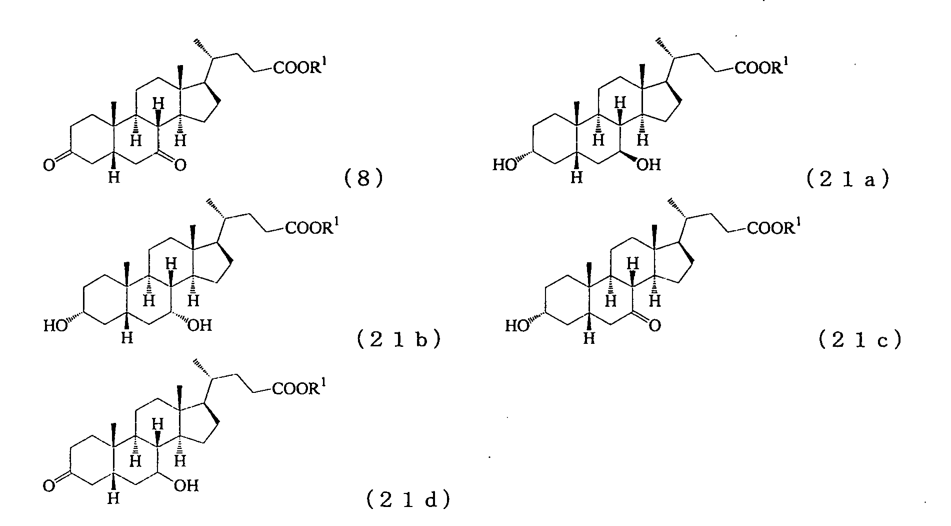 wherein R¹ represents a hydrogen atom, or an alkyl group containing 1 to 6 carbon atoms;
   which is characterized in that it uses a steroid compound containing 22 or more carbon atoms (preferably containing 24 or more carbon atoms) generated from carbohydrate by a fermentation method, and in that it comprises a step of constructing a 5β-configuration by reduction of a double bond at position 4.
(2) A method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d): 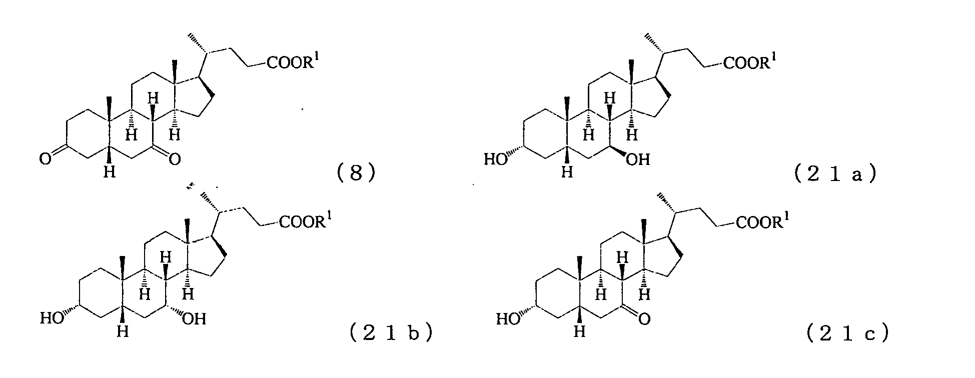 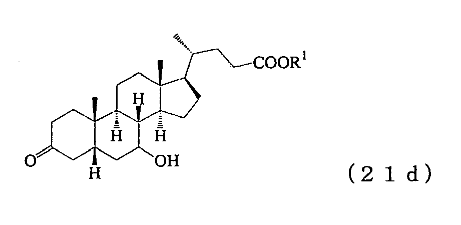 wherein R¹ represents a hydrogen atom, or an alkyl group containing 1 to 6 carbon atoms;
   which is characterized in that a 5β-configuration is constructed by reduction of a double bond at position 4 in a steroid compound represented by the following formula (A1), (A2), (A3), (A4), (A5), (A6), (A7), or (A8): 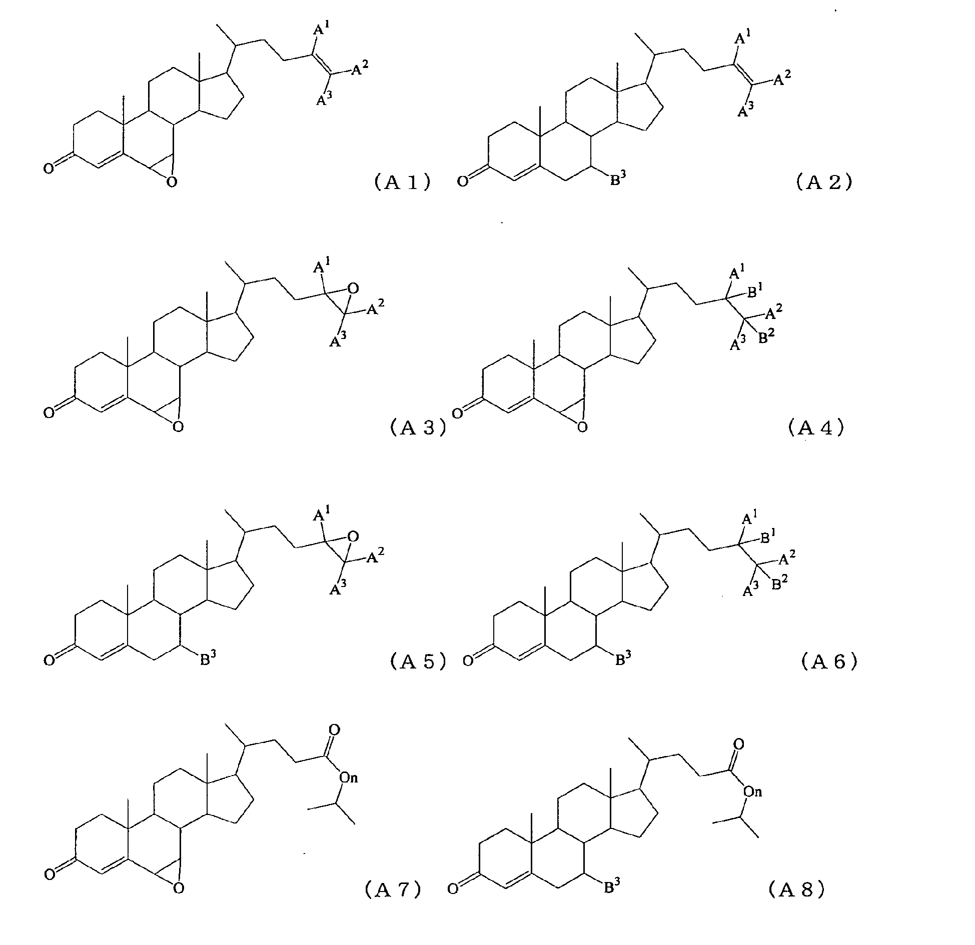 wherein A¹ represents a hydrogen atom or isopropyl group; each of A² and A³ independently represents a methyl group when A¹ is a hydrogen atom, and represents a hydrogen atom or methyl group when A¹ is an isopropyl group; each of B¹, B², and B³ independently represents a hydroxyl group or protected hydroxyl group; and n represents an integer of 0 or 1.
(3) The method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d), according to (2) above: 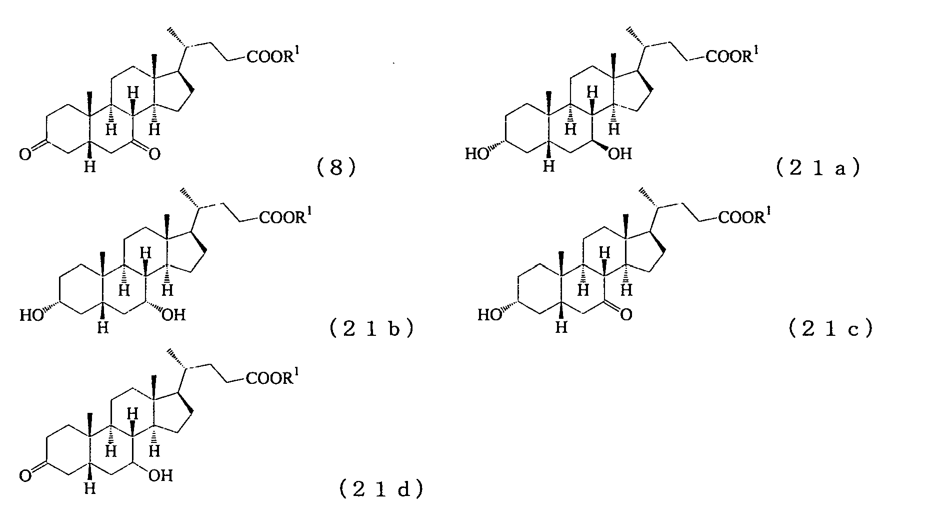 wherein R¹ represents a hydrogen atom, or an alkyl group containing 1 to 6 carbon atoms;
   which is characterized in that the steroid compound represented by the following formula (A1), (A2), (A3), (A4), (A5), (A6), (A7), or (A8) is induced from a sterol compound represented by the following formula (1): 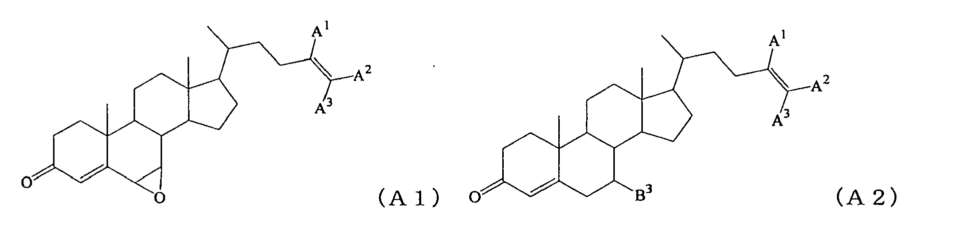 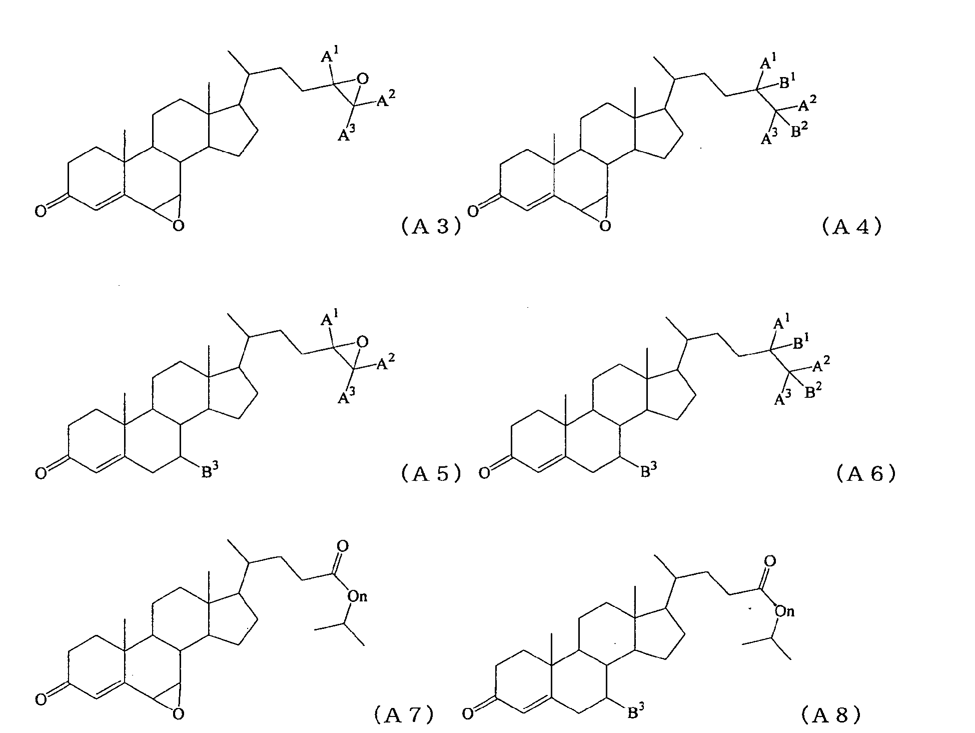 wherein A¹ represents a hydrogen atom or isopropyl group; each of A² and A³ independently represents a methyl group when A¹ is a hydrogen atom, and represents a hydrogen atom or methyl group when A¹ is an isopropyl group; each of B¹, B², and B³ independently represents a hydroxyl group or protected hydroxyl group; and n represents an integer of 0 or 1, 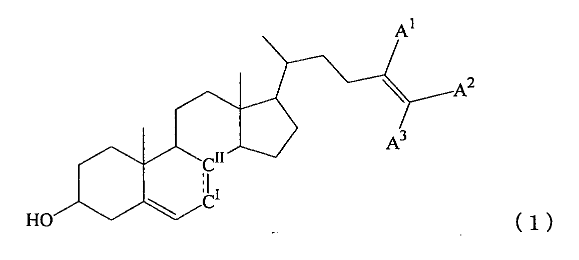 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.
(4) A method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d): 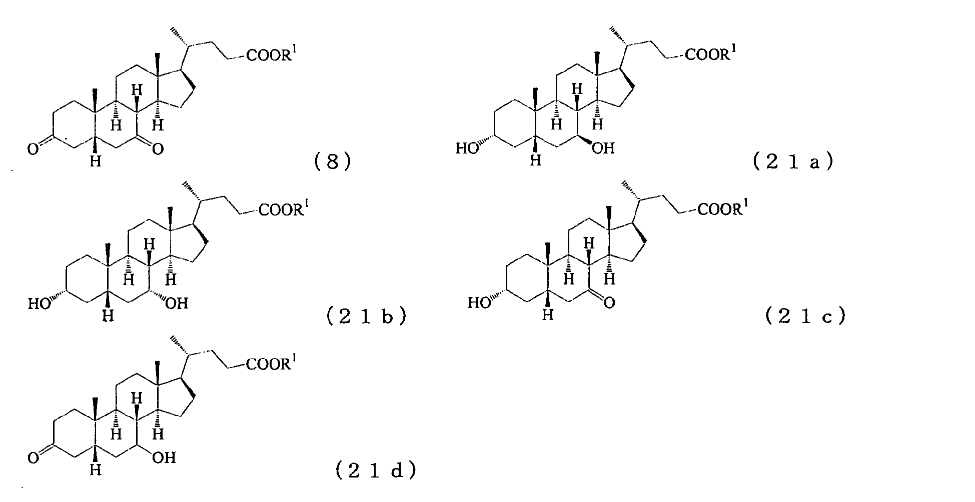 wherein R¹ represents a hydrogen atom, or an alkyl group containing 1 to 6 carbon atoms;
   which uses, as a raw material, a sterol compound represented by the following formula (1): 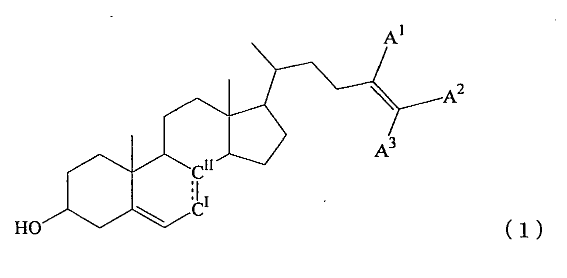 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond, and
   which comprises the following steps:
   (I) a step of performing oxidation of a hydroxyl group at position 3 and isomerization of a double bond at position 5 to position 4;
   (II) a step of converting position 24 to a carboxyl group or ester derivatives thereof by the oxidative cleavage of a side chain;
   (III) a step of introducing an oxygen functional group into position 7; and
   (IV) a step of constructing a 5β-configuration by reduction of a double bond at position 4.
(5) The method for producing 3,7-dioxo-5β-cholanic acid (8), ursodeoxycholic acid (21a), chenodeoxycholic acid (21b), 3α,-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d), according to (4) above: 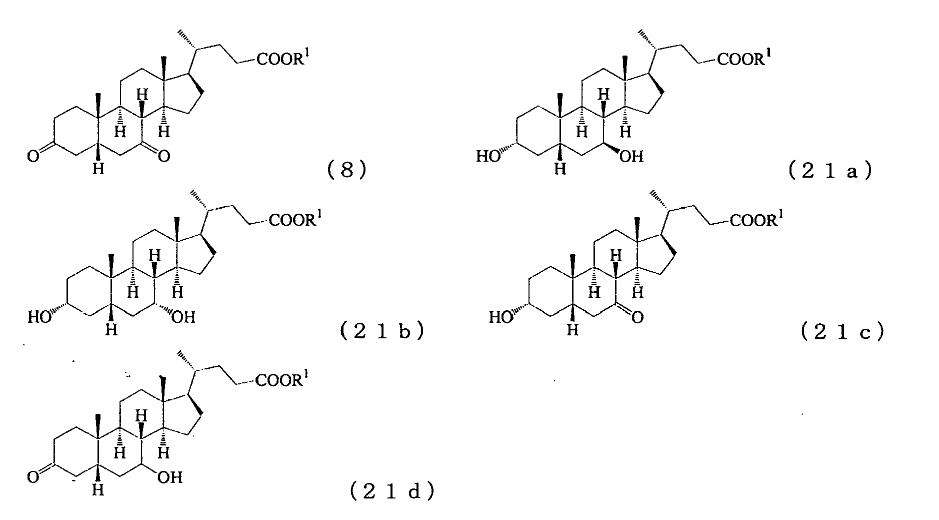 wherein R¹ represents a hydrogen atom, or an alkyl group containing 1 to 6 carbon atoms;
   wherein the sterol compound represented by the following formula (1) is cholesta-5,7,24-trien-3β-ol, ergosta-5,7,24(28)-trien-3β-ol, desmosterol, fucosterol, or ergosta-5,24(28)-dien-3β-ol: 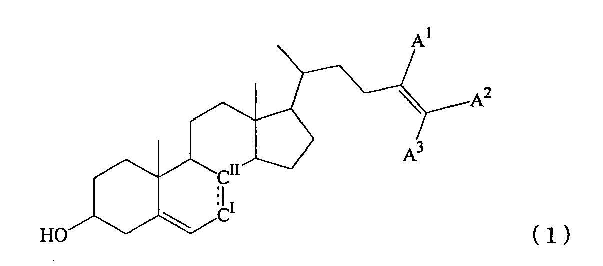 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.
(6) The method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d), according to (4) above: 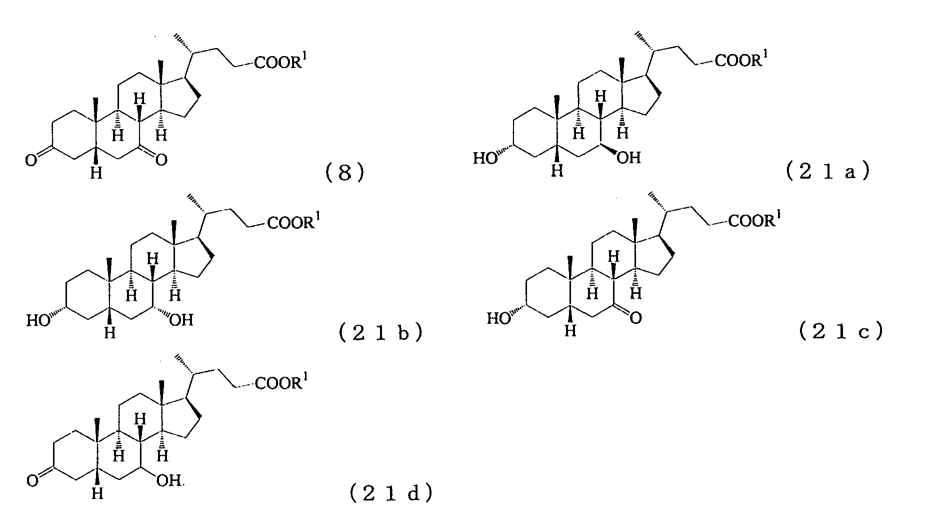 wherein R¹ represents a hydrogen atom, or an alkyl group containing 1 to 6 carbon atoms;
   wherein the sterol compound represented by the following formula (1) is cholesta-5,7,24-trien-3β-ol: 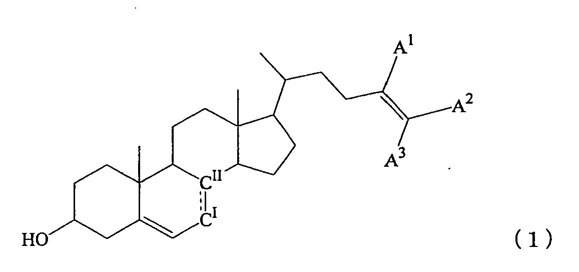 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.
(7) A method for producing cholesta-4,6,24-trien-3-one represented by the following formula (4): 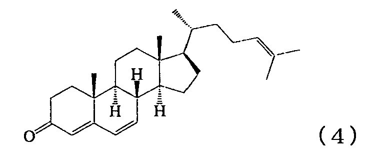 which is characterized in that cholesta-5,7,24-trien-3β-ol represented by the following formula (2) is oxidized so as to obtain cholesta-4,7,24-trien-3-one represented by the following formula (3), and in that the obtained cholesta-4,7,24-trien-3-one is then isomerized: 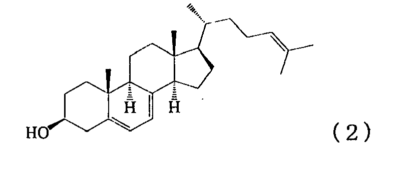 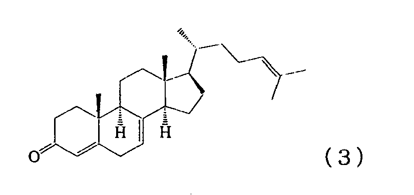
(8) The method for producing cholesta-4,6,24-trien-3-one according to (7) above, which is characterized in that the oxidation reaction is carried out in the presence of a ketone compound and a metal alkoxide.
(9) The method for producing cholesta-4,6,24-trien-3-one according to (8) above, which is characterized in that the oxidation reaction is carried out while oxygen is blocked.
(10) The method for producing cholesta-4,6,24-trien-3-one according to (8) above, which is characterized in that the ketone compound is represented by the formula R²(C=O)R³ wherein each of R² and R³ independently represents a chain or cyclic alkyl group containing 1 to 10 carbon atoms, or R² and R³ may bind to each other, so as to form a cyclic structure containing 3 to 8 carbon atoms.
(11) The method for producing cholesta-4,6,24-trien-3-one according to (7) above, which is characterized in that the isomerization reaction is carried out in the presence of a basic compound.
(12) The method for producing cholesta-4,6,24-trien-3-one according to (11) above, which is characterized in that the basic compound is hydroxide, carbonate or alkoxide of alkaline metal or alkaline-earth metal.
(13) The method for producing cholesta-4,6,24-trien-3-one according to (11) above, which is characterized in that the isomerization reaction is carried out while oxygen is blocked.
(14) A cholesta-4,7,24-trien-3-one represented by the following formula (3): 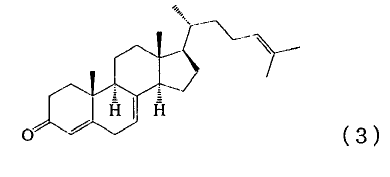
(15) A method for producing a 3-oxo-4,7-diene steroid compound, which comprises oxidizing a 3-hydroxy-5,7-diene steroid compound represented by the following formula (2a), (2b), (2c), (2d), or (2e), to a compound represented by the following formula (3a), (3b), (3c), (3d), or (3e): 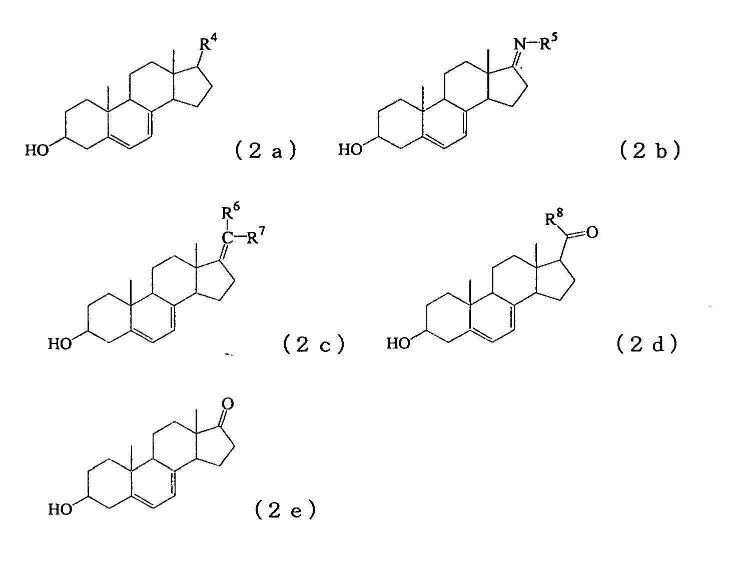 wherein each of R⁴ to R⁸ independently represents a hydrogen atom, a protected hydroxyl group or halogen atom, or an alkyl, alkenyl, or alkynyl group containing 1 to 10 carbon atoms, which may be substituted with a carbonyl group, an ether group, a protected hydroxyl group, a halogen atom, or a carboxyl group), 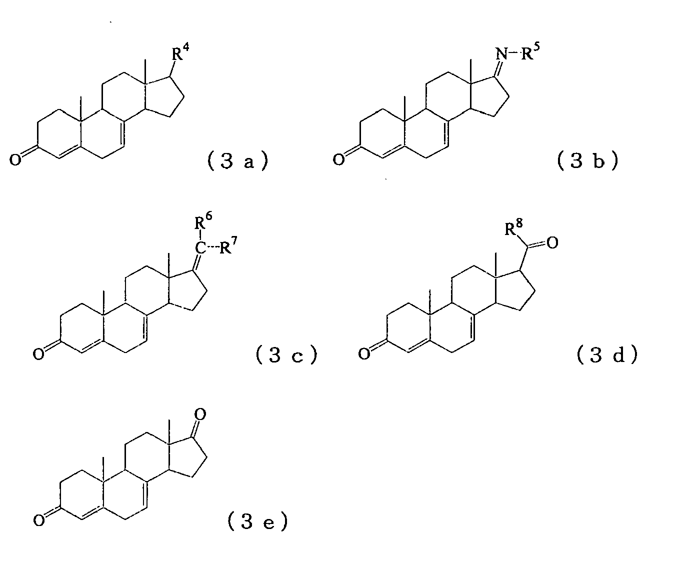 wherein each of R⁴ to R⁸ independently represents a hydrogen atom, a protected hydroxyl group or halogen atom, or an alkyl, alkenyl, or alkynyl group containing 1 to 10 carbon atoms, which may be substituted with a carbonyl group, an ether group, a protected hydroxyl group, a halogen atom, or a carboxyl group;
   which is characterized in that the above-described oxidation is carried out in the presence of a ketone compound and a metal alkoxide, while oxygen is blocked.
(16) A method for producing a 3-oxo-4,6-diene steroid compound, which is characterized in that 3-oxo-4,7-diene steroid compound represented by the following formula (3a), (3b), (3c), (3d), or (3e) is isomerized to a compound represented by the following formula (4a), (4b), (4c), (4d), or (4e), respectively, using a base as a catalyst: 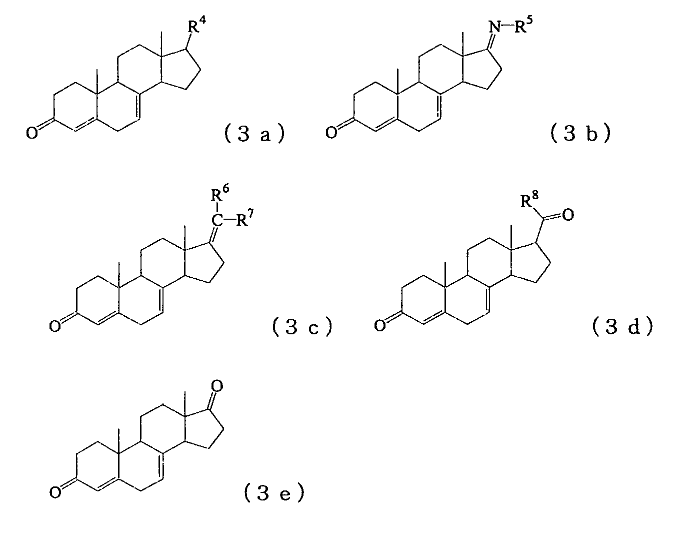 wherein each of R⁴ to R⁸ independently represents a hydrogen atom, a hydroxyl group, a protected hydroxyl group or halogen atom, or an alkyl, alkenyl, or alkynyl group containing 1 to 10 carbon atoms, which may be substituted with a carbonyl group, an ether group, a hydroxyl group, a protected hydroxyl group, a halogen atom, or a carboxyl group, 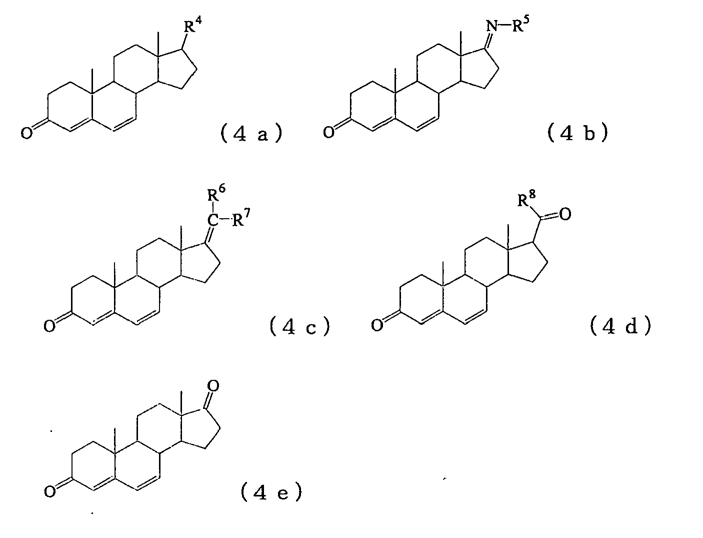 wherein each of R⁴ to R⁸ independently represents a hydrogen atom, a hydroxyl group, a protected hydroxyl group or halogen atom, or an alkyl, alkenyl, or alkynyl group containing 1 to 10 carbon atoms, which may be substituted with a carbonyl group, an ether group, a hydroxyl group, a protected hydroxyl group, a halogen atom, or a carboxyl group.
(17) A method for producing 3,7-dioxo-5β-cholanic acid represented by the following formula (8) or ester derivatives thereof: 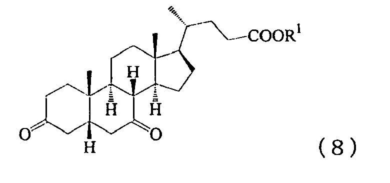 wherein R¹ represents a hydrogen atom, or an alkyl group containing 1 to 6 carbon atoms which is characterized in that it comprises:
   epoxidizing cholesta-4,6,24-trien-3-one represented by the following formula (4): 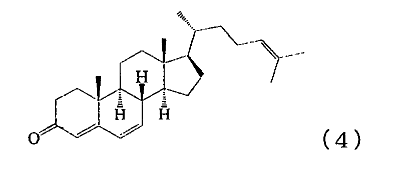
   so as to obtain 6,7:24,25-diepoxycholest-4-en-3-one represented by the following formula (5): 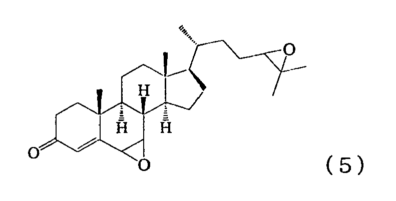
   then hydrogenating the obtained compound, so as to obtain 5β-24,25-epoxycholestan-3-one-7-ol represented by the following formula (6): 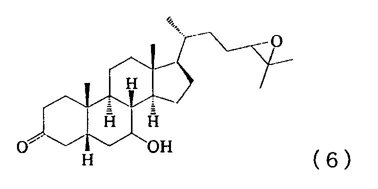
   then hydrolyzing the obtained compound, so as to obtain 5β-cholestan-3-one-7,24,25-triol represented by the following formula (7): 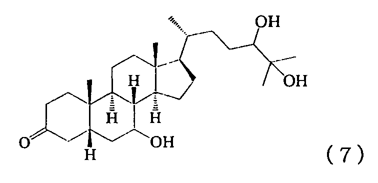
   and then oxidizing the obtained compound, and further esterifying the obtained compound in some cases.
(18) A method for producing 3,7-dioxo-5β-cholanic acid represented by the following formula (8) or ester derivatives thereof: 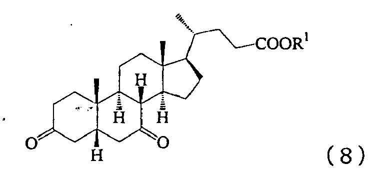 wherein R¹ represents a hydrogen atom, or an alkyl group containing 1 to 6 carbon atoms, which is characterized in that it comprises:
   epoxidizing cholesta-4,6,24-trien-3-one represented by the following formula (4): 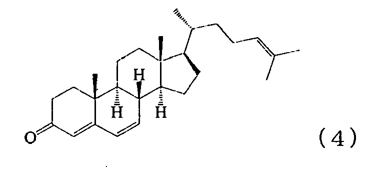
   so as to obtain 6,7:24,25-diepoxycholest-4-en-3-one represented by the following formula (5): 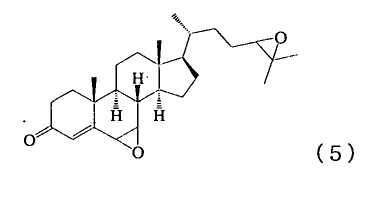
   then hydrolyzing the obtained compound, so as to obtain 6,7-epoxycholest-4-en-3-one-24,25-diol represented by the following formula (9): 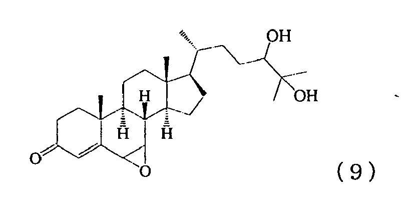
   then hydrogenating the obtained compound, so as to obtain 5β-cholestan-3-one-7,24,25-triol represented by the following formula (7): 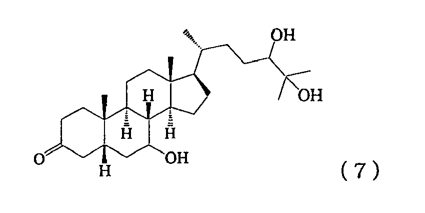
   and then oxidizing the obtained compound, and further esterifying the obtained compound in some cases.
(19) A method for producing 3,7-dioxo-5β-cholanic acid represented by the following formula (8) or ester derivatives thereof: 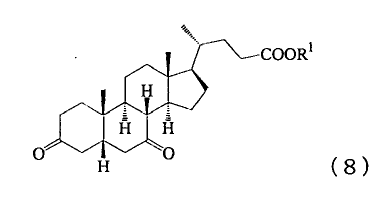 wherein R¹ represents a hydrogen atom, or an alkyl group containing 1 to 6 carbon atoms, which is characterized in that it comprises:
   epoxidizing cholesta-4,6,24-trien-3-one represented by the following formula (4): 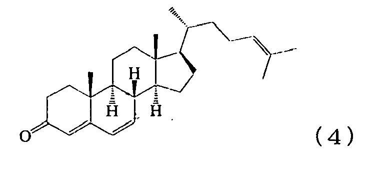
   so as to obtain 24,25-epoxycholesta-4,6-dien-3-one represented by the following formula (10): 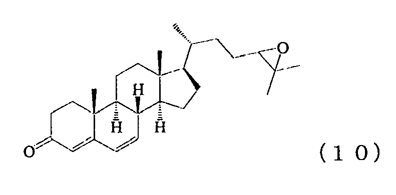
   then hydrolyzing the obtained compound, so as to obtain cholesta-4,6-dien-3-one-24,25-diol represented by the following formula (11): 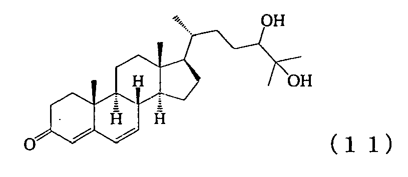
   then epoxidizing the obtained compound, so as to obtain 6,7-epoxycholest-4-en-3-one-24,25-diol represented by the following formula (9): 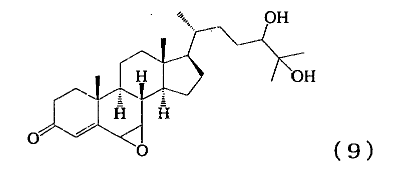
   then hydrogenating the obtained compound, so as to obtain 5β-cholestan-3-one-7,24,25-triol represented by the following formula (7): 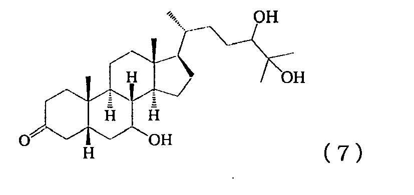
   and then oxidizing the obtained compound, and further esterifying the obtained compound in some cases.
(20) A method for producing 3,7-dioxo-5β-cholanic acid represented by the following formula (8) or ester derivatives thereof: 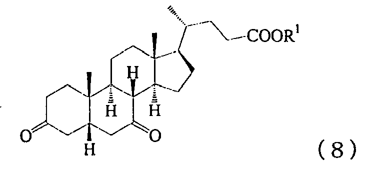 wherein R¹ represents a hydrogen atom, or an alkyl group containing 1 to 6 carbon atoms, which is characterized in that it comprises:
   epoxidizing cholesta-4,6,24-trien-3-one represented by the following formula (4): 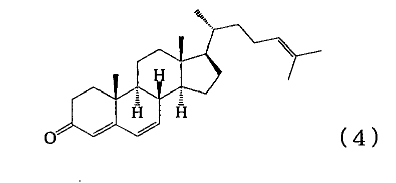
   so as to obtain 6,7:24,25-diepoxycholest-4-en-3-one represented by the following formula (5): 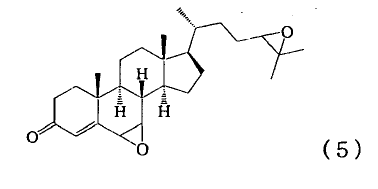
   then hydrogenating the obtained compound, so as to obtain 24,25-epoxy-5β-cholestan-3-one-7-ol represented by the following formula (6): 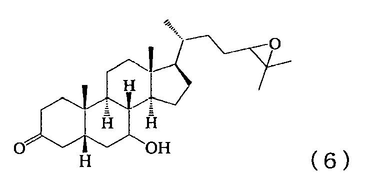
   then oxidizing the obtained compound, so as to obtain 24,25-epoxy-5β-cholestane-3,7-dione represented by the following formula (12): 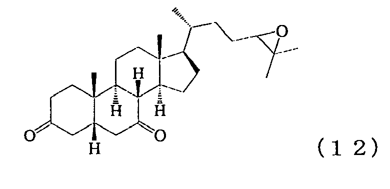
   then hydrolyzing the obtained compound, so as to obtain 5β-cholestane-3,7-dione-24,25-diol represented by the following formula (13): 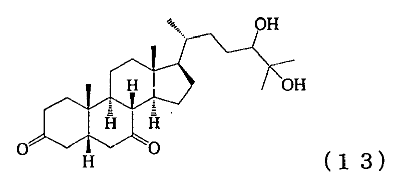
   and then oxidizing the obtained compound, and further esterifying the obtained compound in some cases.
(21) The method for producing 3,7-dioxo-5β-cholanic acid or ester derivatives thereof according to any one of (17) to (20) above, which is characterized in that an organic peroxide is used as an epoxidizing agent.
(22) The method for producing 3,7-dioxo-5β-cholanic acid or ester derivatives thereof according to (21) above, which is characterized in that as an organic peroxide, it uses percarboxylic acid represented by the formula A⁴CO₃H wherein A⁴ represents a hydrogen atom, an alkyl group containing 1 to 20 carbon atoms that may be substituted with a halogen atom, or an aryl group that may have a substituent, periminocarboxylic acid represented by the formula A⁵(C=NH)OOH wherein A⁵ represents a hydrogen atom, an alkyl group containing 1 to 20 carbon atoms that may be substituted with a halogen atom, or an aryl group that may have a substituent, or a dioxirane derivative represented by the following formula (14): 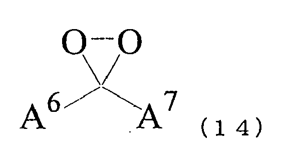 wherein each of A⁶ and A⁷ independently represents an alkyl group containing 1 to 20 carbon atoms that may be substituted with halogen, or A⁶ and A⁷ may bind to each other, so as to form a cyclic structure containing 3 to 8 carbon atoms.
(23) The method for producing 3,7-dioxo-5β-cholanic acid or ester derivatives thereof according to (21) above, which is characterized in that it uses perbenzoic acid or 2-methylperbenzoic acid as an organic peroxide.
(24) The method for producing 3,7-dioxo-5β-cholanic acid or ester derivatives thereof according to (23) above, which is characterized in that water is added during the epoxidation reaction.
(25) The method for producing 3,7-dioxo-5β-cholanic acid or ester derivatives thereof according to (23) above, which is characterized in that the concentration of peracid and that of carboxylic acid are maintained at 0.3 M or less during the epoxidation reaction.
(26) The method for producing 3,7-dioxo-5β-cholanic acid or ester derivatives thereof according to (17), (18), or (20) above, which is characterized in that it comprises halo-esterifying cholesta-4,6,24-trien-3-one represented by the following formula (4): 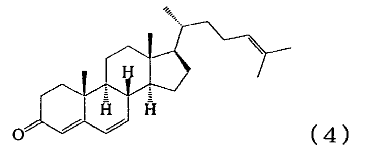
   so as to obtain 7,24-dihalo-cholest-4-en-3-one-6,25-diol diester represented by the following formula (15): 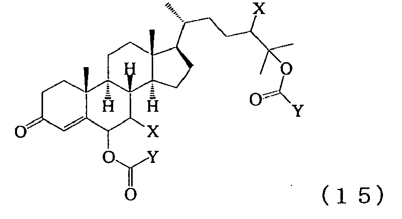 wherein X represents a halogen atom, and Y represents a hydrogen atom, or an alkyl group containing 1 to 10 carbon atoms that may be substituted with halogen, and then performing the alkaline hydrolysis of the ester and cyclization, so as to obtain 6,7:24, 25-diepoxyhcholest-4-en-3-one represented by the following formula (5): 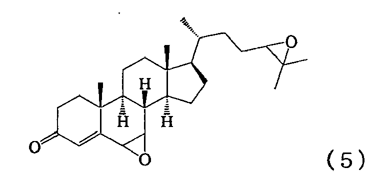
(27) The method for producing 3,7-dioxo-5β-cholanic acid or ester derivatives thereof according to (26) above, which is characterized in that it uses, as halo-esterifying agents, organic carboxylic acid and a halocation generator represented by the formula Z-X wherein X represents a halogen atom, and Z represents succinimide, phthalimide, acetamide, hydantoin, or a t-butoxy group.
(28) The method for producing 3,7-dioxo-5β-cholanic acid or ester derivatives thereof according to any one of (17) to (20) and (26) above, which is characterized in that the hydrogenation reaction is carried out in the presence of a noble metal catalyst.
(29) The method for producing 3,7-dioxo-5β-cholanic acid or ester derivatives thereof according to (28) above, which is characterized in that it uses, as a noble metal catalyst, one or more types of metal palladium selected from the group consisting of powder palladium, and activated carbon-supporting palladium, aluminum oxide-supporting palladium, barium carbonate-supporting palladium, barium sulfate-supporting palladium, and calcium carbonate-supporting palladium, each of which contains 0.5% to 50% by weight of palladium.
(30) The method for producing 3,7-dioxo-5β-cholanic acid or ester derivatives thereof according to (28) or (29) above, which is characterized in that a base is allowed to coexist during the hydrogenation reaction in the presence of a noble metal catalyst.
(31) The method for producing 3,7-dioxo-5β-cholanic acid or ester derivatives thereof according to (30) above, which is characterized in that it uses amines as bases.
(32) The method for producing 3,7-dioxo-5β-cholanic acid or ester derivatives thereof according to any one of (17) to (20) and (26) above, which is characterized in that the hydrolysis reaction of epoxides is carried out in the presence of silica gel or proton acid.
(33) The method for producing 3,7-dioxo-5β-cholanic acid or ester derivatives thereof according to (32) above, which is characterized in that it uses, as proton acid, hydrochloric acid, sulfuric acid, nitric acid, perchloric acid, phosphoric acid, phosphorous acid, hypophosphorous acid, organic carboxylic acids, or organic sulfonic acids.
(34) The method for producing 3,7-dioxo-5β-cholanic acid or ester derivatives thereof according to any one of (17) to (20) and (26) above, which is characterized in that it uses, as an oxidizing agent for the oxidation reaction, oxy-halogen acids or salts thereof, molecular halogen, permanganic acids, dichromic acids, or chromic acids.
(35) The method for producing 3,7-dioxo-5β-cholanic acid or ester derivatives thereof according to any one of (17) to (20) and (26) above, which is characterized in that it uses a compound obtained by isomerizing cholesta-4,7,24-trien-3-one represented by the following formula (3): 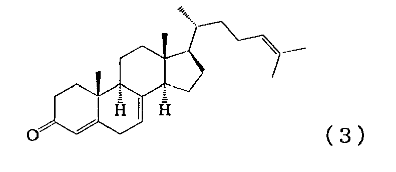
   as cholesta-4,6,24-trien-3-one represented by the following formula (4): 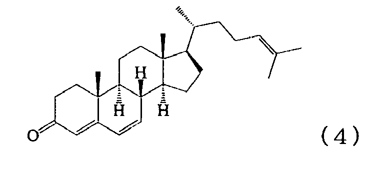
(36) The method for producing 3,7-dioxo-5β-cholanic acid or ester derivatives thereof according to (35) above, which is characterized in that it uses a compound obtained by oxidizing cholesta-5,7,24-trien-3β-ol represented by the following formula (2): 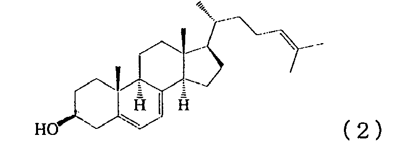
   as cholesta-4,7,24-trien-3-one represented by the following formula (3): 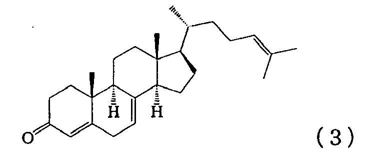
(37) A method for producing a vicinal diol compound represented by the following formula (17): 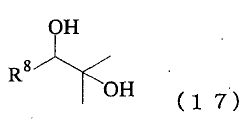 wherein R⁸ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom,
   which is characterized in that an epoxy compound represented by the following formula (16) is hydrolyzed using silica gel as a catalyst: 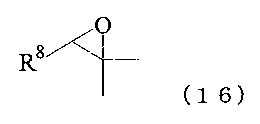 wherein R⁸ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom.
(38) A method for producing a vicinal diol compound represented by the following formula (19): 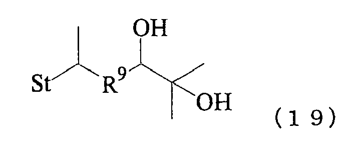 wherein St represents a steroid skeleton consisting of ring A, ring B, ring C, and ring D, and such a steroid skeleton (1) binds to the side chain shown in the formula at position C17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C13, and C14 may be substituted with a methyl group(s); and R⁹ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom;
   the above-described production method being characterized in that a steroid epoxy compound represented by the following formula (18) is hydrolyzed using silica gel as a catalyst: 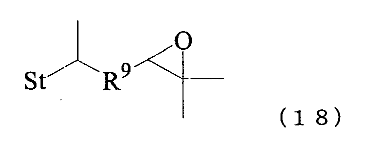 wherein St represents a steroid skeleton consisting of ring A, ring B, ring C, and ring D, and such a steroid skeleton (1) binds to the side chain shown in the formula at position C17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C13, and C14 may be substituted with a methyl group(s); and R⁹ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom.
(39) A 6,7:24,25-diepoxycholest-4-en-3-one represented by the following formula (5): 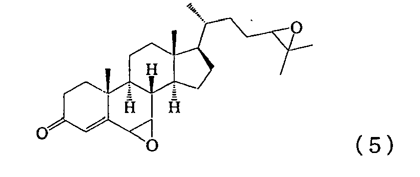
(40) A 24,25-epoxycholesta-4,6-dien-3-one represented by the following formula (10): 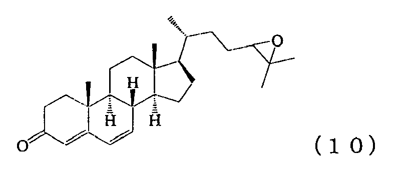
(41) A cholesta-4,6-dien-3-one-24,25-diol represented by the following formula (11): 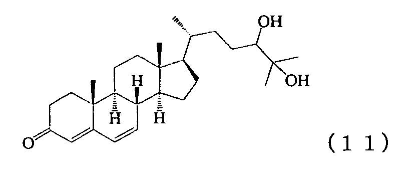
(42) A 24,25-epoxy-5β-cholestan-3-one-7-ol represented by the following formula (6): 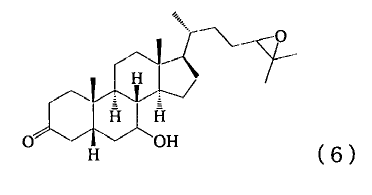
(43) A 5β-cholestan-3-one-7,24,25-triol represented by the following formula (7): 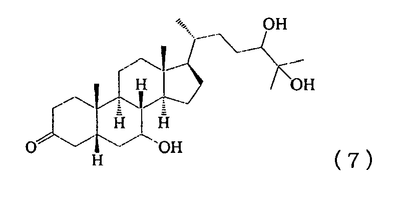
(44) A 6,7-epoxycholest-4-en-3-one-24,25-diol represented by the following formula (9): 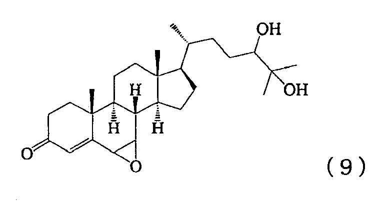
(45) A 24,25-epoxy-5β-cholestane-3,7-dione represented by the following formula (12): 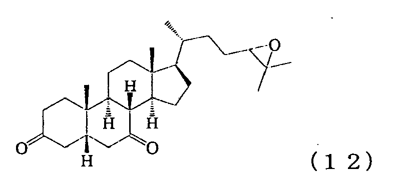
(46) A 5β-cholestane-3,7-dione-24,25-diol represented by the following formula (13): 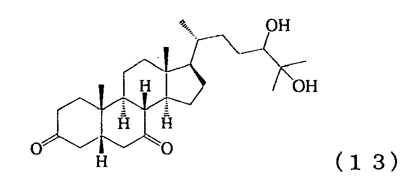
(47) A 7,24-dichloro-cholest-4-en-3-one-6,25-diol diformyl ester represented by the following formula (15a): 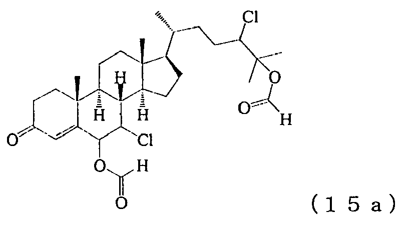
(48) A 24,25-epoxycholest-4-en-3-one-7-ol represented by the following formula (20): 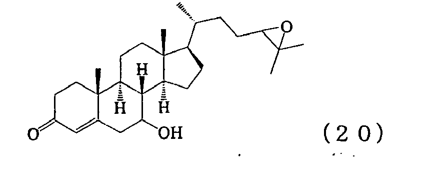
(49) A method for producing ursodeoxycholic acid (21a), chenodeoxycholic acid (21b), 3α-hydroxy-7-oxo-5β-cholanic acid (21c) or 7-hydroxy-3-oxo-5β-cholanic acid (21d), or the ester derivatives of these acids, represented by the following formula (21a), (21b), (21c), or (21d): 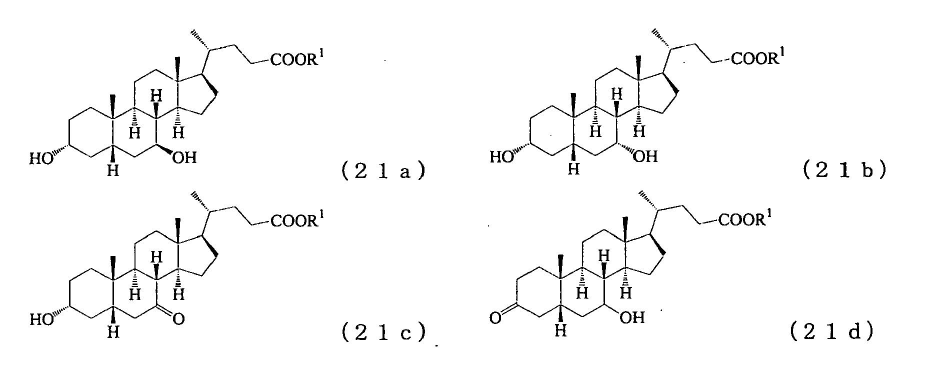 wherein R¹ represents a hydrogen atom, or an alkyl group containing 1 to 6 carbon atoms;
   which is characterized in that the 3,7-dioxo-5β-cholanic acid represented by the following formula (8) or ester derivatives thereof, produced by the method according to any one of (17) to (20), (26), (35), and (36) above, is reduced, and further reoxidized in some cases: 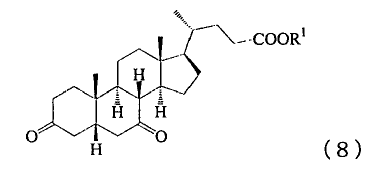 wherein R¹ represents a hydrogen atom, or an alkyl group containing 1 to 6 carbon atoms.

### Best Mode for Carrying out the Invention

Hereafter, the mode for carrying out the invention is described in detail.

Hereafter, the entire scheme of the present invention including reaction steps will be shown below.

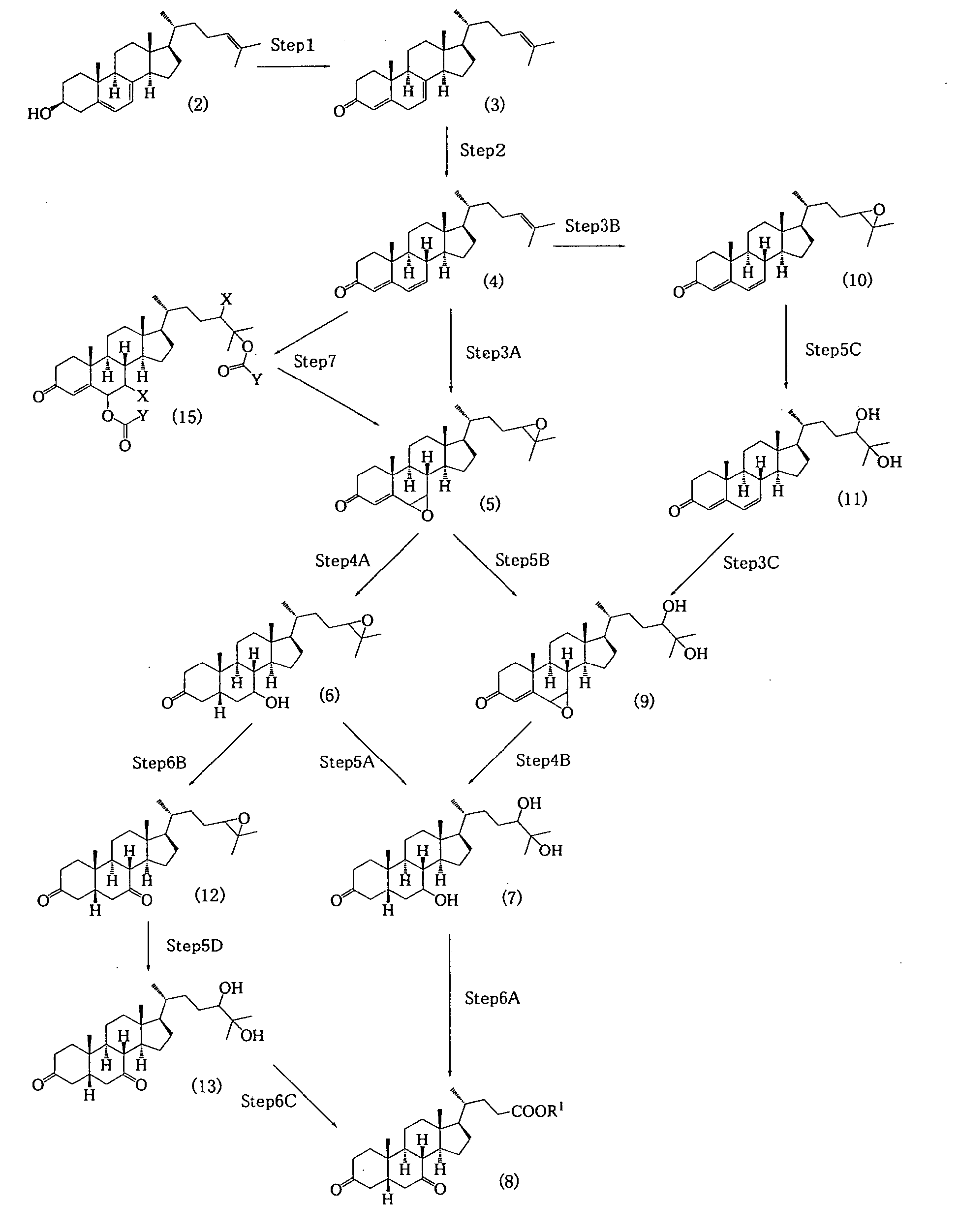

Hereafter, the explanation will be given with reference to Compound Nos. (2) to (15) shown in the above scheme.

A raw material used in the production method of the present invention, cholesta-5,7,24-trien-3β-ol, is a known substance. This compound can be produced, for example, by modifying in a metabolic engineering manner Eumycetes that produce ergosterol via zymosterol, then culturing the thus produced mutant strain, and then collecting cholesta-5,7,24-trien-3β-ol from the culture product. For the details of such a production method, reference can be made to the methods described in Japanese Patent Application Laid-Open Nos. 5-192184 and 2004-141125.

### <Step 1> A step of producing cholesta-4,7,24-trien-3-one represented by the following formula (3) from cholesta-5,7,24-trien-3β-ol represented by the following formula (2)

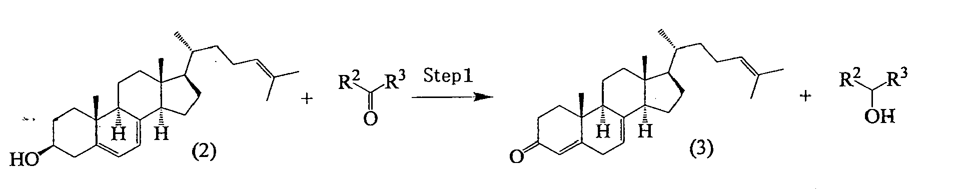

As is clear from the aforementioned reaction formula, in step 1 of the present invention, oxidation of a hydroxyl group at position 3 of cholesta-5,7,24-trien-3β-ol (hereinafter abbreviated as "compound 2" at times) and isomerization of a double bond at position 5 to position 4 are simultaneously carried out. This step has been known as a method of converting ergosterol to ergosteron, and it is called "Oppenauer Oxidation."

This oxidation reaction is carried out using a metal alkoxide as a catalyst and using a ketone compound as a hydrogen acceptor. Examples of a metal alkoxide may include aluminum isopropoxide, aluminum-t-butoxide, magnesium ethoxide, magnesium propoxide, and titanium propoxide. A preferred example of a ketone compound may be a compound represented by the formula R²(C=O)R³ wherein each of R² and R³ independently represents a chain or cyclic alkyl group containing 1 to 10 carbon atoms, or R² and R³ may bind to each other, so as to form a cyclic structure containing 3 to 8 carbon atoms. Specific examples of such a ketone compound may include: chain ketones such as acetone or methyl isobutyl ketone; and cyclic ketones such as cyclohexanone or cyclopentanone. As a particularly preferred catalyst, aluminum isopropoxide is used. As a particularly preferred ketone compound, cyclohexanone or methyl isobutyl ketone is used. Such a catalyst is used at a molar ratio generally between 0.1 : 1 and 20 : 1, and preferably between 0.2 : 1 and 0.5 : 1, with respect to the "compound 2." The reaction hardly progresses with no catalysts. On the other hand, if the amount of a catalyst is too large, side reactions frequently occur. Thus, the amount of a catalyst is determined within the aforementioned range. A hydrogen acceptor is used at a molar ratio generally between 1 : 1 and 50 : 1, and preferably between 2 : 1 and 10 : 1, with respect to the "compound 2."

The present reaction can be carried out with no solvents. However, a solvent may also be used. Examples of a solvent used herein may include: aliphatic hydrocarbons such as hexane; aromatic hydrocarbons such as toluene; halogen solvents such as dichloromethane; ethers such as diethyl ether or tetrahydrofuran; and aprotic polar solvents such as dimethyl sulfoxide or dimethyl formamide. Aprotic solvents are preferably used, and toluene or heptane is more preferably used. The reaction temperature is set generally between 90°C and 130°C, and preferably between 100°C and 120°C. The reaction time is approximately between 1 and 3 hours. After completion of the reaction, the reaction product is cooled to room temperature, and water is then added thereto, so as to inactivate the catalyst. The deposited precipitate is filtrated, and the filtrate is then concentrated, so as to obtain a product of interest, cholesta-4,7,24-trien-3-one (compound 3). The compound 3 can be isolated and purified by methods such as silica gel column chromatography or crystallization.

In addition, from the viewpoint of the stability of a product, the present reaction is preferably carried out while oxygen is blocked. For example, a solvent or a ketone compound has previously been subjected to a deoxygenation treatment, and the reaction is then carried out in a nitrogen or argon atmosphere. Specifically, a method of deaerating a solvent and a ketone compound under a reduced pressure for nitrogen substitution, or a method of heating to reflux in a nitrogen atmosphere for nitrogen substitution, is applied, and thereafter, the reaction is carried out in a nitrogen atmosphere.

The isomerization reaction of the present invention can also be applied to relative compounds of the compound 2, that are, 3-hydroxy-5,7-diene steroid compounds represented by the following formulas (2a), (2b), (2c), (2d), and (2e): 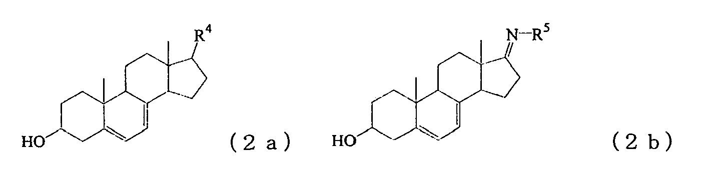 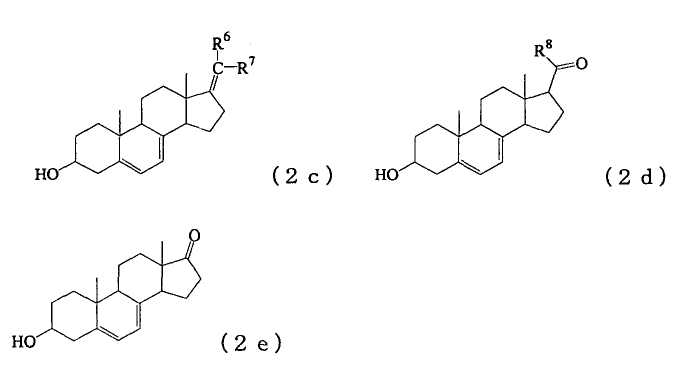 wherein each of R⁴ to R⁸ independently represents a hydrogen atom, a protected hydroxyl group or halogen atom, or an alkyl, alkenyl, or alkynyl group containing 1 to 10 carbon atoms, which may be substituted with a carbonyl group, an ether group, a protected hydroxyl group, a halogen atom, or a carboxyl group.

That is to say, these 3-hydroxy-5,7-diene steroid compounds are allowed to react in the presence of a ketone compound and a metal alkoxide, while oxygen is blocked, so that they are oxidized to compounds represented by the following formulas (3a), (3b), (3c), (3d), and (3e), respectively, at high yields: 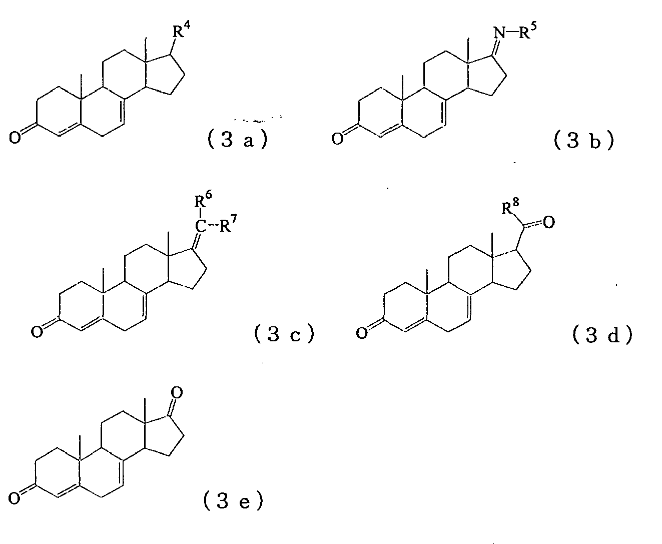 wherein each of R⁴ to R⁸ independently represents a hydrogen atom, a protected hydroxyl group or halogen atom, or an alkyl, alkenyl, or alkynyl group containing 1 to 10 carbon atoms, which may be substituted with a carbonyl group, an ether group, a protected hydroxyl group, a halogen atom, or a carboxyl group.

Specific examples of the aforementioned compound (2a) may include cholesta-5,7,24-trien-3β-ol and ergosterol.

### <Step 2> A step of producing cholesta-4,6,24-trien-3-one represented by the following formula (4) from cholesta-4,7,24-trien-3-one represented by the following formula (3):

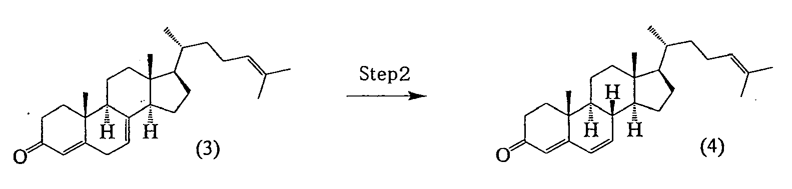

As is clear from the above reaction formula, step 2 of the present invention involves a reaction of isomerizing a double bond at position 7 of cholesta-4,7,24-trien-3-one (hereinafter abbreviated as "compound 3" at times) to position 6.

The isomerization reaction is carried out using a basic compound as a catalyst. Examples of such a basic compound may include alkaline metal hydroxide, alkaline-earth metal hydroxide, alkaline metal carbonate, alkaline-earth metal carbonate, alkaline metal hydrogencarbonate, alkaline metal acetate, alkaline-earth metal acetate, alkaline metal alkoxide, and alkaline-earth metal alkoxide. Of these, alkaline metal hydroxide is preferably used, and potassium hydroxide and sodium hydroxide are more preferably used. Such a basic compound is used at a molar ratio generally between 1 : 1 and 20 : 1, and preferably between 2 : 1 and 10 : 1, with respect to the "compound 3."

The type of a reaction solvent used herein is not particularly limited. Examples of a reaction solvent may include: aliphatic hydrocarbons such as hexane; aromatic hydrocarbons such as toluene; halogen solvents such as dichloromethane; ethers such as diethyl ether or tetrahydrofuran; alcohols such as methanol or ethanol; and aprotic polar solvents such as dimethyl sulfoxide or dimethyl formamide. Preferably, methanol is used. The reaction is carried out generally between 40°C and 80°C, and preferably between 50°C and 70°C, for approximately 5 to 10 hours. After completion of the reaction for a certain period of time, acid is added to the reaction solution to neutralize the base used as a catalyst, and the reaction is terminated. The type of acid used herein is not particularly limited. Examples of acid used herein may include: mineral acids such as hydrochloric acid or sulfuric acid; organic carboxylic acids such as formic acid or acetic acid; and organic sulfonic acids such as p-toluenesulfonic acid. After completion of neutralization, the solvent is distilled away under a reduced pressure, so as to obtain a product of interest, cholesta-4,6,24-trien-3-one (hereinafter abbreviated as "compound 4" at times). According to circumstances, water may be added to the reaction solution obtained after the neutralization treatment, so as to crystallize compound 4. Otherwise, an organic solvent may be added thereto for extraction, and the organic solvent layer may be washed with water, dried, and concentrated, followed by isolation and purification by silica gel column chromatography or other methods.

In addition, from the viewpoint of the stability of a substrate, the present reaction is preferably carried out while oxygen is blocked. For example, a solvent has previously been subjected to a deoxygenation treatment, and the reaction is then carried out in a nitrogen or argon atmosphere. Specifically, a method of deaerating a solvent under a reduced pressure for nitrogen substitution, or a method of heating to reflux in a nitrogen atmosphere for nitrogen substitution, is first applied, and thereafter, the reaction is carried out in a nitrogen atmosphere.

The isomerization reaction of the present invention can also be applied to relative compounds of the compound 3, that are, 3-oxo-4,7-diene steroid compounds represented by the following formulas (3a), (3b), (3c), (3d), and (3e): 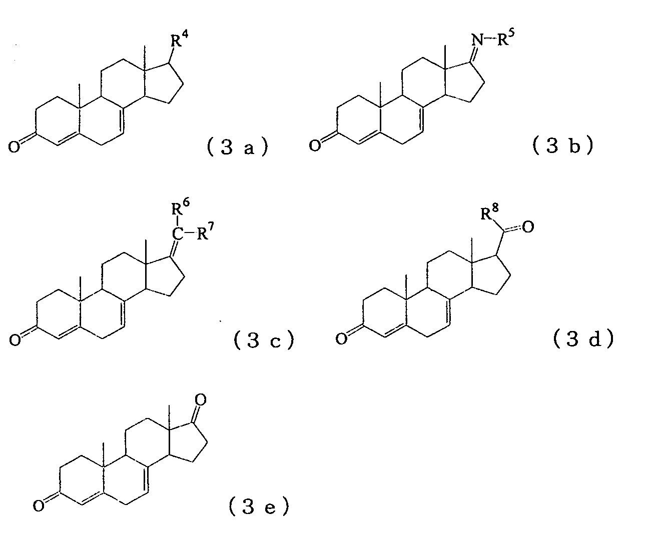 wherein each of R⁴ to R⁸ independently represents a hydrogen atom, a hydroxyl group, a protected hydroxyl group or halogen atom, or an alkyl, alkenyl, or alkynyl group containing 1 to 10 carbon atoms, which may be substituted with a carbonyl group, an ether group, a hydroxyl group, a protected hydroxyl group, a halogen atom, or a carboxyl group.

That is to say, these 3-oxo-4,7-diene steroid compounds can be isomerized to 3-oxo-4,6-diene steroid compounds represented by the following formulas (4a), (4b), (4c), (4d), and (4e), respectively, using a base as a catalyst: 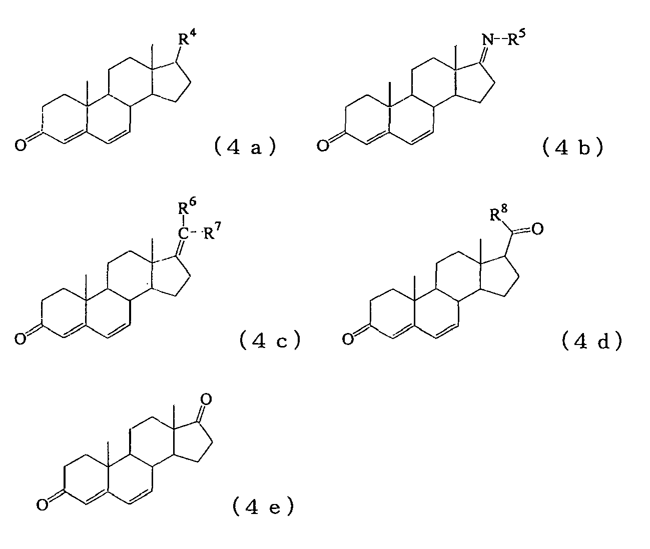 wherein each of R⁴ to R⁸ independently represents a hydrogen atom, a hydroxyl group, a protected hydroxyl group or halogen atom, or an alkyl, alkenyl, or alkynyl group containing 1 to 10 carbon atoms, which may be substituted with a carbonyl group, an ether group, a hydroxyl group, a protected hydroxyl group, a halogen atom, or a carboxyl group.

An example of the aforementioned protected hydroxyl group may be a hydroxyl group protected with an ether-type protecting group.

### <Step 3A> A step of producing 6,7:24,25-diepoxycholest-4-en-3-one represented by the following formula (5) from cholesta-4,6,24-trien-3-one represented by the following formula (4)

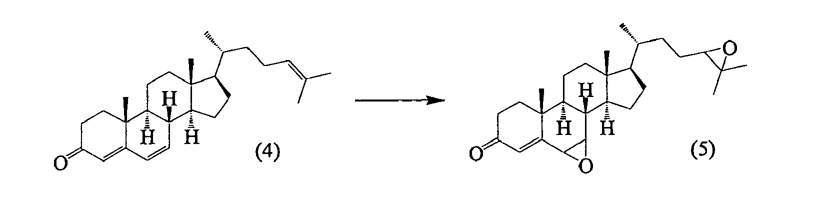

As is clear from the above reaction formula, step 3 of the present invention involves a reaction of epoxidizing double bonds at positions 6 and 24 of cholesta-4,6,24-trien-3-one (hereinafter abbreviated as "compound 4" at times). As an epoxidizing agent, an organic peroxide is generally used.

Examples of an organic peroxide used herein may include: percarboxylic acid represented by the formula A⁴CO₃H wherein A⁴ represents a hydrogen atom, an alkyl group containing 1 to 20 carbon atoms that may be substituted with a halogen atom, or an aryl group that may have a substituent; periminocarboxylic acid represented by the formula A⁵(C=NH)OOH wherein A⁵ represents a hydrogen atom, an alkyl group containing 1 to 20 carbon atoms that may be substituted with a halogen atom, or an aryl group that may have a substituent; and a dioxirane derivative represented by the following formula (14): 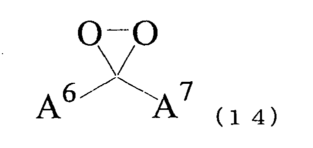 wherein each of A⁶ and A⁷ independently represents an alkyl group containing 1 to 20 carbon atoms that may be substituted with halogen, or A⁶ and A⁷ may bind to each other, so as to form a cyclic structure containing 3 to 8 carbon atoms. Specific examples of percarboxylic acid may include performic acid, peracetic acid, perpropionic acid, perbenzoic acid, 2-methylperbenzoic acid, and monoperphthalic acid. A specific example of periminocarboxylic acid may be CH₃C(=NH)OOH (peroxyacetimidic acid). Specific examples of a dioxirane derivative may include dimethyldioxirane (acetone peroxide) and methyl ethyl dioxirane (methyl ethyl ketone peroxide).

From the viewpoint of reaction selectivity, perbenzoic acid and 2-methylperbenzoic acid are particularly preferably used.

Such an organic peroxide is used at a molar ratio generally between 2 : 1 and 10 : 1, and preferably between 2 : 1 and 3 : 1, with respect to the "compound 4." The temperature applied for epoxidation is set generally between 0°C and 100°C, and preferably between 40°C and 90°C.

The type of a reaction solvent used herein is not particularly limited. Examples of a reaction solvent may include: aliphatic hydrocarbons such as hexane; aromatic hydrocarbons such as toluene; halogen solvents such as dichloromethane; ethers such as diethyl ether or tetrahydrofuran; esters such as ethyl acetate or butyl acetate; nitriles such as acetonitrile; alcohols such as methanol or ethanol; aprotic polar solvents such as dimethyl sulfoxide or dimethyl formamide; and water. Of these, esters are preferably used. When percarboxylic acid is used as an oxidizing agent, reaction selectivity is significantly improved by adding water, or by maintaining the concentration of peracid and that of carboxylic acid at low in a reaction solution.

The obtained 6,7:24,25-diepoxycholest-4-en-3-one (compound 5) can be isolated and purified by methods such as silica gel column chromatography or crystallization.

### <Step 3B> A step of producing 24,25-epoxycholesta-4,6-dien-3-one represented by the following formula (10) from cholesta-4,6,24-trien-3-one represented by the following formula (4)

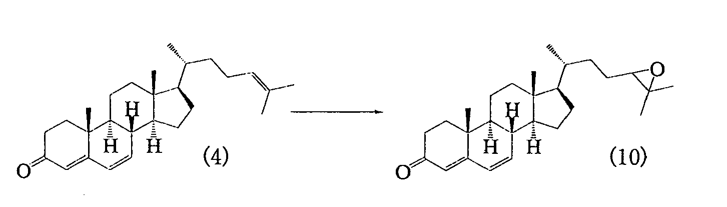

As is clear from the above reaction formula, step 3B of the present invention involves a reaction of epoxidizing a double bond at position 24 of cholesta-4,6,24-trien-3-one (compound 4).

In the epoxidation reaction of step 3A, the double bond at position 24 is preferentially epoxidized, and the double bond at position 6 is then epoxidized. Thus, if the amount of an epoxidizing agent used is small, or if the reaction temperature is low, only the double bond at position 24 shown in the formula (4) is epoxidized, so as to obtain a monoepoxy compound (10). Accordingly, it may be adequate that an epoxidizing agent be used at a molar ratio of 1 : 1 with respect to the "compound 4," and that the reaction be carried out around room temperature for 1 to 3 hours. Other reaction conditions are the same as those in step 3A.

### <Step 3C> A step of producing 6,7-epoxycholest-4-en-3-one-24,25-diol represented by the following formula (9) from cholesta-4,6-dien-3-one-24,25-diol represented by the following formula (11)

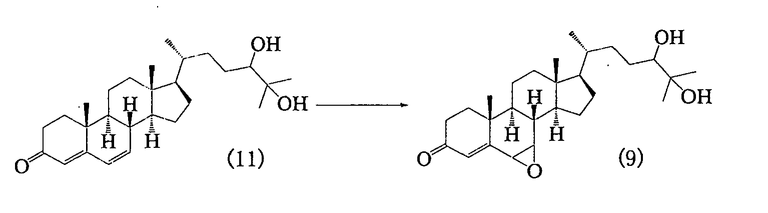

As is clear from the above reaction formula, step 3C of the present invention involves a reaction of epoxidizing a double bond at position 6 of cholesta-4,6-dien-3-one-24,25-diol (compound 11).

The present reaction is the same as that in the aforementioned step 3A in that it is a reaction of epoxidizing a double bond at position 6 of a 3-keto-4,6-diene steroid compound. Accordingly, the same oxidizing agent and solvent as those in the case of step 3A can be used. Reaction conditions are also the same as those in step 3A. An epoxidizing agent is preferably used at a molar ratio between 1 : 1 and 2 : 1 with respect to the "compound 11."

### <Step 7> A step of producing 6,7:24,25-diepoxycholest-4-en-3-one represented by the following formula (5) from cholesta-4,6,24-trien-3-one represented by the following formula (4)

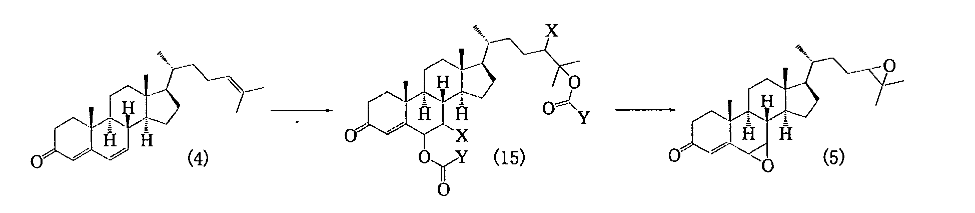

wherein X represents a halogen atom; and Y represents a hydrogen atom, or an alkyl group containing 1 to 10 carbon atoms that may be substituted with halogen.

As shown in the above formula, as an alternative method of obtaining the aforementioned diepoxy compound (5), it is also possible to apply a method involving the combination of the hydrolysis of an ester with cyclization to an epoxide, wherein the reaction is performed via a haloester.

For such halo-esterification, organic carboxylic acid and a halocation generator represented by the formula Z-X wherein X represents a halogen atom, and Z represents succinimide, phthalimide, acetamide, hydantoin, or a t-butoxy group are used. Preferably, formic acid is used as organic carboxylic acid, and t-butyl hypochloride is used as a halocation generator.

Such organic carboxylic acid is used at a molar ratio generally between 2 : 1 and 50 : 1, and preferably between 2 : 1 and 10 : 1, with respect to the "compound 4." Such a halocation generator is used at a molar ratio generally between 2 : 1 and 10 : 1, and preferably between 2 : 1 and 5:1, with respect to the "compound 4."

The reaction temperature is generally between 0°C and 50°C, and preferably between 0°C and 30°C. Examples of a reaction solvent used herein may include: aliphatic hydrocarbons such as hexane; aromatic hydrocarbons such as toluene; halogen solvents such as dichloromethane; ethers such as diethyl ether or tetrahydrofuran; esters such as ethyl acetate or butyl acetate; nitriles such as acetonitrile; ketones such as acetone; and organic carboxylic acids such as acetic acid or formic acid.

Moreover, for the hydrolysis of an ester in the obtained haloester and the cyclization to an epoxy group, a base is used. Examples of a base used herein may include hydroxide, carbonate and alkoxide of an alkaline metal or alkaline-earth metal. Such a base is used at a molar ratio generally between 2 : 1 and 50 : 1, and preferably between 4 : 1 and 10 : 1, with respect to the "compound 15." The reaction temperature is generally between 0°C and 50°C, and preferably between 0°C and 30°C. The type of a reaction solvent used herein is not particularly limited. Preferred examples of a reaction solvent may include: alcohols such as methanol or ethanol; ketones such as acetone; nitriles such as acetonitrile; and water.

### <Step 4A> A step of producing 24,25-epoxy-5β-cholestan-3-one-7-ol represented by the following formula (6) from 6,7:24,25-diepoxycholest-4-en-3-one represented by the following formula (5)

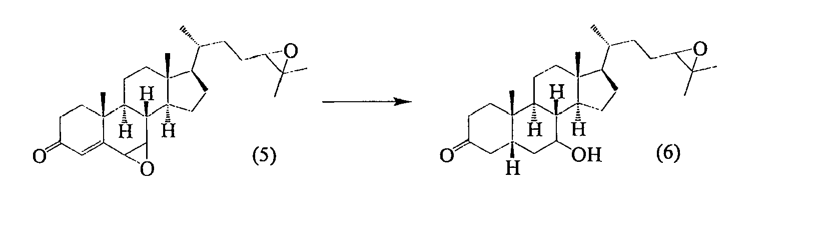

As is clear from the above reaction formula, step 4A of the present invention involves the hydrogenation (reduction) of a double bond at position 4 of 6,7:24,25-diepoxycholest-4-en-3-one (compound 5) and the reductive cleavage of a carbon-oxygen bond at position 6. Hydrogenation is carried out using hydrogen in the presence of a noble metal catalyst such as palladium, platinum, or ruthenium. Examples of a palladium catalyst used herein may include powder palladium, activated carbon-supporting palladium, aluminum oxide-supporting palladium, barium carbonate-supporting palladium, barium sulfate-supporting palladium, and calcium carbonate-supporting palladium, each of which contains 0.5% to 50% by weight of palladium. A noble metal catalyst is used at a molar ratio generally between 0.005 : 1 and 0.5 : 1 with respect to the "compound 5." A hydrogen pressure is not particularly limited. The reaction is generally carried out under a pressure of 1 MPa or less. Examples of a solvent used herein may include alcohols, ethers, esters, and aliphatic or aromatic hydrocarbons, but examples are not limited thereto. From the viewpoint of selectivity, it is preferable that a base be allowed to coexist in the present reaction. Preferred examples of a base used herein may include pyridine and amines such as triethylamine, tetramethylethylenediamine, or diisopropylamine. Such a base is used at a molar ratio generally between 0.1 : 1 and 100 : 1 with respect to the "compound 5." The reaction temperature is generally between 0°C and 50°C, and preferably between 0°C and 20°C.

After filtration of the catalyst, the obtained 24,25-epoxy-5β-cholestan-3-one-7-ol (compound 6) can be isolated and purified by methods such as silica gel column chromatography or crystallization.

### <Step 5A> A step of producing 5β-cholestan-3-one-7,24,25-triol represented by the following formula (7) from 24,25-epoxy-5β-cholestan-3-one-7-ol represented by the following formula (6)

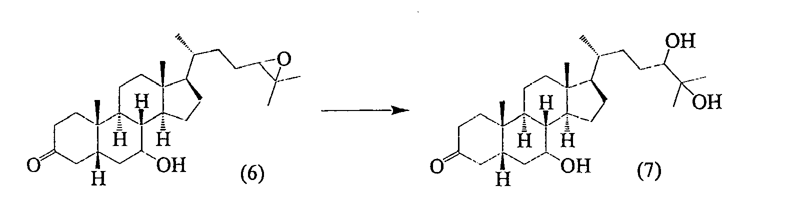

As is clear from the above reaction formula, step 5A of the present invention involves the hydrolysis of 24,25-epoxy group of 24,25-epoxy-5β-cholestan-3-one-7-ol (compound 6) to 24,25-vicinal diol.

The hydrolysis reaction is carried out by allowing water to react with the compound in the presence of a catalyst. Examples of a catalyst used herein may include proton acid and silica gel. Examples of such proton acid may include hydrochloric acid, sulfuric acid, nitric acid, perchloric acid, phosphoric acid, phosphorous acid, hypophosphorous acid, organic carboxylic acids, and organic sulfonic acids. The reaction temperature is generally between 10°C and 60°C, and particularly preferably room temperature. The reaction mixture is stirred at the aforementioned temperature for approximately 1 to 4 hours, and if necessary alkaline neutralization is carried out, so as to separate a product of interest.

The type of a reaction solvent is not particularly limited. Esters, ethers, nitriles, and other solvents can be used. Preferred examples of a solvent used may include ethyl acetate, tetrahydrofuran, and acetonitrile. When proton acid is used as a catalyst, the catalyst is used at a molar ratio between approximately 0.01 : 1 and 2 : 1 with respect to the "compound 6." It is also possible to obtain compound 7, wherein an epoxy group at positions 24 and 25 of compound 6 are hydrolyzed, by supplying an organic solvent solution of compound 6 to gel-state silica, so as to allow the compound 6 to adsorb on it, and then by supplying the organic solvent again.

The obtained 5β-cholestan-3-one-7,24,25-triol (compound 7) can be isolated and purified by methods such as silica gel column chromatography or crystallization.

### <Step 5B> A step of producing 6,7-epoxycholest-4-en-3-one-24,25-diol represented by the following formula (9) from 6,7:24,25-diepoxycholest-4-en-3-one represented by the following formula (5)

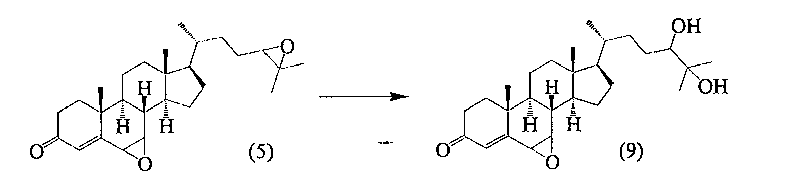

As is clear from the above reaction formula, step 5B of the present invention involves the hydrolysis of 24,25-epoxy group of 6,7:24,25-diepoxycholest-4-en-3-one (compound 5) to 24,25-vicinal diol.

The present reaction is the same as that in the aforementioned step 5A in that it is the hydrolysis reaction of side chain epoxy group of a steroid compound. Accordingly, the same catalyst and solvent as those in the case of step 5A can be used, and reaction conditions are also the same.

### <Step 5C> A step of producing cholesta-4,6-dien-3-one-24,25-diol represented by the following formula (11) from 24,25-epoxycholesta-4,6-dien-3-one represented by the following formula (10)

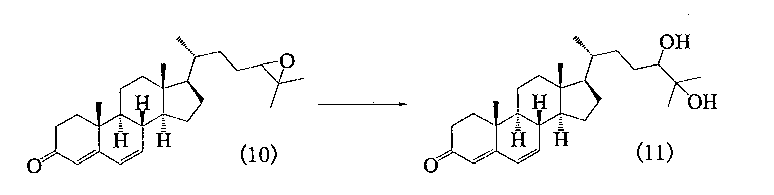

As is clear from the above reaction formula, step 5C of the present invention involves the hydrolysis of 24,25-epoxy group of 24,25-epoxycholesta-4,6-dien-3-one (compound 10) to 24,25-vicinal diol.

The present reaction is the same as that in the aforementioned step 5A in that it is the hydrolysis reaction of side chain epoxy group of a steroid compound. Accordingly, the same catalyst and solvent as those in the case of step 5A can be used, and reaction conditions are also the same.

### <Step 5D> A step of producing 5β-cholestane-3,7-dione-24,25-diol represented by the following formula (13) from 24,25-epoxy-5β-cholestane-3,7-dione represented by the following formula (12)

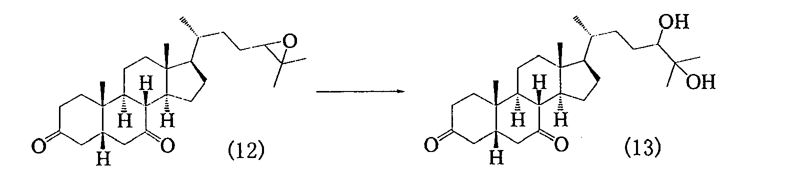

As is clear from the above reaction formula, step 5D of the present invention involves the hydrolysis of 24,25-epoxy group of 24,25-epoxy-5β-cholestane-3,7-dione (compound 12) to 24,25-vicinal diol.

The present reaction is the same as that in the aforementioned step 5A in that it is the hydrolysis reaction of side chain epoxy group of a steroid compound. Accordingly, the same catalyst and solvent as those in the case of step 5A can be used, and reaction conditions are also the same.

### <Step 4B> A step of producing 5β-cholestan-3-one-7,24,25-triol represented by the following formula (7) from 6,7-epoxycholest-4-en-3-one-24,25-diol represented by the following formula (9)

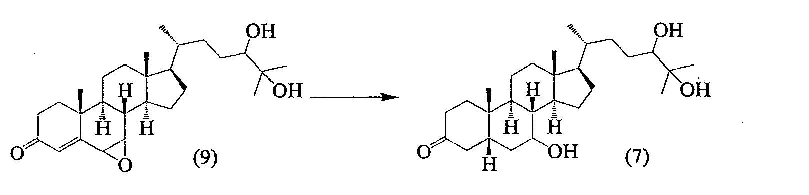

As is clear from the above reaction formula, step 4B of the present invention involves the hydrogenation (reduction) of a double bond at position 4 of 6,7-epoxycholest-4-en-3-one-24,25-diol (compound 9) and the reductive cleavage of a carbon-oxygen bond at position 6 thereof.

The present reaction is the same as that in the aforementioned step 4A in that it is the hydrogenation reaction of 6,7-epoxy group of steroid ring B. Accordingly, the same noble metal catalyst, base, and solvent as those in the case of step 4A can be used, and reaction conditions are also the same.

### <Step 6A> A step of producing 3,7-dioxo-5β-cholanic acid represented by the formula (8) or ester derivatives thereof from 5β-cholestan-3-one-7,24,25-triol represented by the following formula (7)

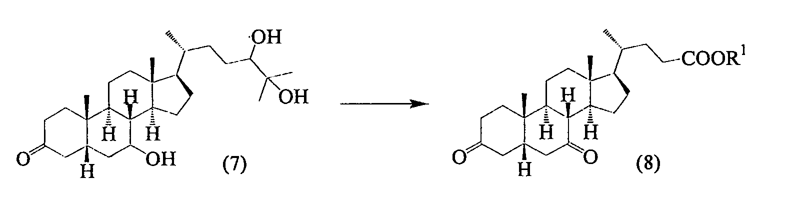

As is clear from the above reaction formula, step 6A of the present invention involves the oxidative cleavage of a 24,25-diol portion of 5β-cholestan-3-one-7,24,25-triol and oxidation of a hydroxyl group at position 7, and the esterification reaction if necessary.

Examples of an oxidizing agent used herein may include oxo-halogen acids or salts thereof, molecular halogen, permanganic acids, dichromic acids, and chromic acids. Examples of such oxo-halogen acids may include hypohalogenous acid, halogenous acid, halogenic acid and perhalogenic acid of chlorine, bromine and iodine. Examples of salts of such oxo-halogen acids may include: salts of alkaline metal such as lithium, potassium or sodium; and salts of alkaline-earth metal such as calcium or magnesium. Preferably, hypohalogenous acid or a salt thereof is used. More preferably, calcium hypochlorite or sodium hypochlorite is used. In addition, examples of an oxidizing agent used herein may include molecular halogen such as chlorine gas or bromine gas. In some cases, oxo-halogen acids or salts thereof can be used with the combination of molecular halogen. As permanganic acids, potassium permanganate is used. As dichromic acids, dichromic acid or a pyridine salt thereof is used. As chromic acids, chromic acid or a pyridine salt thereof is used.

This oxidation reaction can be carried out, using an oxidizing agent at a molar ratio between 3 : 1 and 20 : 1, and preferably between 3 : 1 and 6 : 1, with respect to the "compound 7," in the presence of a solvent such as ketones, esters, nitriles, ethers, halogenated aliphatic hydrocarbons, or halogenated aromatic hydrocarbons, at a temperature between 0°C and 100°C, and preferably between 0°C and 30°C.

5β-3,7-dioxocholanic acid (compound 8 wherein R¹ is a hydrogen atom) obtained by the present oxidation method can be isolated and purified by methods such as silica gel column chromatography or crystallization.

Moreover, carboxylic acid at position 24 is then esterified by a known method, so as to induce the above carboxylic acid to an ester derivative thereof (compound 8 wherein R¹ is an alkyl group containing 1 to 6 carbon atoms), and it can be then isolated and purified by methods such as silica gel column chromatography or crystallization, as described above. Examples of alcohol used for esterification may include linear, branched, and cyclic alcohols, such as methanol, ethanol, n-butanol, t-butanol, or cyclohexanol. Of these, methanol is preferable. The reaction can easily be carried out by heating in alcohol in the presence of an acid catalyst such as sulfuric acid or p-toluenesulfonic acid. Otherwise, the compound may also be esterified in an organic solvent other than alcohol, using dialkyl sulfate and a base (for example, potassium carbonate).

### <Step 6B> A step of producing 24,25-epoxy-5β-cholestane-3,7-dione represented by the following formula (12) from 24,25-epoxy-5β-cholestan-3-one-7-ol represented by the following formula (6)

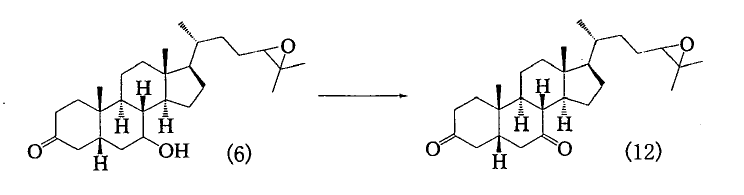

As is clear from the above reaction formula, step 6B of the present invention involves oxidation of a hydroxyl group at position 7 of 24,25-epoxy-5β-cholestan-3-one-7-ol (compound 6) to ketone.

The present reaction is the same as that in the aforementioned step 6A in that it is a reaction of oxidizing a hydroxyl group at position 7 of a steroid compound to ketone. Accordingly, the same oxidizing agent and solvent as those in the case of step 6A can be used, and reaction conditions are also the same. The necessary amount of an oxidizing agent is at a molar equivalence ratio between 1 : 1 and 5 : 1, and preferably between 1 : 1 and 2 : 1, with respect to the "compound 6."

### <Step 6C> A step of producing 3,7-dioxo-5β-cholanic acid represented by the formula (8) or ester derivatives thereof from 5β-cholestane-3,7-dione-24,25-diol represented by the following formula (13)

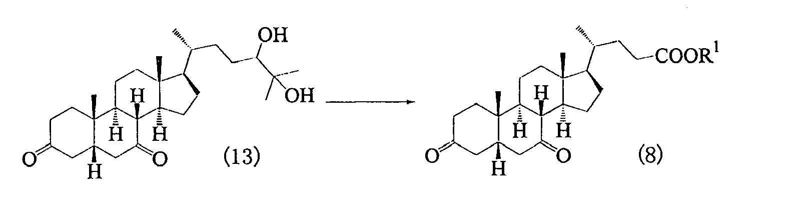

As is clear from the above reaction formula, step 6C of the present invention involves the oxidative cleavage of a 24,25-diol portion of 5β-cholestane-3,7-dione-24,25-diol (compound 13).

The present reaction is the same as that in the aforementioned step 6A in that it is an oxidative cleavage reaction of a 24,25-diol portion of a steroid compound. Accordingly, the same oxidizing agent and solvent as those in the case of step 6A can be used, and reaction conditions are also the same. The necessary amount of an oxidizing agent is at a molar equivalence ratio between 3 : 1 and 10 : 1, and preferably between 2 : 1 and 3 : 1, with respect to the "compound 13."

The method for producing 5β-3,7-dioxocholanic acid or ester derivatives thereof of the present invention is as described above.

The hydrolysis reaction of epoxides of the present invention is extremely useful for conversion of the compound 6 to the compound 7 described in the aforementioned Step 5A, conversion of the compound 5 to the compound 9 described in the aforementioned Step 5B, conversion of the compound 10 to the compound 11 described in the aforementioned Step 5C, and conversion of the compound 12 to the compound 13 described in the aforementioned Step 5D. Furthermore, the above hydrolysis reaction can also be applied to an epoxy compound represented by the following formula (16): 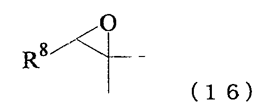 (wherein R⁸ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom). In addition, the hydrolysis reaction can also be applied to a steroid epoxy compound represented by the following formula (18): 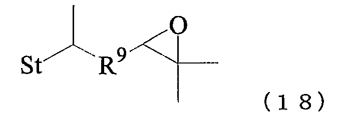 (wherein St represents a steroid skeleton consisting of ring A, ring B, ring C, and ring D, and such a steroid skeleton (1) binds to the side chain shown in the formula at position C17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C 13, and C14 may be substituted with a methyl group(s); and R⁹ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom).

That is to say, the aforementioned epoxy compound can be converted to a vicinal diol compound represented by the following formula (17): 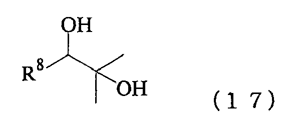 (wherein R⁸ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom). In addition, the aforementioned steroid epoxy compound can be converted to a vicinal diol compound represented by the following formula (19): 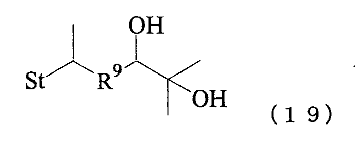 (wherein St represents a steroid skeleton consisting of ring A, ring B, ring C, and ring D, and such a steroid skeleton (1) binds to the side chain shown in the formula at position C17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C13, and C14 may be substituted with a methyl group(s); and R⁹ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom).

An example of the aforementioned epoxy compound represented by the formula (16) may be an epoxy compound derived from citronellol. Examples of the aforementioned steroid epoxy compound represented by the formula (18) may include 24,25-epoxycholesta-4,6-dien-3-one and 24,25-epoxycholest-4-en-3-one. The method of the present invention is applied to these epoxy compounds, so that vicinal diol can also be advantageously produced.

3,7-dioxo-5β-cholanic acid or ester derivatives thereof (compound 8) obtained by the method of the present invention is an intermediate of steroid medicaments. When the compound 8 is reduced by a known method, it can be converted to ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formula (21a), (21b), (21c), or (21d): 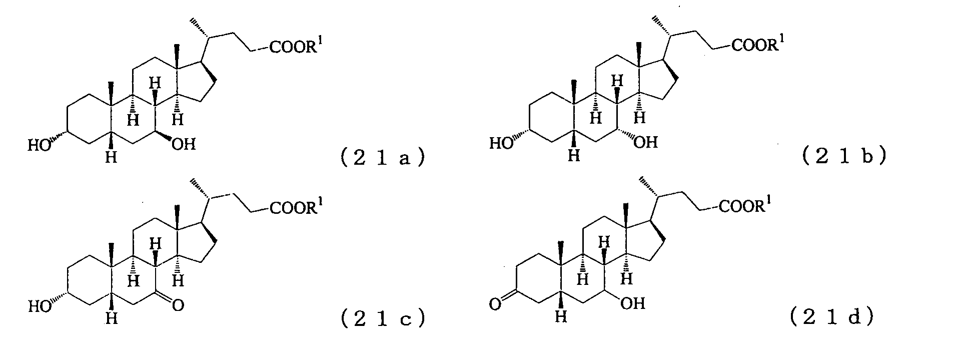 (wherein R¹ represents a hydrogen atom, or an alkyl group containing 1 to 6 carbon atoms).

Examples of a reduction method may include: a method of allowing the compound to react with hydrogen in the presence of a catalyst such as nickel (in particular, Raney nickel), cobalt, or copper-chromium, preferably in the coexistence of alkali such as sodium hydroxide, using, as a solvent, water, methanol, ethanol, tetrahydrofuran, or the like (catalytic hydrogenation method); and a method of allowing the compound to react with alkaline metal in alcohol (metal reduction method). In addition, a method of using a specific organic boron compound at an extremely low temperature of around -45°C, using tetrahydrofuran as a solvent (hydride reduction method using K-selectride) can also be applied.

For example, the following reaction steps can be used:
(1) 3,7-dioxo-5β-cholanic acid → (metal reduction or catalytic hydrogenation) → ursodeoxycholic acid
(2) 3,7-dioxo-5β-cholanic acid → (catalytic hydrogenation) → 3α-hydroxy-7-oxo-5β-cholanic acid
(3) 3α-hydroxy-7-oxo-5β-cholanic acid → (metal reduction) → ursodeoxycholic acid
(4) 3α-hydroxy-7-oxo-5β-cholanic acid → (hydride reduction) → chenodeoxycholic acid
(5) chenodeoxycholic acid → (silver carbonate oxidation) → 7-hydroxy-3-oxo-5β-cholanic acid

These methods are described in Japanese Patent Application Nos. 52-78863, 52-78864 and 60-228500, and Tetrahedron (1984) vol. 40, No. 5, p. 851.

In addition, 3,7-dioxo-5β-cholanic acid or ester derivatives thereof (compound 8) can be converted to ursodeoxycholic acid or ester derivatives thereof (compound 21a) with reference to Japanese Patent Application Laid-Open Nos. 60-228500 and 5-32692. Moreover, 3,7-dioxo-5β-cholanic acid or ester derivatives thereof (compound 8) can be converted to 3α-hydroxy-7-oxo-5β-cholanic acid and ester derivatives thereof (compound 21c) with reference to Japanese Patent Application Laid-Open Nos. 52-78863 and 52-78864. Furthermore, 3α-hydroxy-7-oxo-5β-cholanic acid or ester derivatives thereof (compound 21 c) can be converted to ursodeoxycholic acid or ester derivatives thereof (compound 21a) with reference to Japanese Patent Application Laid-Open Nos. 52-78863, 52-78864, and 5-32692.

Next, embodiments regarding the aforementioned schematic view 1 will be described more in detail.

3,7-dioxo-5β-cholanic or ester derivatives thereof can be produced, using, as raw materials, sterols having double bonds at positions 5 and 24, such as cholesta-5,7,24-trien-3β-ol, ergosta-5,7,24(28)-trien-3β-ol, desmosterol, fucosterol, or ergosta-5, 24(28)-dien-3β-ol, by performing the following 4 steps:
(I) a step of performing oxidation of a hydroxyl group at position 3 and isomerization of a double bond at position 5 to position 4;
(II) a step of converting position 24 to a carboxyl group or an ester derivative thereof by the oxidative cleavage of a side chain;
(III) a step of introducing an oxygen functional group into position 7; and
(IV) a step of constructing a 5β-configuration by reduction of a double bond at position 4.

Regarding (I) above, both sterol having a double bond at position 5 and sterol having double bonds at positions 5 and 7 can be treated by the same means as in the aforementioned step 1.

Moreover, regarding (III) above, a steroid substrate having a double bond at position 6 can be treated by the same means as in the aforementioned steps 3A, 3B, 3C, and 7, for example. Furthermore, a steroid substrate that does not have a double bond at position 6 can be treated by the methods described in, for example, Appl. Environ. Microbiol., 1986, vol. 51, p. 946; J. Chem. Res., Synop., 1986, No. 2, p. 48; and Appl. Environ. Microbiol., 1982, vol. 44, p. 6.

Further, regarding (IV) above, a steroid substrate can be treated by the same means as in the aforementioned steps 4A and 4B, for example.

Still further, the aforementioned step (II) will be described in detail below, based on the aforementioned schematic view 2.

In the case of sterols including cholesta-5,7,24-trien-3β-ol and desmosterol as typical examples, a double bond at position 24 can be epoxidized by the same means as in the aforementioned steps 3A and 3B. Thereafter, epoxide can be hydrolyzed to glycol by the same means as in the aforementioned steps 5A, 5B, 5C, and 5D. Thereafter, glycol can be converted to a carboxyl group at position 24 due to oxidative cleavage by the same means as in the aforementioned steps 6A and 6C.

On the other hand, in the case of sterols including ergosta-5,7,24(28)-trien-3β-ol, fucosterol, and ergosta-5,24(28)-dien-3β-ol as typical examples, a double bond at position 24(28) can be epoxidized by the same means as in the aforementioned steps 3A and 3B, for example. Thereafter, epoxide can be hydrolyzed to glycol by the same means as in the aforementioned steps 5A, 5B, 5C, and 5D. Thereafter, glycol can be converted to a ketone at position 24 due to oxidative cleavage by the same means as in the aforementioned steps 6A and 6C. Thereafter, the ketone at position 24 can be induced to a carboxylate or isopropyl ester at position 24 by Baeyer-Villiger oxidation, using peracid in a common organic chemistry manner. For example, the above ketone can be treated by the same means as in the aforementioned steps 3A and 3B.

Moreover, all of the aforementioned substrates can be induced to an aldehyde body at position 24 or a carboxylate at position 24 by directly subjecting a double bond at position 24 to ozone oxidation, resulting in oxidative cleavage.

Examples of a steroid compound containing 22 or more carbon atoms that is generated from carbohydrate by the fermentation method may include zymosterol, cholesta-7,24-dien-3 β-ol, cholesta-5,7,24-trien-3 β-ol, desmosterol, fucosterol, episterol, ergosta-5,7,24(28)-trienol, ergosta-5,7,22,24(28)-tetraenol, and ergosterol. These compounds are treated, for example, by the same means as in the aforementioned steps 4A and 4B. Thus, these compounds are subjected to a step of constructing a 5β-configuration by reduction of a double bond at position 4, and as necessary, are also subjected to steps:
(I) a step of performing oxidation of a hydroxyl group at position 3 and isomerization of a double bond at position 5 to position 4; (II) a step of converting position 24 to a carboxyl group or ester derivatives thereof by the oxidative cleavage of a side chain; and (III) a step of introducing an oxygen functional group into position 7, thereby producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the above formula (8), (21a), (21b), (21c), or (21d) (wherein R¹ represents a hydrogen atom, or an alkyl group containing 1 to 6 carbon atoms).

### EXAMPLES

The present invention will be more specifically described in the following examples. However, these examples are not intended to limit the scope of the present invention. The structures of the generated products were confirmed by ¹H-NMR (300 or 400 MHz, TMS/CDCl₃).

### [Example 1]

### <Production of cholesta-4,7,24-trien-3-one (compound 3)>

4.38 g (11.47 mmol) of cholesta-5,7,24-trien-3β -ol (compound 2) and 11.24 g (114.66 mmol) of cyclohexanone were dissolved in 44 ml of toluene, and the mixture was subjected to deaeration under a reduced pressure and nitrogen substitution at room temperature. This treatment was repeated several times. Thereafter, 1.17 g (5.74 mmol) of aluminum isopropoxide was added to the reaction solution at room temperature, and the obtained mixture was then stirred in a nitrogen atmosphere at 112°C for 2 hours. After completion of the reaction, the reaction solution was cooled to room temperature, and 2.3 ml of water was then added thereto. The obtained mixture was stirred at room temperature for 1 hour. Thereafter, the deposited precipitate was filtrated, and the filtrate was then concentrated. The concentrate was isolated and purified by silica gel column chromatography, so as to obtain 4.01 g of cholesta-4,7,24-trien-3-one (compound 3). The yield of the isolated and purified compound 3 was found to be 92%. The NMR shift value (δ ppm) is shown below.
δ:0.60 (s, 3H, 18-H), 0.96 (d, J=6.5Hz, 3H ,21-H), 1.18 (s, 3H, 19-H), 1.61 (s, 3H, 26-H), 1.69 (s, 3H, 27-H), 2.22 (m, 1H), 2.30-2.40 (m, 3H), 2.63-2.72 (m, 1H, 6-H), 3.10-3.20 (m, 1H, 6-H), 5.09 (m, 1H, 24-H), 5.18 (m, 1H, 7-H), 5.80 (s, 1H, 4-H)

### [Example 2]

### <Production of cholesta-4,6,24-trien-3-one (compound 4)>

3.49 g (9.18 mmol) of the cholesta-4,7,24-trien-3-one (compound 3) obtained by the method described in Example 1 was dissolved in 70 ml of methanol, and thereafter, deaeration under a reduced pressure and nitrogen substitution were repeated several times at room temperature. Thereafter, 2.53 g (38.40 mmol) of 85% potassium hydroxide was added to the reaction solution, and the obtained mixture was stirred in a nitrogen atmosphere at 64°C for 7 hours. After completion of the reaction, the reaction solution was cooled to room temperature, and 2.37 g of acetic acid was then added thereto, followed by stirring the obtained mixture at room temperature for 0.5 hours. Subsequently, methanol was distilled away under a reduced pressure, and water was then added thereto, followed by extraction with ethyl acetate. The organic layer was washed with water, dried, and then concentrated. The obtained concentrate was isolated and purified by silica gel column chromatography, so as to obtain 3.13 g of cholesta-4,6,24-trien-3-one (compound 4). The yield of the isolated and purified compound 4 was found to be 90%. The NMR shift value (δ ppm) is shown below.
δ:0.76 (s, 3H, 18-H), 0.95 (d, J=6.5Hz, 3H, 21-H),1.12 (s, 3H, 19-H), 1.61 (s, 3H, 26-H), 1.69 (s, 3H, 27-H), 2.19 (m, 1H), 2.39-2.65 (m, 2H), 5.09 (m, 1H, 24-H), 5.67 (s, 1H, 4-H), 6.12 (m, 2H, 6-H and 7-H)

### [Example 3]

### <Production of ergosta-4,6,24-trien-3-one>

0.20 g (0.50 mmol) of ergosta-4,7,24-trien-3-one was dissolved in 10 ml of methanol. Thereafter, deaeration under a reduced pressure and nitrogen substitution were repeated several times at room temperature. Thereafter, 0.10 g (1.52 mmol) of 85% powder potassium hydroxide was added to the reaction solution, and the obtained mixture was stirred in a nitrogen atmosphere at 65°C for 12 hours. After completion of the reaction, the reaction solution was cooled to room temperature, and 0.18 g of acetic acid was then added thereto, followed by stirring the obtained mixture at room temperature for 0.5 hours. Subsequently, methanol was distilled away under a reduced pressure, and water was then added thereto, followed by extraction with ethyl acetate. The organic layer was washed with water, dried, and then concentrated. The obtained concentrate was isolated and purified by silica gel column chromatography, so as to obtain 0.20 g of ergosta-4,6,24-trien-3-one. The yield thereof was found to be 100%. The NMR shift value (δ ppm) is shown below.
δ:0.77 (s, 3H), 0.81 (d, J=7.3Hz, 3H), 0.83 (d, J=7.3Hz, 3H), 0.91 (d, J=7.3Hz, 3H), 1.01 (d, J=7.3Hz, 3H), 1.12 (s, 3H), 2.20 (m, 1H), 2.38-2.66 (m, 2H), 5.11-5.27 (m, 2H), 5.67 (s, 1H), 6.06-6.17 (m, 2H)

The reaction formula of the present example is shown below.

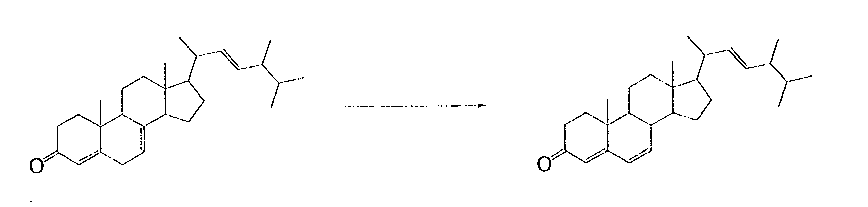

### [Comparative example 1]

### <Production of cholesta-4,7,24-trien-3-one (compound 3)>

0.20 g (0.5 mmol) of cholesta-5,7,24-trien-3β-ol (compound 2) and 0.26 g (2.6 mmol) of cyclohexanone were dissolved in 2 ml of heptane. Thereafter, 0.02 g (0.1 mmol) of aluminum isopropoxide was added thereto at room temperature, and the obtained mixture was heated to reflux for 4 hours in the air. After completion of the reaction, the reaction solution was cooled to room temperature, and water was added thereto. The obtained mixture was stirred at room temperature for 1 hour. The deposited precipitate was filtrated, and the filtrate was concentrated. The concentrate was calibrated by HPLC. As a result, the yield of compound 3 was found to be 85%.

### [Comparative example 2]

### <Production of cholesta-4,6,24-trien-3-one (compound 4)>

0.3 g (0.8 mmol) of the cholesta-4,7,24-trien-3-one (compound 3) obtained by the method described in Example 1 was dissolved in 5.3 ml of methanol. Thereafter, 0.09 g (1.4 mmol) of 85% potassium hydroxide was added thereto, and the obtained mixture was heated to reflux for 4 hours in the air. After completion of the reaction, the reaction solution was cooled to room temperature, and 2.37 g of acetic acid was added thereto. The obtained mixture was stirred at room temperature for 0.5 hours. Thereafter, methanol was distilled away under a reduced pressure. Water was added thereto, followed by extraction with ethyl acetate. The organic layer was washed with water, dried, and then concentrated. The concentrate was calibrated by HPLC. As a result, the yield of compound 4 was found to be 52%.

### [Example 4-1]

### <Step 3A: production of 6,7:24,25-diepoxycholest-4-en-3-one (compound 5)>

0.10 g (0.26 mmol) of cholesta-4,6,24-trien-3-one was dissolved in 3.5 ml (1 mmol) of a 0.3 M perbenzoic acid/ethyl acetate solution, and the obtained mixture was then stirred at 45°C for 8 hours. After completion of the reaction, an aqueous 10% sodium sulfite solution was added to the reaction solution to decompose the residual peroxide, and then extracted with ethyl acetate. Subsequently, the organic layer was washed with an aqueous saturated potassium bicarbonate solution, dried, and then concentrated, so as to obtain 0.138 g of a crude compound, 6,7:24,25-diepoxycholest-4-en-3-one. The yield thereof was found to be 70%. The NMR shift value (δ ppm) is shown below. δ: 0.75 (s, 3H, 18-H), 0.95 (d, J=5.7Hz, 3H, 21-H), 1.10 (s, 3H, 19-H), 1.27 (s, 3H, 26-H), 1.30 (s, 3H, 27-H), 2.4-2.6 (m, 2H), 2.69 (t, J=5.5Hz, 1H, 24-H), 3.3-3.4 (m, 1H, 7-H), 3.47 (d, J=3.3Hz, 1H, 6-H), 6.12 (s, 1H, 4-H)

### [Example 4-2]

### <Step 3A: production of 6,7:24,25-diepoxycholest-4-en-3-one (compound 5)>

1.00 g (2.63 mmol) of cholesta-4,6,24-trien-3-one was dissolved in 11.7 ml of n-butyl acetate, and 4 ml of water was added thereto. Thereafter, 2.53 ml (2.63 mmol) of a 1.04 M 2-methylperbenzoic acid/n-butyl acetate solution (hereinafter abbreviated as a peracid solution) was added dropwise to the above mixed solution at 78°C, and obtained mixture was then stirred at 78°C for 1 hour. Thereafter, the water layer was separated and eliminated, and then washed with a saturated sodium bicarbonate solution and then with water. Thereafter, 4 ml of water was added to the resultant. (*1) 0.50 ml (0.53 mmol) of a peracid solution was added to the mixed solution at 78°C, and the obtained mixture was then stirred at 78°C for 0.5 hours. Thereafter, 0.50 ml (0.53 mmol) of a peracid solution was further added to the reaction solution, and the obtained mixture was then stirred at 78°C for 0.5 hours. Thereafter, the water layer was separated and eliminated, and then washed with a saturated sodium bicarbonate solution and then with water. Thereafter, 4 ml of water was added to the resultant. The same operations as described in (*1) were repeated twice on the above mixed solution. Thereafter, 0.50 ml (0.53 mmol) of a peracid solution was added thereto at 78°C, and the mixture was then stirred at 78°C for 0.5 hours. Thereafter, 0.50 ml (0.53 mmol) of a peracid solution was further added thereto, and the mixture was then stirred at 78°C for 0.5 hours. Thereafter, the reaction solution was cooled to room temperature, and sodium sulfite was added thereto to decompose the residual peroxide, followed by extraction with ethyl acetate. Subsequently, the organic layer was washed with a saturated sodium bicarbonate solution, dried, and then concentrated, so as to obtain 1.33 g of a crude compound, 6,7:24,25-diepoxycholest-4-en-3-one. The yield thereof was found to be 82%.

The obtained crude compound, 6,7:24,25-diepoxycholest-4-en-3-one, was subjected to thin-layer chromatography, so as to separate 6α-, 7α-, and 6β-, 7β-forms. The NMR shift values (δ ppm) thereof are shown below. 6α,7α;24,25-diepoxycholest-4-en-3-one
δ: 0.75 (s, 3H, 18-H), 0.94 (d, J=6.8Hz, 3H, 21-H), 1.09 (s, 3H, 19-H), 1.27 and 1.31 (s, 6H, 26-H and 27-H), 2.42-2.60 (m, 2H), 2.69 (t, J=6.0Hz, 1H, 24-H), 3.35 (d, J=3.6Hz, 1H, 7-H), 3.46 (d, J=4.0Hz, 1H, 6-H), 6.11 (s, 1H, 4-H) 6β,7β;24,25-diepoxycholest-4-en-3-one
δ: 0.75 (s, 3H, 18-H), 0.95 (d, J=6.5Hz, 3H, 21-H), 1.21 (s, 3H, 19-H), 1.27 and 1.31 (s, 6H, 26-H and 27-H), 2.36-2.64 (m, 2H), 2.69 (t, J=5.5Hz, 1H, 24-H), 3.37 (s, 2H, 6-H and 7-H), 6.15 (s, 1H, 4-H)

### [Example 5-1]

### <Step 4A: production of 24,25-epoxy-5β-cholestan-3-one-7-ol (compound 6)>

0.128 g of the crude compound obtained in the aforementioned Example 4-1, 6,7:24,25-diepoxycholest-4-en-3-one, was dissolved in 1.8 ml of ethyl acetate. Thereafter, 0.5 ml of triethylamine and 7 mg of 10% palladium carbon were added thereto, and the obtained mixture was stirred at room temperature for 15 hours under the hydrogen atmosphere of 1 atm. After completion of the reaction, the catalyst was filtrated, and the filtrate was then concentrated. The concentrate was subjected to short silica gel column chromatography for concentration, thereby obtaining 0.077 g of a crude compound, 24,25-epoxy-5β-cholestan-3-one-7-ol. The yield thereof was found to be 87%. The NMR shift value (δ ppm) is shown below.
δ: 0.70 (s, 3H, 18-H), 0.95 (d, J=5.5Hz, 3H, 21-H), 1.00 (s, 3H, 19-H), 1.27 (s, 3H, 26-H), 1.30 (s, 3H, 27-H), 2.15-2.25 (m, 2H), 2.3-2.5 (m, 1H), 2.69 (t, J=5.5Hz, 1H, 24-H), 3.40 (t, J=13.3Hz, 1H, 4-H), 3.92 (m, 1H, 7-H)

The obtained crude compound, 24,25-epoxy-5β-cholestan-3-one-7-ol, was subjected to thin-layer chromatography, so as to separate 7α- and 7β-forms and a reaction intermediate. The NMR shift values (δ ppm) thereof are shown below.
24,25-epoxy-5β-cholestan-3-one-7α-ol
δ: 0.71 (s, 3H, 18-H), 0.96 (d, J=6.0Hz, 3H, 21-H), 1.01 (s, 3H, 19-H), 1.27 and 1.31 (s, 6H, 26-H and 27-H), 2.12-2.23 (m, 2H), 2.41 (dt, J=14Hz and 4.8Hz, 1H), 2.69 (t, J=6.0Hz, 1H, 24-H), 3.39 (t, J=13.2Hz, 1H, 4-H), 3.93 (m, 1H, 7-H)
24,25-epoxy-5β-cholestan-3-one-7β -ol
δ: 0.73 (s, 3H, 18-H), 0.96 (d, J=6.6Hz, 3H, 21-H), 1.06 (s, 3H, 19-H), 1.27 and 1.31 (s, 6H, 26-H and 27-H), 2.15-2.35 (m, 3H), 2.52 (t, J=11.0Hz, 1H), 2.69 (t, J=6.1Hz, 1H, 24-H), 3.56-3.68 (m, 1H, 7-H)
24,25-epoxycholest-4-en-3-one-7α-ol
δ: 0.73 (s, 3H, 18-H), 0.94 (d, J=6.8Hz, 3H, 21-H), 1.19 (s, 3H, 19-H), 1.27 and 1.31 (s, 6H, 26-H and 27-H), 2.33-2.46 (m, 3H), 2.60-2.64 (m, 1H), 2.69 (t, J=6.4Hz, 1H, 24-H), 3.97 (m, 1H, 7-H), 5.81 (d, J=1.6Hz, 1H, 4-H)
24,25-epoxycholest-4-en-3-one-7β-ol
δ: 0.74 (s, 3H, 18-H), 0.95 (d, J=6.8Hz, 3H, 21-H), 1.21 (s, 3H, 19-H), 1.27 and 1.31 (s, 6H, 26-H and 27-H), 2.31-2.56 (m, 4H), 2.69 (t, J=6.0Hz, 1H, 24-H), 3.42-3.50 (m, 1H, 7-H), 5.76 (d, J=1.2Hz, 1H, 4-H)

### [Example 5-2]

### <Step 4A: production of 24,25-epoxy-5 β-cholestan-3-one-7-ol (compound 6)>

2.6 mg of 5% palladium carbon was suspended in 1,060 µl of methanol, and 25 µl (0.165 mmol) of tetramethylethylenediamine was added thereto. The obtained mixture was stirred in a nitrogen atmosphere at 50°C for 3 hours. Subsequently, the reaction solution was cooled to room temperature, and 206 µl of water was added thereto. The inside of the reaction system was substituted with hydrogen of 1 atm, and the reaction solution was then stirred at room temperature for 1 hour. Subsequently, the pre-treated catalyst suspension was cooled to 5°C. Then, 310 µl of an n-butyl acetate solution containing 100 mg (0.242 mmol) of the crude 6,7:24,25-diepoxycholest-4-en-3-one that had been obtained in the aforementioned Example 4-2 and had been then purified with a silica gel column, and 424 µl of methanol was added thereto in a hydrogen atmosphere. The obtained mixture was stirred under the hydrogen atmosphere of 1 atm at 5°C for 49 hours. After completion of the reaction, the catalyst was filtrated, and the filtrate was then concentrated. The concentrate was concentrated by short silica gel column chromatography, so as to obtain 115 mg of a crude compound, 24,25-epoxy-5β-cholestan-3-one-7-ol. The yield thereof was found to be 96%.

### [Example 6-1]

### <Step 5A: production of 5β-cholestan-3-one-7,24,25-triol (compound 7)>

0.077 g (0.19 mmol) of the crude 24,25-epoxy-5β-cholestan-3-one-7-ol obtained in the aforementioned Example 5-1 was adsorbed on a silica gel column, and it was then eluted with a mixed solution consisting of hexane and ethyl acetate, so as to obtain 0.080 g of 5β-cholestan-3-one-7,24,25-triol. The yield thereof was found to be 100%. The NMR shift value (δ ppm) thereof is shown below.
δ: 0.71 (s, 3H, 18-H), 0.95 and 0.96 (d, J=6.8Hz, 3H, 21-H), 1.01 (s, 3H, 19-H), 1.17 and 1.21 (s, 6H, 26-H and 27-H), 2.15-2.23 (m, 2H), 2.35-2.45 (m, 1H), 3.27-3.33 (m, 1H, 24-H), 3.39 (t, J=15.6Hz, 1H, 4-H), 3.93 (m, 1H, 7-H)

### [Example 6-2]

### <Step 5A: production of 5β-cholestan-3-one-7,24,25-triol (compound 7)>

145 mg (0.35 mmol) of the 24,25-epoxy-5β-cholestan-3-one-7-ol obtained in the aforementioned Example 5-2 was dissolved in a mixed solution consisting of 1 ml of acetonitrile and 1 ml of water. Thereafter, 73 mg (0.35 mmol) of citric acid monohydrate was added thereto, and the obtained mixture was stirred at room temperature for 9 hours. After completion of the reaction, sodium bicarbonate was added to the reaction solution, so as to neutralize citric acid. Thereafter, acetonitrile was distilled away under a reduced pressure, followed by extraction with ethyl acetate. The extract was concentrated, so as to obtain 150 mg of a crude compound, 5β-cholestan-3-one-7,24,25-triol. The yield thereof was found to be 98%.

### [Example 7]

### <Step 6A: production of 3,7-dioxo-5β-cholanic acid (compound 8)>

93 mg (0.22 mmol) of 5β-cholestan-3-one-7,24,25-triol was dissolved in 1.5 ml of acetonitrile and 1.1 ml of water. Thereafter, 0.17 g (0.44 mmol) of 60% calcium hypochlorite was added thereto at room temperature, and 0.12 g of acetic acid was further added to the obtained mixture, followed by stirring the obtained mixture at room temperature for 40 minutes. After completion of the reaction, an aqueous 10% sodium sulfite solution was added to the reaction solution to decompose an excessive oxidizing agent. Thereafter, concentrated hydrochloric acid was added thereto, so that the pH thereof could be adjusted to pH 1. After the mixture had been subjected to vacuum concentration, the concentrate was isolated and purified by silica gel column chromatography, so as to obtain 75 mg of 3,7-dioxo-5β-cholanic acid. The yield thereof was found to be 92%. The NMR shift value (δ ppm) thereof is shown below.
δ: 0.72 (s, 3H, 18-H), 0.95 (d, J=6.3Hz, 3H, 21-H), 1.30 (s, 3H, 19-H), 2.50 (t, J=10.7Hz, 1H), 2.88 (dd, J=5.1Hz and 14.2Hz, 1H)

### [Example 8]

### <Step 5B: production of 6,7-epoxycholest-4-en-3-one-24,25-diol (compound 9)>

0.120 g of the crude 6,7:24,25-diepoxycholest-4-en-3-one synthesized by the same method as that in Example 4-1 was adsorbed on a silica gel column, and it was then eluted with a mixed solution consisting of hexane and ethyl acetate, so as to obtain 0.080 g of a crude compound, 6,7-epoxycholest-4-en-3-one-24,25-diol. The yield thereof was found to be 80%. The NMR shift value (δ ppm) thereof is shown below.
δ: 0.75 (s, 3H, 18-H), 0.95 and 0.96 (d, J=6.8Hz, 3H, 21-H), 1.10 (s, 3H,19-H), 1.18 (s, 3H, 26-H), 1.23 (s, 3H, 27-H), 2.4-2.6 (m, 2H), 3.25-3.31 (m, 1H, 24-H), 3.33-3.38 (m, 1H, 7-H), 3.46 (d, J=3.7Hz, 1H, 6-H), 6.12 (s, 1H, 4-H)

### [Example 9]

### <Step 4B: production of 5β-cholestan-3-one-7,24,25-triol (compound 6)>

The crude 6,7-epoxycholest-4-en-3-one-24,25-diol obtained by the same method as that in the aforementioned Example 5 was purified with a silica gel column. 0.075 g of the thus purified product was reduced by the same method as that in Example 5-1, and it was then purified by silica gel column chromatography, so as to obtain 0.068 g of 5β-cholestan-3-one-7α,24,25-triol. The yield thereof was found to be 90%. In addition, it was confirmed that the NMR data thereof were identical to those in Example 6-1.

### [Example 10]

### <Step 3B: production of 24,25-epoxycholesta-4,6-dien-3-one (compound 10)>

0.10 g (0.27 mmol) of cholesta-4,6,24-trien-3-one was dissolved in 2 ml of ethyl acetate. Thereafter, 0.76 ml (0.32 mmol) of a 0.42 M monoperphthalic acid/ethyl acetate solution was added thereto, and the obtained mixture was then stirred at room temperature for 3 hours. After completion of the reaction, an aqueous 10% sodium sulfite solution was added to the reaction solution to decompose the residual peroxide, followed by extraction with ethyl acetate. Subsequently, the organic layer was washed with an aqueous saturated potassium bicarbonate solution, dried, and then concentrated, so as to obtain 0.11 g of a crude compound, 24,25-epoxycholesta-4,6-dien-3-one. The yield thereof was found to be 100%. The NMR shift value (δ ppm) thereof is shown below.
δ: 0.77 (s, 3H, 18-H), 0.95 (d, J=6.4Hz, 3H, 21-H), 1.11 (s, 3H, 19-H), 1.27 (s, 3H, 26-H), 1.31 (s, 3H, 27-H), 2.15-2.23 (m, 1H), 2.38-2.65 (m, 2H, 2-H), 2.69 (t, J=5.5Hz, 1H, 24-H), 5.67 (s, 1H, 4-H), 6.07-6.16 (m, 2H, 6-H and 7-H)

### [Example 11]

### <Step 5C: production of cholesta-4,6-dien-3-one-24,25-diol (compound 11)>

0.110 g of the crude 24,25-epoxycholesta-4,6-dien-3-one (compound 10) synthesized in accordance with Example 10 was adsorbed on a silica gel column, and it was then eluted with a mixed solution consisting of hexane and ethyl acetate, so as to obtain 0.070 g of cholesta-4,6-dien-3-one-24,25-diol. The yield thereof was found to be 61 %. The NMR shift value (δ ppm) thereof is shown below.
δ: 0.76 and 0.77 (s, 3H, 18-H), 0.94 and 0.95 (d, J=6.6Hz, 3H, 21-H), 1.11 (s, 3H, 19-H), 1.17 (s, 3H, 26-H), 1.21 (s, 3H, 27-H), 2.37-2.64 (m, 2H), 3.25-3.37 (m, 1H, 24-H), 5.67 (s, 1H, 4-H), 6.05-6.16 (m, 2H, 6-H and 7-H)

### [Example 12]

### <Step 3C: production of 6,7-epoxycholest-4-en-3-one-24,25-diol (compound 9)>

66 mg (0.159 mmol) of the cholesta-4,6-dien-3-one-24,25-diol (compound 11) synthesized in accordance with Example 11 was epoxidized by the same method as that in Example 4-2, and the resultant product was then purified by silica gel column chromatography, so as to obtain 48 mg of 6,7-epoxycholest-4-en-3-one-24,25-diol. The yield thereof was found to be 70%. In addition, it was confirmed that the NMR data thereof were identical to those in Example 8.

### [Example 13]

### <Step 6B: production of 24,25-epoxy-5β-cholestane-3,7-dione (compound 12)>

667 mg of the crude 24,25-epoxy-5β-cholestan-3-one-7-ol (compound 6) synthesized in accordance with Example 5-2 was dissolved in 5.4 ml of acetonitrile and 2.7 ml of water. Thereafter, 1.21 ml of acetic acid and 31 mg of NaBr were added thereto. Thereafter, 0.36 g (1.51 mmol) of 60% calcium hypochlorite was added to the obtained mixture at 0°C, and the obtained mixture was then stirred at 0°C for 23 hours. After completion of the reaction, sodium sulfite was added to the reaction solution to decompose an excessive oxidizing agent, followed by extraction with ethyl acetate. Thereafter, the extract was washed with a saturated sodium bicarbonate solution and then with water. The resultant was dried and then subjected to vacuum concentration, so as to obtain 576 mg of a crude compound, 24,25-epoxy-5β-cholestane-3,7-dione. The yield thereof was found to be 96%. The NMR shift value (δ ppm) thereof is shown below.
δ: 0.70 (s, 3H, 18-H), 0.95 (d, J=6.4Hz, 3H, 21-H), 1.27 and 1.31 (s, 6H, 26-H and 27-H), 1.31 (s, 3H, 19-H), 2.50 (t, J=11.2Hz, 1H), 2.69 (t, J=6.4Hz, 1H, 24-H), 2.89 (dd, J=5.6Hz and 12.8Hz, 1H)

### [Example 14]

### <Step 5D: production of 5β-cholestane-3,7-dione-24,25-diol (compound 13)>

419 mg of the crude 24,25-epoxy-5β-cholestane-3,7-dione (compound 12) obtained in Example 13 was hydrolyzed by the same method as that in Example 6-2, so as to obtain 410 mg of a crude compound, 5β-cholestane-3,7-dione-24,25-diol. The yield thereof was found to be 97%. The NMR shift value (δ ppm) thereof is shown below.
δ: 0.70 and 0.71 (s, 3H, 18-H), 0.94 and 0.95 (d, J=6.0Hz, 3H, 21-H), 1.16 and 1.21 (s, 6H, 26-H and 27-H), 1.31 (s, 3H, 19-H), 2.51 (t, J=11.6Hz, 1H), 2.89 (dd, J=5.6Hz and 12.8Hz, 1H), 3.27-3.35 (m, 1H, 24-H)

### [Example 15]

### <Step 6C: production of 3,7-dioxo-5β-cholanic acid (compound 8)>

390 mg of the crude 5β-cholestane-3,7-dione-24,25-diol (compound 13) obtained in Example 14 was dissolved in 6 ml of acetonitrile and 2.7 ml of water. Thereafter, 0.17 ml of acetic acid was further added thereto. Thereafter, 294 mg (1.23 mmol) 60% calcium hypochlorite was added to the obtained mixture at 10°C, and the obtained mixture was then stirred at 10°C for 26.5 hours. After completion of the reaction, sodium sulfite was added to the reaction solution to decompose an excessive oxidizing agent. Thereafter, concentrated hydrochloric acid was added thereto, resulting in pH < 1. The reaction solution was extracted with ethyl acetate, and the extract was washed with water, dried, and then concentrated, so as to obtain 381 mg of a crude compound, 3,7-dioxo-5β-cholanic acid. The yield thereof was found to be 84%. In addition, it was confirmed that the NMR data thereof were identical to those in Example 7.

### [Example 16]

### <Step 7: production of 6,7:24,25-diepoxycholest-4-en-3-one (compound 5)>

547 mg (1.44 mmol) of cholesta-4,6,24-trien-3-one was dissolved in 10 ml of formic acid at 10°C, and 520 µl of t-butyl hypochloride was then added thereto. The obtained mixture was stirred at 10°C for 30 minutes. After completion of the reaction, water was added to the reaction solution, followed by extraction with ethyl acetate. The extract was washed with water and then with a saturated sodium bicarbonate solution. The resultant was dried and then concentrated, so as to obtain 870 mg of a crude compound, 7,24-dichloro-cholest-4-en-3-one-6,25-diol diformyl ester (compound 15a). The yield thereof was found to be approximately 70%. The NMR shift value (δ ppm) thereof is shown below.
δ: 0.77 (s, 3H, 18-H), 0.93 and 0.95 (d, J=4.9Hz and 6.5Hz, 3H, 21-H), 1.28 (s, 3H, 19-H), 1.60 (s, 6H, 26-H and 27-H), 2.38-2.57 (m, 2H), 4.14 (t, J=3.0Hz, 1H), 4.25 (t, J=12.0Hz, 1H), 5.55 (d, J=1.9Hz, 1H, 6-H), 6.04 (s, 1H, 4-H), 8.00 (s, 1H, Formyl), 8.05 (s, 1H, Formyl)

Subsequently, 870 mg of the thus obtained crude 7,24-dichloro-cholest-4-en-3-one-6,25-diol diformyl ester (compound 15a) was dissolved in 15 ml of methanol, and 300 mg of KHCO₃ (3 mmol) was then added thereto. The obtained mixture was stirred at room temperature for 8 hours, so as to cleave the ester. Thereafter, 694 mg of K₂CO₃ (5 mmol) was added thereto, and the obtained mixture was then stirred at room temperature for 24 hours, so as to cyclize an intermediate, chlorohydrin, to epoxide. After completion of the reaction, 1 ml of acetic acid was added thereto, and methanol was concentrated. Water was added thereto, followed by extraction with ethyl acetate. The extract was dried and then concentrated, and the concentrate was then purified by silica gel column chromatography, so as to obtain 341 mg of a crude compound, 6,7:24,25-diepoxycholest-4-en-3-one. The total yield obtained from the two steps was found to be approximately 57%.

It was also found that the obtained crude 6,7:24,25-diepoxycholest-4-en-3-one was only 6β,7β -epoxide.

### [Example 17]

### <Production of benzoic acid 6,7-dihydroxy-3,7-dimethyloctyl ester >

0.50 g (1.92 mmol) of 1-benzoyl citronellol was dissolved in 6 ml of chloroform, and 0.99 g (5.76 mmol) of m-chloroperbenzoic acid was then added thereto. The obtained mixture was stirred at room temperature for 1 hour. After completion of the reaction, an aqueous 10% sodium sulfite solution was added to the reaction solution to decompose the residual peroxide, followed by extraction with chloroform. Subsequently, the organic layer was washed with an aqueous saturated potassium bicarbonate solution. The resultant was dried and then concentrated, so as to obtain 0.49 g of a crude compound, benzoic acid 5- (3,3-dimethyloxiranyl)-3-methylpentyl ester. The yield thereof was found to be 92%. The NMR shift value (δ ppm) thereof is shown below.
δ: 1.00 (d, J=7.7Hz, 3H), 1.28 (s, 3H), 1.30 (s, 3H), 1.4-1.9 (m, 7H), 2.72(t, J=6.6Hz, 1H), 4.37 (m, 2H), 7.45 (m, 2H), 7.57 (m, 1H), 8.04 (m, 2H)

Subsequently, 0.10 g (0.36 mmol) of the obtained crude benzoic acid 5- (3,3-dimethyloxiranyl)-3-methylpentyl ester was dissolved in 2 ml of ethyl acetate. Thereafter, 22 mg of water, 17 mg of acetic acid, and 0.20 g of silica gel were added thereto, and the obtained mixture was stirred at 40°C for 24 hours. After completion of the reaction, silica gel was filtrated, and the filtrate was then concentrated, so as to obtain 0.10 g of a crude compound, benzoic acid 6,7-dihydroxy-3,7-dimethyloctyl ester. It was found that the conversion rate thereof was 100% and that the yield thereof was 94%. Other than the product of interest, no by-products were detected. The NMR shift value (δ ppm) thereof is shown below.
δ: 0.99 (t, J=5.3Hz, 3H), 1.16 (s, 3H), 1.21 (s, 3H), 1.2-1.95 (m, 7H), 2.14-2.25 (m, 1H), 3.32 (m, 1H), 4.3-4.45 (m, 2H), 7.44 (t, J=7.7Hz, 2H), 7.54 (m, 1H), 8.04 (d, J=8.2Hz, 2H)

The reaction formula of the present example is shown below.

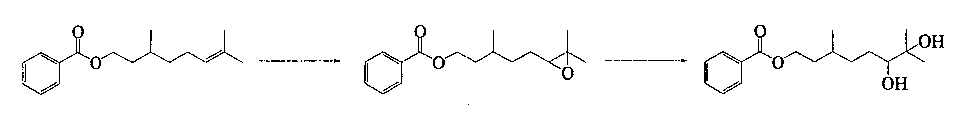

### INDUSTRIAL APPLICABILITY

According to the present invention, cholesta-4,6,24-trien-3-one useful as a synthetic intermediate of various steroid medicaments, or 3,7-dioxo-5β-cholanic acid and ester derivatives thereof useful as important synthetic intermediates of various steroid medicaments, such as ursodeoxycholic acid or chenodeoxycholic acid, can be efficiently and economically produced, by using, as raw materials, cholesta-5,7,24-trien-3β-ol which is a sterol having double bonds at positions 5 and 24. Various steroid medicaments can be stably supplied as a result of the establishment of an inexpensive chemical synthesis method, thereby greatly contributing to expansion of the intended use.

## Claims

1. A method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d): 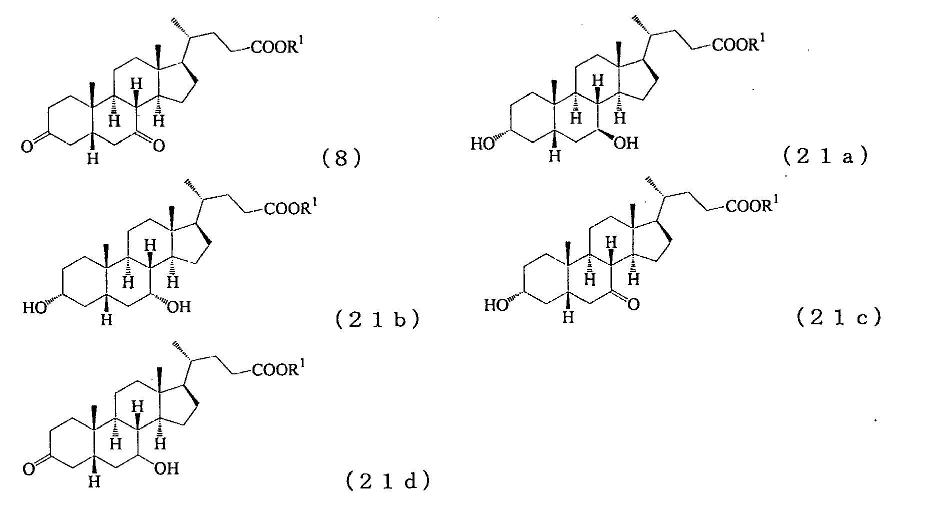 wherein R¹ represents a hydrogen atom, or an alkyl group containing 1 to 6 carbon atoms;
which is **characterized in that** it uses a steroid compound containing 22 or more carbon atoms generated from carbohydrate by a fermentation method, and **in that** it comprises a step of constructing a 5β-configuration by reduction of a double bond at position 4.

2. A method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d): 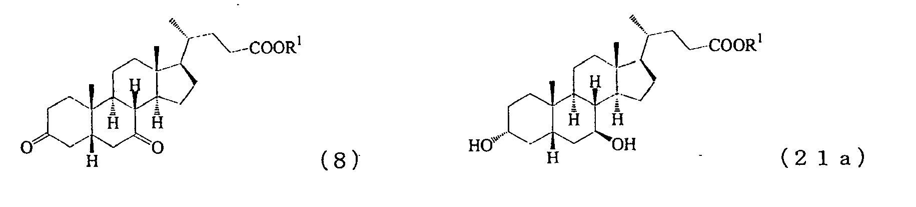 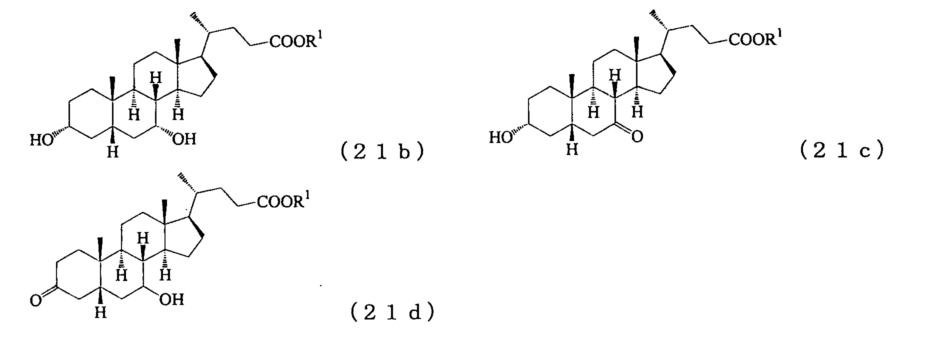 wherein R¹ represents a hydrogen atom, or an alkyl group containing 1 to 6 carbon atoms;
which is **characterized in that** a 5β-configuration is constructed by reduction of a double bond at position 4 in a steroid compound represented by the following formula (A1), (A2), (A3), (A4), (A5), (A6), (A7), or (A8): 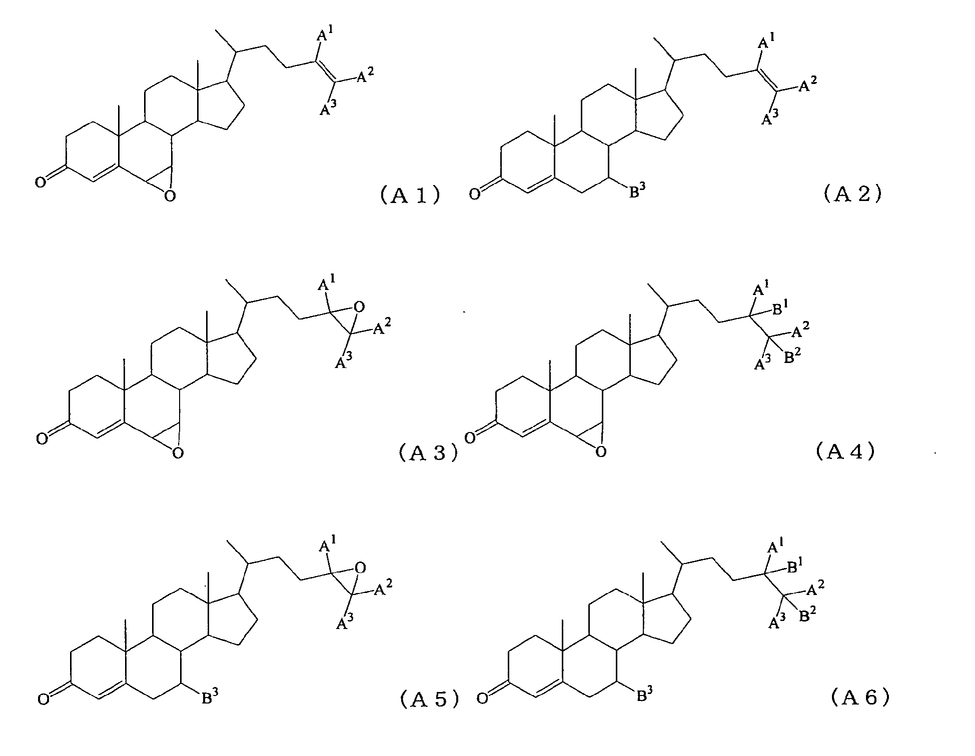 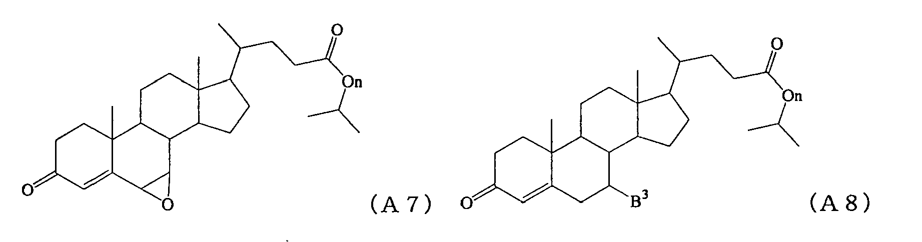 wherein A¹ represents a hydrogen atom or isopropyl group; each of A² and A³ independently represents a methyl group when A¹ is a hydrogen atom, and represents a hydrogen atom or methyl group when A¹ is an isopropyl group; each of B¹, B², and B³ independently represents a hydroxyl group or protected hydroxyl group; and n represents an integer of 0 or 1.

3. The method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d), according to claim 2: 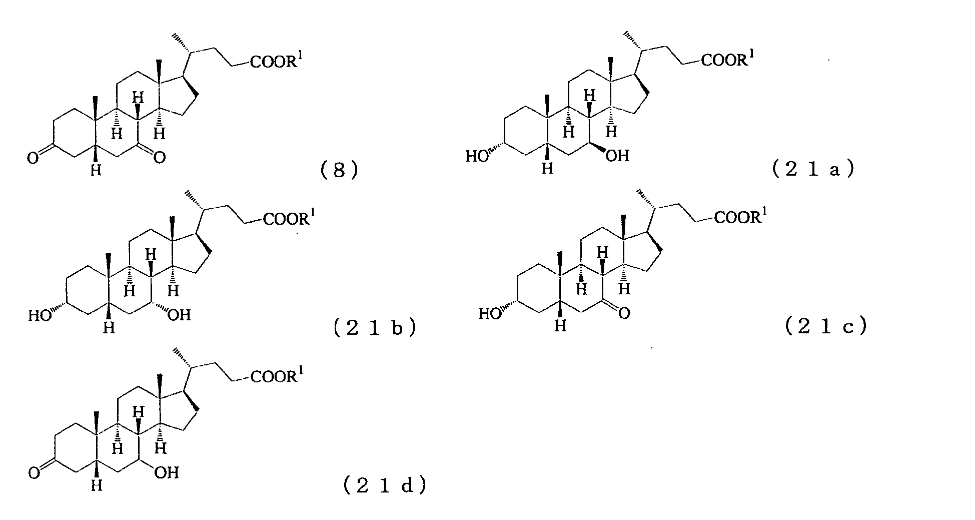 wherein R¹ represents a hydrogen atom, or an alkyl group containing 1 to 6 carbon atoms;
which is **characterized in that** the steroid compound represented by the following formula (A1), (A2), (A3), (A4), (A5), (A6), (A7), or (A8) is induced from a sterol compound represented by the following formula (1): 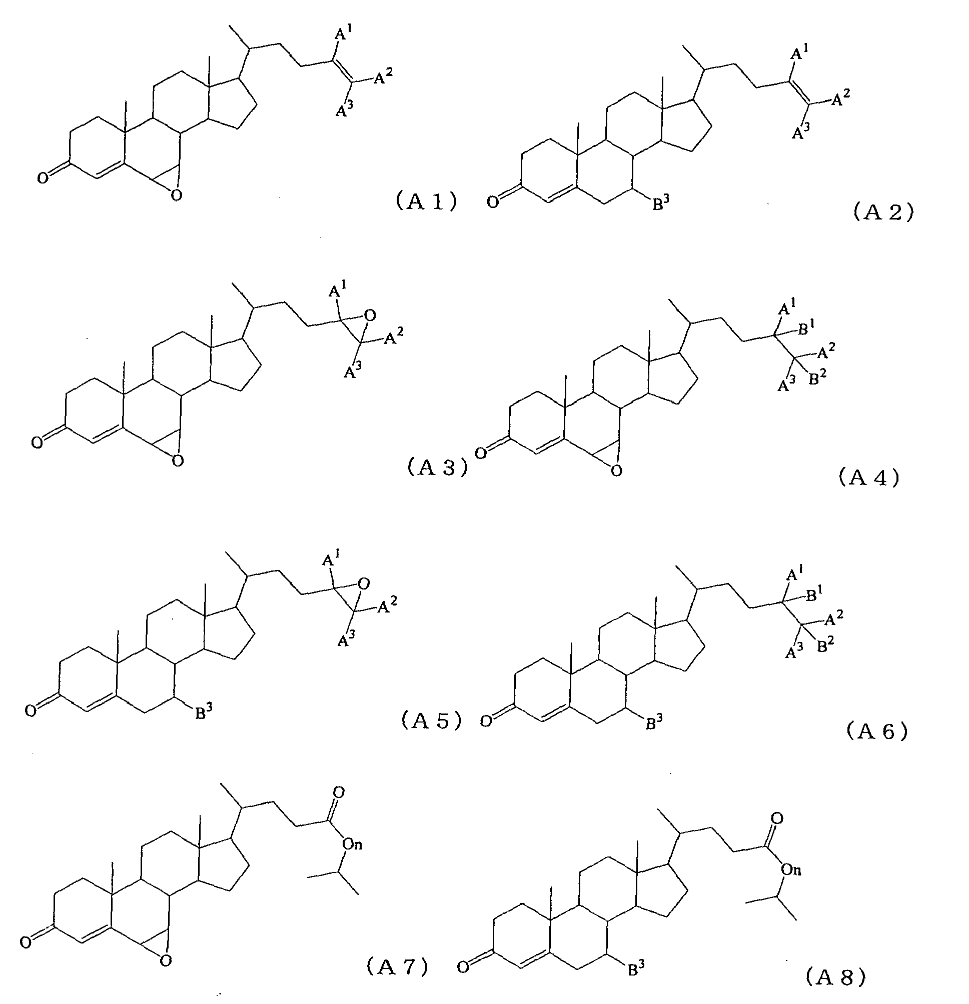 wherein A¹ represents a hydrogen atom or isopropyl group; each of A² and A³ independently represents a methyl group when A¹ is a hydrogen atom, and represents a hydrogen atom or methyl group when A¹ is an isopropyl group; each of B¹, B², and B³ independently represents a hydroxyl group or protected hydroxyl group; and n represents an integer of 0 or 1, 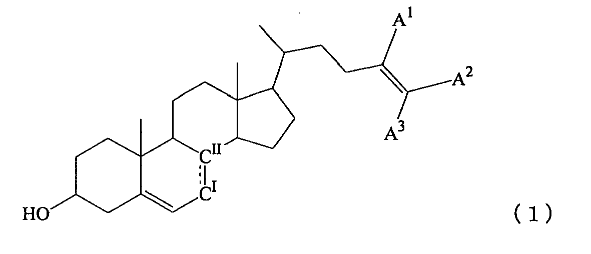 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.

4. A method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d): 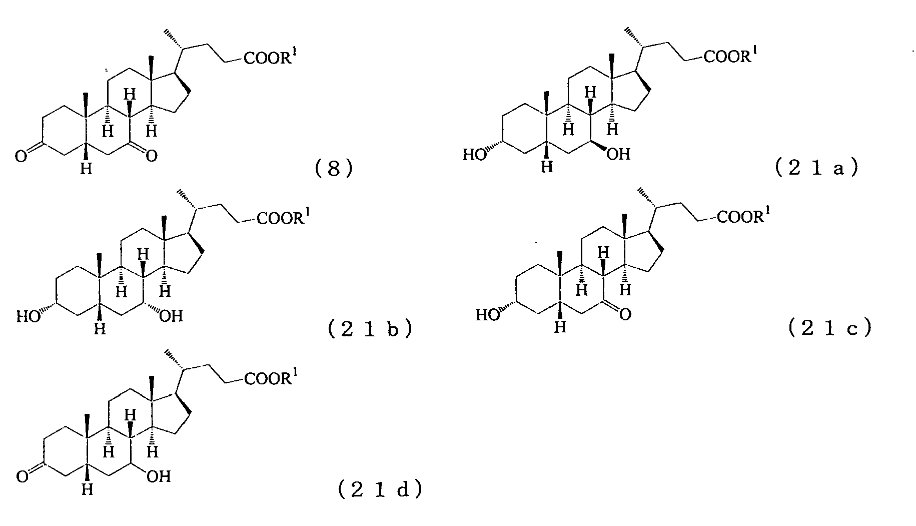 wherein R¹ represents a hydrogen atom, or an alkyl group containing 1 to 6 carbon atoms;
which uses, as a raw material, a sterol compound represented by the following formula (1): 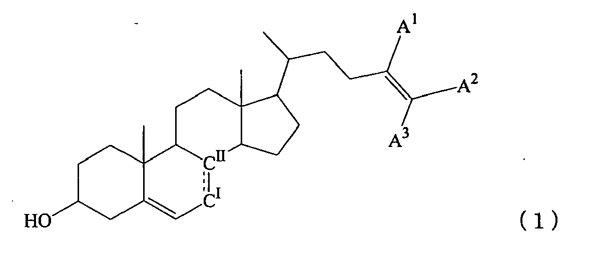 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond, and
which comprises the following steps:
(I) a step of performing oxidation of a hydroxyl group at position 3 and isomerization of a double bond at position 5 to position 4;
(II) a step of converting position 24 to a carboxyl group or ester derivatives thereof by the oxidative cleavage of a side chain;
(III) a step of introducing an oxygen functional group into position 7; and
(IV) a step of constructing a 5β-configuration by reduction of a double bond at position 4.

5. The method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d), according to claim 4: 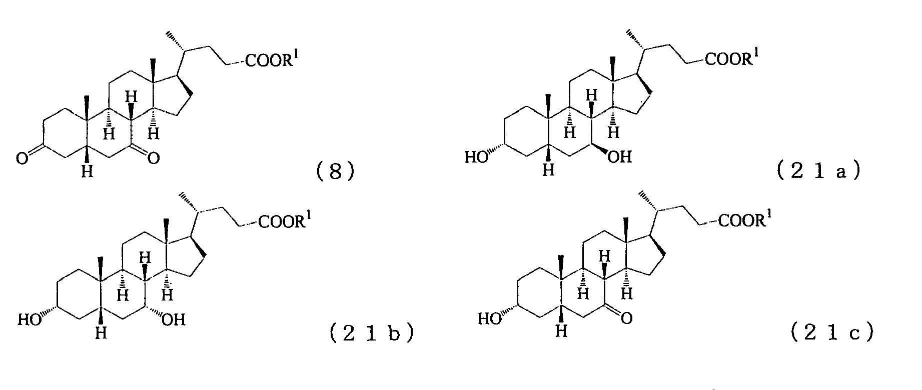 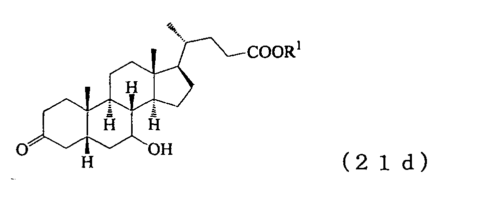 wherein R¹ represents a hydrogen atom, or an alkyl group containing 1 to 6 carbon atoms;
wherein the sterol compound represented by the following formula (1) is cholesta-5,7,24-trien-3β-ol, ergosta-5,7,24(28)-trien-3β-ol, desmosterol, fucosterol, or ergosta-5,24(28)-dien-3β-ol: 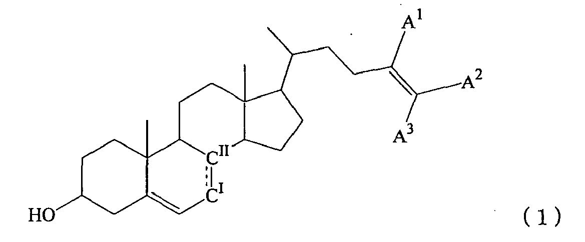 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.

6. The method for producing 3,7-dioxo-5β-cholanic acid (8), ursodeoxycholic acid (21a), chenodeoxycholic acid (21b), 3α-hydroxy-7-oxo-5β-cholanic acid (21c), 7-hydroxy-3-oxo-5β-cholanic acid (21d), or the ester derivatives of these acids, represented by the following formulas (8), (21a), (21b), (21c), or (21d), according to claim 4: 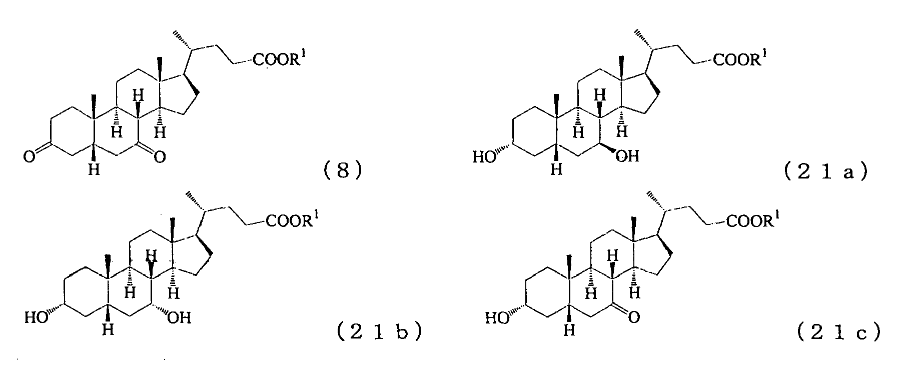 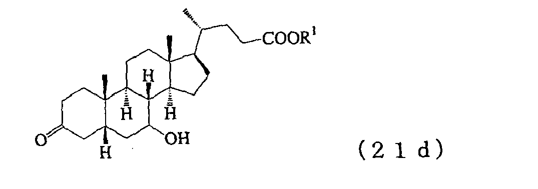 wherein R¹ represents a hydrogen atom, or an alkyl group containing 1 to 6 carbon atoms;
wherein the sterol compound represented by the following formula (1) is cholesta-5,7,24-trien-3β-ol: 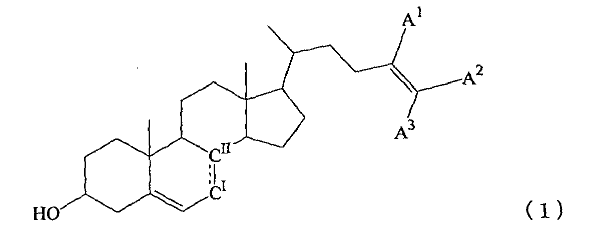 wherein A¹ represents a hydrogen atom or isopropyl group; each of A² and A³ independently represents a methyl group when A¹ is a hydrogen atom, and represents a hydrogen atom or methyl group when A¹ is an isopropyl group; and the bond between C^{I} and C^{II} represents a single bond or double bond.

7. A method for producing cholesta-4,6,24-trien-3-one represented by the following formula (4): 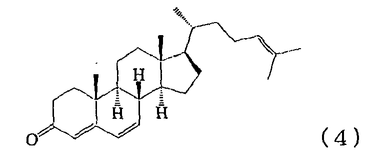 which is **characterized in that** cholesta-5,7,24-trien-3β-ol represented by the following formula (2) is oxidized so as to obtain cholesta-4,7,24-trien-3-one represented by the following formula (3), and **in that** the obtained cholesta-4,7,24-trien-3-one is then isomerized:

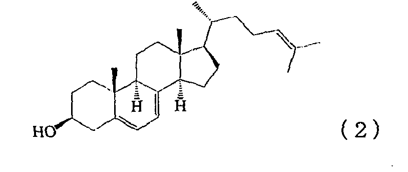

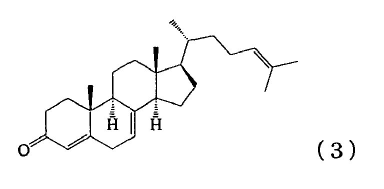

8. The method for producing cholesta-4,6,24-trien-3-one according to claim 7, which is **characterized in that** the oxidation reaction is carried out in the presence of a ketone compound and a metal alkoxide.

9. The method for producing cholesta-4,6,24-trien-3-one according to claim 8, which is **characterized in that** the oxidation reaction is carried out while oxygen is blocked.

10. The method for producing cholesta-4,6,24-trien-3-one according to claim 8, which is **characterized in that** the ketone compound is represented by the formula R²(C=O)R³ wherein each of R² and R³ independently represents a chain or cyclic alkyl group containing 1 to 10 carbon atoms, or R² and R³ may bind to each other, so as to form a cyclic structure containing 3 to 8 carbon atoms.

11. The method for producing cholesta-4,6,24-trien-3-one according to claim 7, which is **characterized in that** the isomerization reaction is carried out in the presence of a basic compound.

12. The method for producing cholesta-4,6,24-trien-3-one according to claim 11, which is **characterized in that** the basic compound is hydroxide, carbonate or alkoxide of alkaline metal or alkaline-earth metal.

13. The method for producing cholesta-4,6,24-trien-3-one according to claim 11, which is **characterized in that** the isomerization reaction is carried out while oxygen is blocked.

14. A cholesta-4,7,24-trien-3-one represented by the following formula (3):

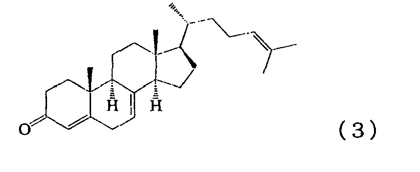

15. A method for producing a 3-oxo-4,7-diene steroid compound, which comprises oxidizing a 3-hydroxy-5,7-diene steroid compound represented by the following formula (2a), (2b), (2c), (2d), or (2e), to a compound represented by the following formula (3a), (3b), (3c), (3d), or (3e): 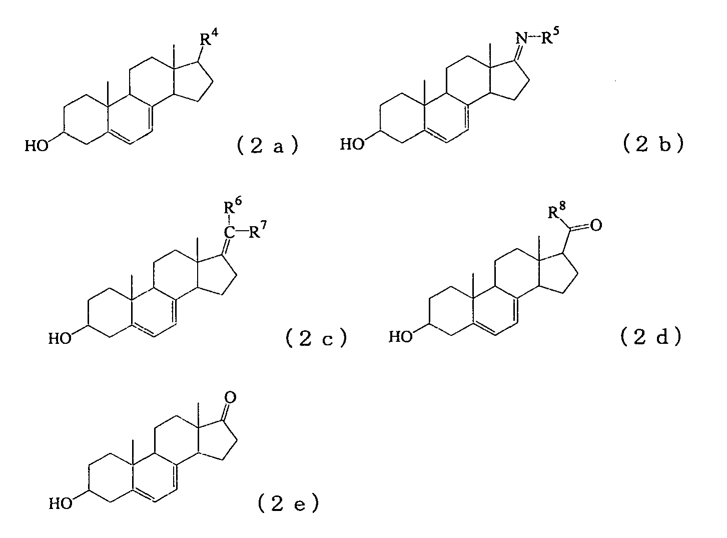 wherein each of R⁴ to R⁸ independently represents a hydrogen atom, a protected hydroxyl group or halogen atom, or an alkyl, alkenyl, or alkynyl group containing 1 to 10 carbon atoms, which may be substituted with a carbonyl group, an ether group, a protected hydroxyl group, a halogen atom, or a carboxyl group), 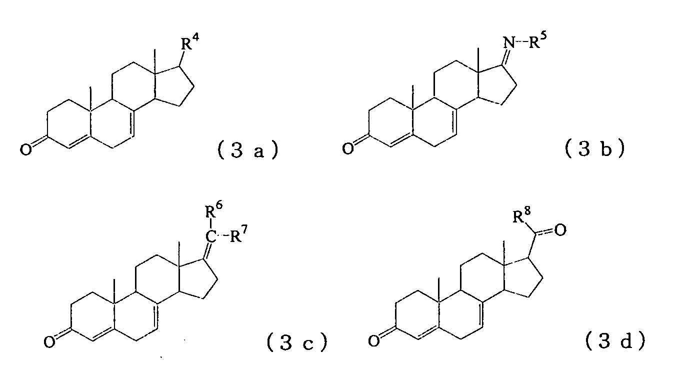 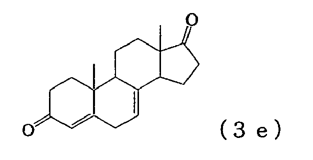 wherein each of R⁴ to R⁸ independently represents a hydrogen atom, a protected hydroxyl group or halogen atom, or an alkyl, alkenyl, or alkynyl group containing 1 to 10 carbon atoms, which may be substituted with a carbonyl group, an ether group, a protected hydroxyl group, a halogen atom, or a carboxyl group;
which is **characterized in that** the above-described oxidation is carried out in the presence of a ketone compound and a metal alkoxide, while oxygen is blocked.

16. A method for producing a 3-oxo-4,6-diene steroid compound, which is **characterized in that** 3-oxo-4,7-diene steroid compound represented by the following formula (3a), (3b), (3c), (3d), or (3e) is isomerized to a compound represented by the following formula (4a), (4b), (4c), (4d), or (4e), respectively, using a base as a catalyst: 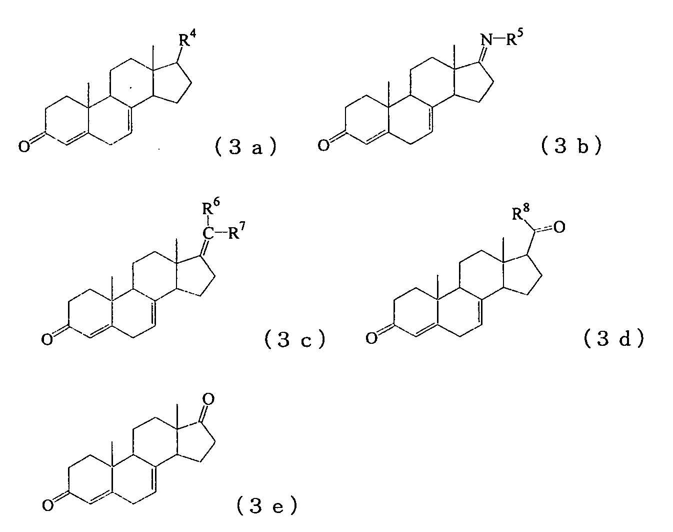 wherein each of R⁴ to R⁸ independently represents a hydrogen atom, a hydroxyl group, a protected hydroxyl group or halogen atom, or an alkyl, alkenyl, or alkynyl group containing 1 to 10 carbon atoms, which may be substituted with a carbonyl group, an ether group, a hydroxyl group, a protected hydroxyl group, a halogen atom, or a carboxyl group, 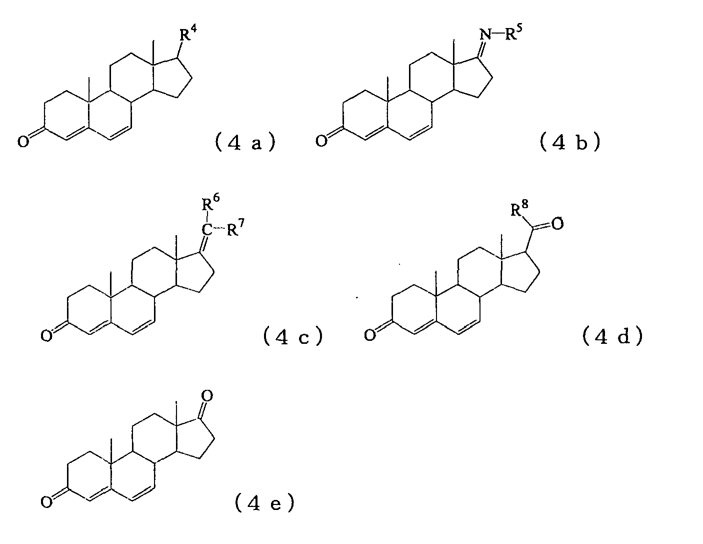 wherein each of R⁴ to R⁸ independently represents a hydrogen atom, a hydroxyl group, a protected hydroxyl group or halogen atom, or an alkyl, alkenyl, or alkynyl group containing 1 to 10 carbon atoms, which may be substituted with a carbonyl group, an ether group, a hydroxyl group, a protected hydroxyl group, a halogen atom, or a carboxyl group.

17. A method for producing 3,7-dioxo-5β-cholanic acid represented by the following formula (8) or ester derivatives thereof: 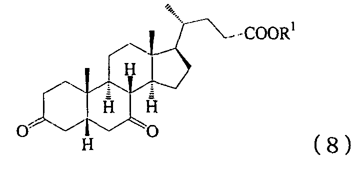 wherein R¹ represents a hydrogen atom, or an alkyl group containing 1 to 6 carbon atoms which is **characterized in that** it comprises:
epoxidizing cholesta-4,6,24-trien-3-one represented by the following formula (4): 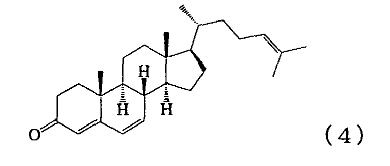
so as to obtain 6,7:24,25-diepoxycholest-4-en-3-one represented by the following formula (5): 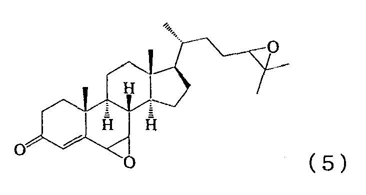
then hydrogenating the obtained compound, so as to obtain 5β-24,25-epoxycholestan-3-one-7-ol represented by the following formula (6): 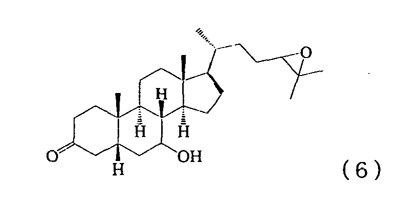
then hydrolyzing the obtained compound, so as to obtain 5β-cholestan-3-one-7,24,25-triol represented by the following formula (7): 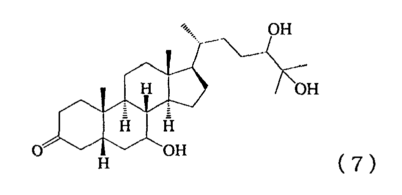
and then oxidizing the obtained compound, and further esterifying the obtained compound in some cases.

18. A method for producing 3,7-dioxo-5β-cholanic acid represented by the following formula (8) or ester derivatives thereof: 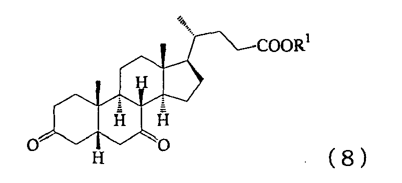 wherein R¹ represents a hydrogen atom, or an alkyl group containing 1 to 6 carbon atoms, which is **characterized in that** it comprises:
epoxidizing cholesta-4,6,24-trien-3-one represented by the following formula (4): 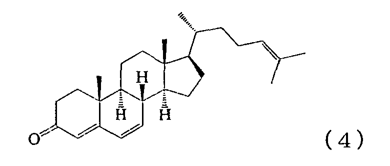
so as to obtain 6,7:24,25-diepoxycholest-4-en-3-one represented by the following formula (5): 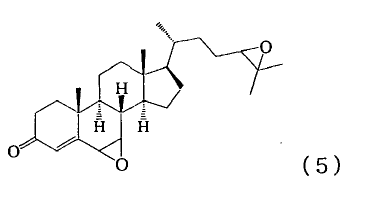
then hydrolyzing the obtained compound, so as to obtain 6,7-epoxycholest-4-en-3-one-24,25-diol represented by the following formula (9): 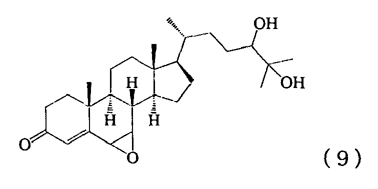
then hydrogenating the obtained compound, so as to obtain 5β-cholestan-3-one-7,24,25-triol represented by the following formula (7): 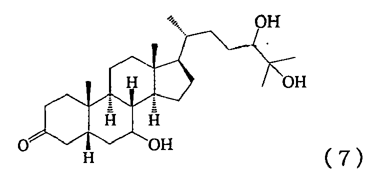
and then oxidizing the obtained compound, and further esterifying the obtained compound in some cases.

19. A method for producing 3,7-dioxo-5β-cholanic acid represented by the following formula (8) or ester derivatives thereof: 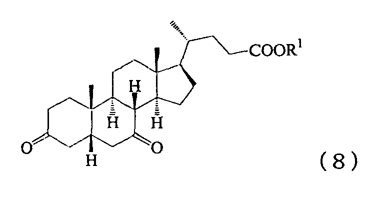 wherein R¹ represents a hydrogen atom, or an alkyl group containing 1 to 6 carbon atoms, which is **characterized in that** it comprises:
epoxidizing cholesta-4,6,24-trien-3-one represented by the following formula (4): 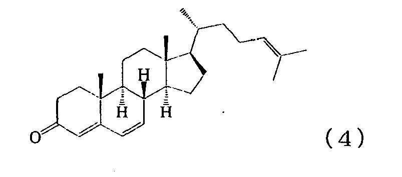
so as to obtain 24,25-epoxycholesta-4,6-dien-3-one represented by the following formula (10): 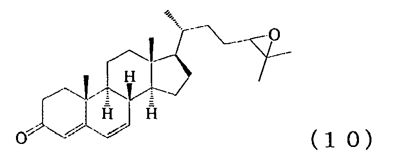
then hydrolyzing the obtained compound, so as to obtain cholesta-4,6-dien-3-one-24,25-diol represented by the following formula (11): 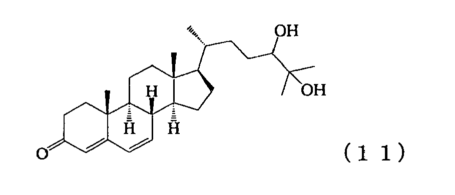
then epoxidizing the obtained compound, so as to obtain 6,7-epoxycholest-4-en-3-one-24,25-diol represented by the following formula (9): 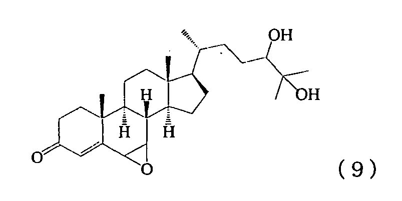
then hydrogenating the obtained compound, so as to obtain 5β-cholestan-3-one-7,24,25-triol represented by the following formula (7): 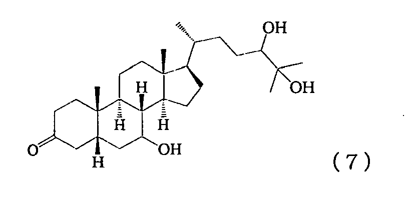
and then oxidizing the obtained compound, and further esterifying the obtained compound in some cases.

20. A method for producing 3,7-dioxo-5β-cholanic acid represented by the following formula (8) or ester derivatives thereof: 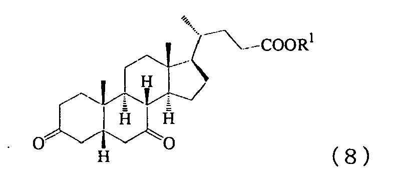 wherein R¹ represents a hydrogen atom, or an alkyl group containing 1 to 6 carbon atoms, which is **characterized in that** it comprises:
epoxidizing cholesta-4,6,24-trien-3-one represented by the following formula (4): 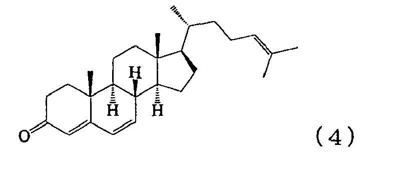
so as to obtain 6,7:24,25-diepoxycholest-4-en-3-one represented by the following formula (5): 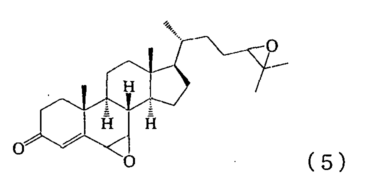
then hydrogenating the obtained compound, so as to obtain 24,25-epoxy-5β-cholestan-3-one-7-ol represented by the following formula (6): 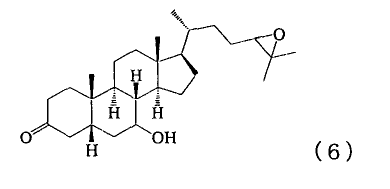
then oxidizing the obtained compound, so as to obtain 24,25-epoxy-5β-cholestane-3,7-dione represented by the following formula (12): 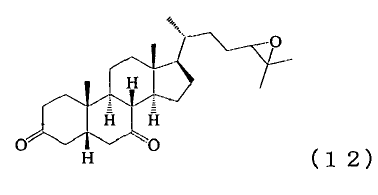
then hydrolyzing the obtained compound, so as to obtain 5β-cholestane-3,7-dione-24,25-diol represented by the following formula (13): 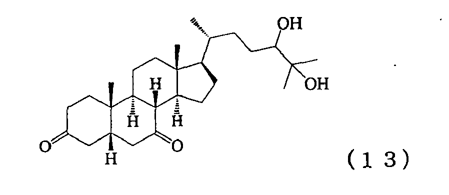
and then oxidizing the obtained compound, and further esterifying the obtained compound in some cases.

21. The method for producing 3,7-dioxo-5β-cholanic acid or ester derivatives thereof according to any one of claims 17 to 20, which is **characterized in that** an organic peroxide is used as an epoxidizing agent.

22. The method for producing 3,7-dioxo-5β-cholanic acid or ester derivatives thereof according to claim 21, which is **characterized in that** as an organic peroxide, it uses percarboxylic acid represented by the formula A⁴CO₃H wherein A⁴ represents a hydrogen atom, an alkyl group containing 1 to 20 carbon atoms that may be substituted with a halogen atom, or an aryl group that may have a substituent, periminocarboxylic acid represented by the formula A⁵(C=NH)OOH wherein A⁵ represents a hydrogen atom, an alkyl group containing 1 to 20 carbon atoms that may be substituted with a halogen atom, or an aryl group that may have a substituent, or a dioxirane derivative represented by the following formula (14): 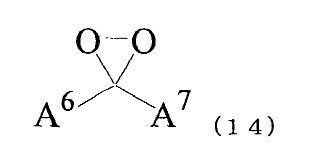 wherein each of A⁶ and A⁷ independently represents an alkyl group containing 1 to 20 carbon atoms that may be substituted with halogen, or A⁶ and A⁷ may bind to each other, so as to form a cyclic structure containing 3 to 8 carbon atoms.

23. The method for producing 3,7-dioxo-5β-cholanic acid or ester derivatives thereof according to claim 21, which is **characterized in that** it uses perbenzoic acid or 2-methylperbenzoic acid as an organic peroxide.

24. The method for producing 3,7-dioxo-5β-cholanic acid or ester derivatives thereof according to claim 23, which is **characterized in that** water is added during the epoxidation reaction.

25. The method for producing 3,7-dioxo-5β-cholanic acid or ester derivatives thereof according to claim 23, which is **characterized in that** the concentration of peracid and that of carboxylic acid are maintained at 0.3 M or less during the epoxidation reaction.

26. The method for producing 3,7-dioxo-5β-cholanic acid or ester derivatives thereof according to claim 17, 18, or 20, which is **characterized in that** it comprises halo-esterifying cholesta-4,6,24-trien-3-one represented by the following formula (4): 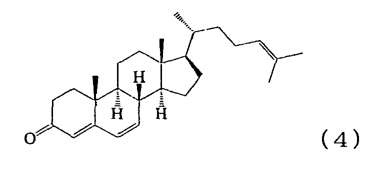 so as to obtain 7,24-dihalo-cholest-4-en-3-one-6,25-diol diester represented by the following formula (15): 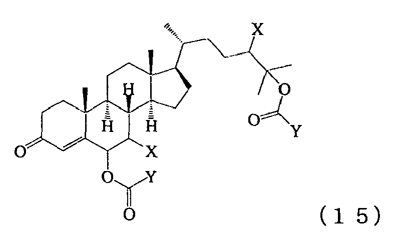 wherein X represents a halogen atom, and Y represents a hydrogen atom, or an alkyl group containing 1 to 10 carbon atoms that may be substituted with halogen, and then performing the alkaline hydrolysis of the ester and cyclization, so as to obtain 6,7:24, 25-diepoxyhcholest-4-en-3-one represented by the following formula (5):

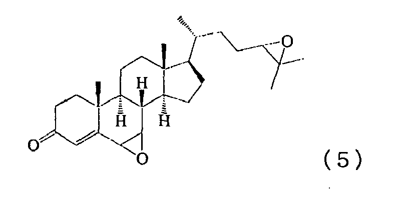

27. The method for producing 3,7-dioxo-5β-cholanic acid or ester derivatives thereof according to claim 26, which is **characterized in that** it uses, as halo-esterifying agents, organic carboxylic acid and a halocation generator represented by the formula Z-X wherein X represents a halogen atom, and Z represents succinimide, phthalimide, acetamide, hydantoin, or a t-butoxy group.

28. The method for producing 3,7-dioxo-5β-cholanic acid or ester derivatives thereof according to any one of claims 17 to 20 and 26, which is **characterized in that** the hydrogenation reaction is carried out in the presence of a noble metal catalyst.

29. The method for producing 3,7-dioxo-5β-cholanic acid or ester derivatives thereof according to claim 28, which is **characterized in that** it uses, as a noble metal catalyst, one or more types of metal palladium selected from the group consisting of powder palladium, and activated carbon-supporting palladium, aluminum oxide-supporting palladium, barium carbonate-supporting palladium, barium sulfate-supporting palladium, and calcium carbonate-supporting palladium, each of which contains 0.5% to 50% by weight of palladium.

30. The method for producing 3,7-dioxo-5β-cholanic acid or ester derivatives thereof according to claim 28 or 29, which is **characterized in that** a base is allowed to coexist during the hydrogenation reaction in the presence of a noble metal catalyst.

31. The method for producing 3,7-dioxo-5β-cholanic acid or ester derivatives thereof according to claim 30, which is **characterized in that** it uses amines as bases.

32. The method for producing 3,7-dioxo-5β-cholanic acid or ester derivatives thereof according to any one of claims 17 to 20 and 26, which is **characterized in that** the hydrolysis reaction of epoxides is carried out in the presence of silica gel or proton acid.

33. The method for producing 3,7-dioxo-5β-cholanic acid or ester derivatives thereof according to claim 32, which is **characterized in that** it uses, as proton acid, hydrochloric acid, sulfuric acid, nitric acid, perchloric acid, phosphoric acid, phosphorous acid, hypophosphorous acid, organic carboxylic acids, or organic sulfonic acids.

34. The method for producing 3,7-dioxo-5β-cholanic acid or ester derivatives thereof according to any one of claims 17 to 20 and 26, which is **characterized in that** it uses, as an oxidizing agent for the oxidation reaction, oxy-halogen acids or salts thereof, molecular halogen, permanganic acids, dichromic acids, or chromic acids.

35. The method for producing 3,7-dioxo-5β-cholanic acid or ester derivatives thereof according to any one of claims 17 to 20 and 26, which is **characterized in that** it uses a compound obtained by isomerizing cholesta-4,7,24-trien-3-one represented by the following formula (3): 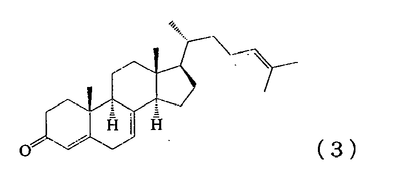
as cholesta-4,6,24-trien-3-one represented by the following formula (4):

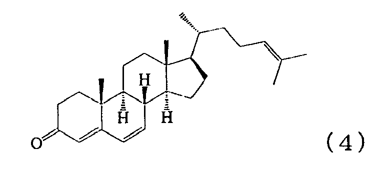

36. The method for producing 3,7-dioxo-5β-cholanic acid or ester derivatives thereof according to claims 35, which is **characterized in that** it uses a compound obtained by oxidizing cholesta-5,7,24-trien-3β-ol represented by the following formula (2): 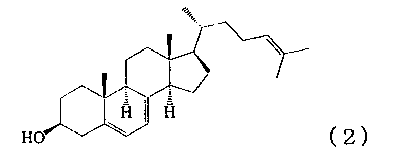
as cholesta-4,7,24-trien-3-one represented by the following formula (3):

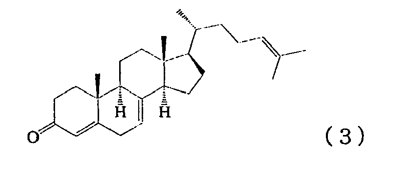

37. A method for producing a vicinal diol compound represented by the following formula (17): 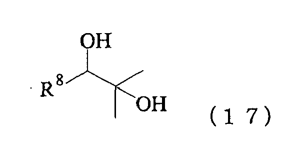 wherein R⁸ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom,
which is **characterized in that** an epoxy compound represented by the following formula (16) is hydrolyzed using silica gel as a catalyst: 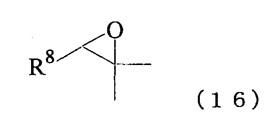 wherein R⁸ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom.

38. A method for producing a vicinal diol compound represented by the following formula (19): 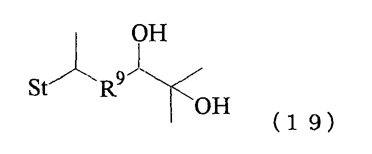 wherein St represents a steroid skeleton consisting of ring A, ring B, ring C, and ring D, and such a steroid skeleton (1) binds to the side chain shown in the formula at position C 17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C13, and C14 may be substituted with a methyl group(s); and R⁹ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom;
the above-described production method being **characterized in that** a steroid epoxy compound represented by the following formula (18) is hydrolyzed using silica gel as a catalyst: 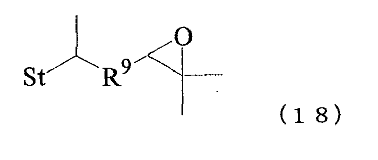 wherein St represents a steroid skeleton consisting of ring A, ring B, ring C, and ring D, and such a steroid skeleton (1) binds to the side chain shown in the formula at position C17, (2) may have a hydroxyl group, a protected hydroxyl group, a keto group, or an epoxy group, on the ring A, ring B, ring C, and ring D, (3) wherein a carbon-carbon bond(s) at one or more positions selected from the group consisting of positions C1 to C8 may have a double bond(s), (4) one or more positions selected from the group consisting of positions C4, C10, C13, and C14 may be substituted with a methyl group(s); and R⁹ represents an alkyl, alkenyl or alkynyl group containing 1 to 20 carbon atoms that may be substituted with a hydroxyl group, a protected hydroxyl group, a carboxyl group, an ester group, a carbonyl group, a cyano group, an amino group, or a halogen atom.

39. A 6,7:24,25-diepoxycholest-4-en-3-one represented by the following formula (5):

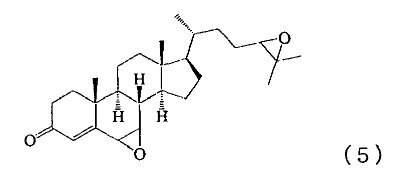

40. A 24,25-epoxycholesta-4,6-dien-3-one represented by the following formula (10):

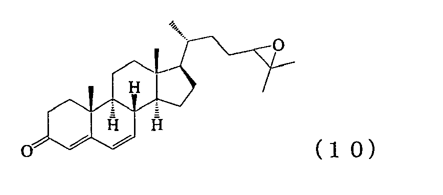

41. A cholesta-4,6-dien-3-one-24,25-diol represented by the following formula (11):

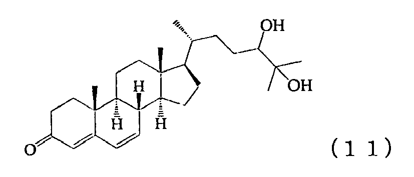

42. A 24,25-epoxy-5β-cholestan-3-one-7-ol represented by the following formula (6):

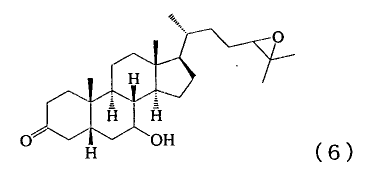

43. A 5β-cholestan-3-one-7,24,25-triol represented by the following formula (7):

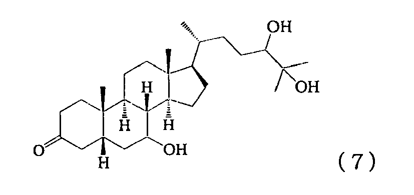

44. A 6,7-epoxycholest-4-en-3-one-24,25-diol represented by the following formula (9):

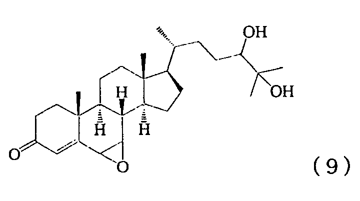

45. A 24,25-epoxy-5β-cholestane-3,7-dione represented by the following formula (12):

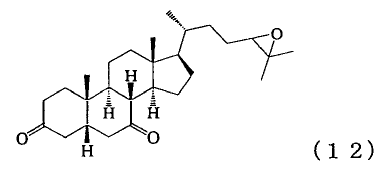

46. A 5β-cholestane-3,7-dione-24,25-diol represented by the following formula (13):

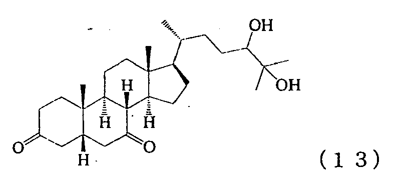

47. A 7,24-dichloro-cholest-4-en-3-one-6,25-diol diformyl ester represented by the following formula (15a):

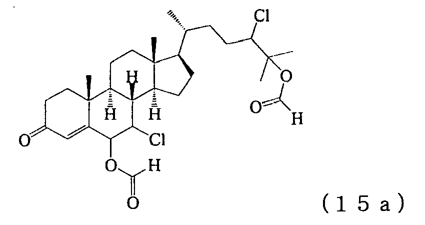

48. A 24,25-epoxycholest-4-en-3-one-7-ol represented by the following formula (20):

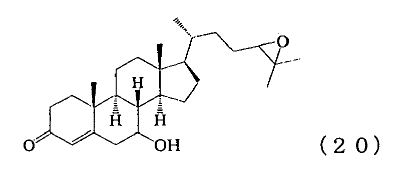

49. A method for producing ursodeoxycholic acid (21a), chenodeoxycholic acid (21b), 3α-hydroxy-7-oxo-5β-cholanic acid (21c) or 7-hydroxy-3-oxo-5β-cholanic acid (21d), or the ester derivatives of these acids, represented by the following formula (21a), (21b), (21c), or (21d): 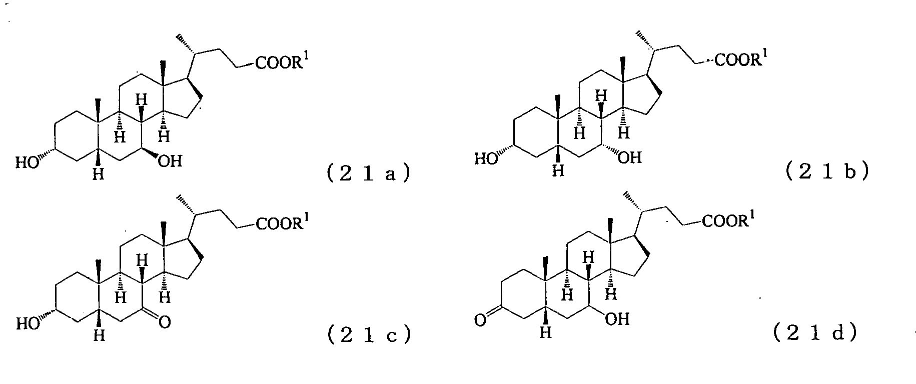 wherein R¹ represents a hydrogen atom, or an alkyl group containing 1 to 6 carbon atoms;
which is **characterized in that** the 3,7-dioxo-5β-cholanic acid represented by the following formula (8) or ester derivatives thereof, produced by the method according to any one of claims 17 to 20, 26, 35, and 36, is reduced, and further reoxidized in some cases: 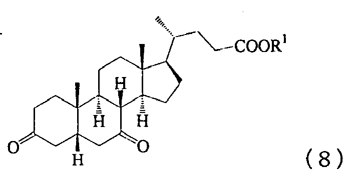 wherein R¹ represents a hydrogen atom, or an alkyl group containing 1 to 6 carbon atoms.
